# EUROPEAN PATENT APPLICATION

(11) **EP 4 130 024 A1**
(43) Date of publication of application: **08.02.2023**
(21) Application number: 21775174.2
(22) Date of filing: 25.03.2021
(51) Int. Cl.: C07K 14/46, G01N 33/68

(54) **METHOD FOR PREDICTING EFFICACY OF TREATMENT OF LUNG CANCER PATIENT USING IMMUNE CHECKPOINT INHIBITOR**

(30) Priority: 26.03.2020 JP 2020056752
(71) Applicant: JSR Corporation, Tokyo 105-8640 (JP); Keio University, Tokyo, 108-8345 (JP)
(72) Inventor: UENO, Masaru, Tokyo 105-8640 (JP); WAKUI, Seiki, Tokyo 105-8640 (JP); MIZUUCHI, Motoaki, Tokyo 105-8640 (JP); WAKIUCHI, Araki, Tokyo 105-8640 (JP); KAWAKAMI, Yutaka, Tokyo 160-8582 (JP); OHTA, Shigeki, Tokyo 160-8582 (JP)
(74) Representative: TBK
(86) International application number: PCT/JP2021/012475
(87) International publication number: WO 2021/193802

(57) **Abstract**

A method for predicting efficacy of treatment of a lung cancer patient using an immune checkpoint inhibitor includes isolating exosomes from a biological sample derived from the lung cancer patient, and determining an expression level of a protein present in the exosomes by a mass spectrometry method, in which the protein is one or more proteins selected from the group of proteins shown in Table 1-1 to Table 1-6.

## Description

### TECHNICAL FIELD

The present invention relates to a method for predicting the efficacy of treatment of a lung cancer patient using an immune checkpoint inhibitor.

Priority is claimed on Japanese Patent Application No. 2020-056752, filed March 26, 2020, the content of which is incorporated herein by reference.

### BACKGROUND ART

As companion diagnoses using immune checkpoint inhibitors (ICI) such as anti-PD-1/PD-L1 antibodies, diagnosis using PD-L1 expression in tumor tissue, diagnosis using microsatellite instability (MSI) testing, and the like are used in practice. However, in diagnosis using PD-L1, there are cases where the determination results of expression differ depending on the antibody and where ICI administration is effective even in cases that are determined to be negative. In diagnosis using MSI testing, patients having microsatellite instability (MSI-High) are 10% or less for most cancer types and there are cases of MSI-High patients also exhibiting drug resistance. Thus, the sensitivity and specificity of these diagnostic methods are not sufficient and the search for biomarkers to predict treatment efficacy is a significant problem.

On the other hand, cells are known to release vesicles called exosomes, which are formed of a lipid bilayer membrane including various cellular contents such as proteins, nucleic acids, lipids and sugars in the inner portion thereof, to the outside of the cell. Exosomes are substances which are important clues to knowing the pathology of living organisms and there is active research on how exosomes are involved in various diseases.

Patent Document 1 discloses the use of perforin in blood samples as a biomarker for evaluating the efficacy of cancer treatment by ICI.

Non-Patent Document 1 discloses PD-L1-positive exosomes as a biomarker for predicting the treatment efficacy of PD-1 inhibitors in metastatic gastric cancer.

However, although biomarkers for companion diagnoses using ICI in lung cancer exist, there is a demand for the development of a method for predicting the effects of ICI on lung cancer with even higher sensitivity and specificity.

### PRIOR ART LITERATURE

### Patent Document

Patent Document 1: Japanese Patent No. 6630026

### Non Patent Document

Non Patent Document 1: Kim ST et al., "Comprehensive molecular characterization of clinical responses to PD-1 inhibition in metastatic gastric cancer. ", Nat med., Vol. 24, pp. 1449-1458, 2018

### DISCLOSURE OF INVENTION

### Problems to be Solved by the Invention

The present invention was created in consideration of the circumstances described above and provides a novel method for predicting the efficacy of treatment of a lung cancer patient using an immune checkpoint inhibitor.

### Means for Solving the Problems

That is, the present invention includes the following aspects.
(1) A method for predicting efficacy of treatment of a lung cancer patient using an immune checkpoint inhibitor, the method including isolating exosomes from a biological sample derived from the lung cancer patient, and determining an expression level of a protein present in the exosomes by a mass spectrometry method, in which the protein is one or more proteins selected from the group of proteins shown in Table 1-1 to Table 1-6.

**[Table 1-1]**

| Protein Name |
|---|
| Band 4.1-like protein 2 |
| Cullin-2 |
| Protein Hikeshi |
| Hippocalcin-like protein 1 |
| UTP--glucose-1-phosphate uridylyltransferase |
| Attractin |
| Annexin A3 |
| Rap1 GTPase-activating protein 2 |
| Radixin |
| Annexin A5 |
| ADP-ribosylation factor 5 |
| L-lactate dehydrogenase B chain |
| Rho GTPase-activating protein 1 |
| Drebrin |
| Serine/threonine-protein kinase PAK 2 |
| Protein BRICK1 |
| Integrin alpha-V |
| Protein kinase C and casein kinase substrate in neurons protein 2 |
| Triokinase/FMN cyclase |
| Adhesion G-protein coupled receptor G1 |
| Integrin beta-5 |
| Isochorismatase domain-containing protein 1 |
| Septin-2 |
| Hydroxyacyl-coenzyme A dehydrogenase, mitochondrial |
| Serine/threonine-protein kinase 38 |
| Transient receptor potential cation channel subfamily M member 4 |
| Cytoskeleton-associated protein 5 |
| FAS-associated factor 1 |
| Insulin receptor |
| Tropomyosin alpha-4 chain |
| Programmed cell death protein 6 |
| Leukocyte elastase inhibitor |
| ADP-ribosylation factor 6 |
| Complement component C9 |
| ATP-dependent (S)-NAD(P)H-hydrate dehydratase |
| Actin, alpha cardiac muscle 1 |
| Heat shock 70 kDa protein 4 |
| 14-3-3 protein theta |
| Phosphatidylinositol transfer protein beta isoform |
| Phosphoribosyltransferase domain-containing protein 1 |
| Costars family protein ABRACL |
| Ubiquitin-conjugating enzyme E2 L5 |
| Sodium/potassium-transporting ATPase subunit alpha-1 |
| 14-3-3 protein epsilon |

**[Table 1-2]**

| Major prion protein |
|---|
| F-actin-capping protein subunit alpha-2 |
| HLA class I histocompatibility antigen, Cw-4 alpha chain |
| GTP-binding protein SAR1a |
| Actin-related protein 2/3 complex subunit 5 |
| Glutathione reductase, mitochondrial |
| Guanine nucleotide-binding protein subunit alpha-11 |
| Protein disulfide-isomerase A5 |
| MARCKS-related protein |
| Septin-7 |
| Hsc70-interacting protein |
| COP9 signalosome complex subunit 8 |
| Vacuolar protein sorting-associated protein 37C |
| Acidic fibroblast growth factor intracellular-binding protein |
| ADP-ribosyl cyclase/cyclic ADP-ribose hydrolase 1 |
| Ribosome maturation protein SBDS |
| Fructose-bisphosphate aldolase A |
| BH3-interacting domain death agonist |
| Charged multivesicular body protein 4b |
| Guanine nucleotide-binding protein G(s) subunit alpha isoforms short |
| cAMP-dependent protein kinase catalytic subunit alpha |
| Cytosolic non-specific dipeptidase |
| Dynamin-1 |
| Neuroplastin |
| IST1 homolog |
| Sorting nexin-5 |
| Mitochondrial import inner membrane translocase subunit Tim13 |
| Exocyst complex component 4 |
| Moesin |
| Serum albumin |
| Myristoylated alanine-rich C-kinase substrate |
| Sodium/potassium-transporting ATPase subunit beta-3 |
| Vinculin |
| Actin-related protein 2/3 complex subunit 1B |
| Alcohol dehydrogenase class-3 |
| Cysteine and glycine-rich protein 1 |
| E3 ubiquitin-protein ligase TRIM23 |
| Actin-related protein 3 |
| Tetraspanin-15 |
| Ras-related C3 botulinum toxin substrate 1 |
| COP9 signalosome complex subunit 2 |
| Myotrophin |
| Rho-related GTP-binding protein RhoF |
| COP9 signalosome complex subunit 3 |
| Glutathione synthetase |
| DnaJ homolog subfamily C member 5 |

**[Table 1-3]**

| |
|---|
| Glutathione S-transferase omega-1 |
| Glutathione S-transferase Mu 2 |
| Ragulator complex protein LAMTOR2 |
| Cytochrome b5 type B |
| Progressive ankylosis protein homolog |
| Septin-11 |
| Rho-associated protein kinase 1 |
| Ras-related C3 botulinum toxin substrate 3 |
| Isocitrate dehydrogenase [NAD] subunit alpha, mitochondrial |
| Inactive tyrosine-protein kinase transmembrane receptor ROR1 |
| Abl interactor 2 |
| Cell division control protein 42 homolog |
| Transgelin-2 |
| Ras-related protein Rab-2A |
| Charged multivesicular body protein 6 |
| ADP-ribosylation factor 3 |
| Glyoxylate reductase/hydroxypyruvate reductase |
| Profilin-1 |
| Ras-related protein Rab-11B |
| Rab GDP dissociation inhibitor alpha |
| CD97 antigen |
| Programmed cell death protein 10 |
| Antithrombin-III |
| F-actin-capping protein subunit beta |
| Actin-related protein 2/3 complex subunit 2 |
| Twinfilin-2 |
| Ras-related protein Rab-21 |
| Vacuolar-sorting protein SNF8 |
| Caspase-6 |
| Calcineurin subunit B type 1 |
| Extended synaptotagmin-2 |
| Leukocyte surface antigen CD47 |
| COP9 signalosome complex subunit 1 |
| 26S proteasome non-ATPase regulatory subunit 7 |
| Eukaryotic translation initiation factor 3 subunit C-like protein |
| Deoxycytidylate deaminase |
| CD276 antigen |
| Caspase-3 |
| Leucine-rich repeat-containing protein 57 |
| Aldose reductase |
| Diphosphoinositol polyphosphate phosphohydrolase 1 |
| Coagulation factor XIII A chain |
| Hsp70-binding protein 1 |
| Sorting nexin-4 |
| Protein phosphatase 1L |
| Pleckstrin homology domain-containing family N member 1 |

**[Table 1-4]**

| |
|---|
| AP-2 complex subunit alpha-2 |
| Trophoblast glycoprotein |
| Ras-related protein Rab-23 |
| 14-3-3 protein eta |
| Peroxiredoxin-6 |
| 14-3-3 protein beta/alpha |
| Copine-2 |
| 14-3-3 protein gamma |
| Superoxide dismutase [Cu-Zn] |
| Microtubule-associated protein RPIEB family member 1 |
| Vasodilator-stimulated phosphoprotein |
| Phosphoglucomutase-1 |
| Adenylyl cyclase-associated protein 1 |
| Calpain small subunit 1 |
| Dual specificity protein phosphatase 23 |
| Dihydropteridine reductase |
| Ephrin-B1 |
| Golgin subfamily A member 7 |
| Xaa-Pro aminopeptidase 1 |
| Actin-related protein 2/3 complex subunit 3 |
| Sorting nexin-9 |
| Pleckstrin homology domain-containing family O member 2 |
| WD repeat-containing protein 44 |
| N(G),N(G)-dimethylarginine dimethylaminohydrolase 2 |
| COP9 signalosome complex subunit 6 |
| Guanine nucleotide-binding protein G(I)/G(S)/G(O) subunit gamma-10 |
| Rab-like protein 3 |
| Phosphoacetylglucosamine mutase |
| Sorting nexin-2 |
| DDB1- and CUL4-associated factor 11 |
| Ras GTPase-activating protein 1 |
| Ribose-5-phosphate isomerase |
| SHC-transforming protein 1 |
| Glutaminyl-peptide cyclotransferase-like protein |
| Ras-related protein Rab-5C |
| Purine nucleoside phosphorylase |
| Delta-aminolevulinic acid dehydratase |
| Ras-related protein Rab-5B |
| Abl interactor 1 |
| Beta-2-glycoprotein 1 |
| Serine/threonine-protein phosphatase 2B catalytic subunit alpha isoform |
| Actin-related protein 2 |
| Ras-related protein Rap-2a |
| Sorcin |
| Type I inositol 1 ,4,5-trisphosphate 5-phosphatase |
| Ras-related protein Rab-35 |

**[Table 1-5]**

| |
|---|
| Rho GTPase-activating protein 18 |
| Ras-related protein Rab-1A |
| Deoxyribonuclease-1-like 1 |
| Peptidyl-prolyl cis-trans isomerase A |
| Adenylate kinase isoenzyme 1 |
| Equilibrative nucleoside transporter 1 |
| GTPase NRas |
| Rho-related GTP-binding protein RhoG |
| Glycogen phosphorylase, brain form |
| UV excision repair protein RAD23 homolog B |
| Omega-amidase NIT2 |
| Serine hydroxymethyltransferase, cytosolic |
| Sodium/potassium-transporting ATPase subunit beta-1 |
| Endothelial cell-selective adhesion molecule |
| Isocitrate dehydrogenase [NADP] cytoplasmic |
| Peptidyl-prolyl cis-trans isomerase FKBP1A |
| Dystroglycan |
| Tumor susceptibility gene 101 protein |
| Peroxiredoxin-5, mitochondrial |
| Protein XRP2 |
| Actin-related protein 2/3 complex subunit 4 |
| Stathmin |
| NSFL1 cofactor p47 |
| Folate receptor alpha |
| Aldo-keto reductase family 1 member A1 |
| Ran-specific GTPase-activating protein |
| Serine/threonine-protein phosphatase 2A catalytic subunit beta isoform |
| Vacuolar protein sorting-associated protein VTA1 homolog |
| Dual specificity protein phosphatase 3 |
| Membrane cofactor protein |
| Calcium-binding protein 39 |
| Argininosuccinate lyase |
| Mitochondrial import receptor subunit TOM34 |
| Protein numb homolog |
| SUMO-conjugating enzyme UBC9 |
| EH domain-containing protein 3 |
| Ras-related protein Rab-1B |
| Adenine phosphoribosyltransferase |
| Coronin-1A |
| Annexin A4 |
| F-actin-capping protein subunit alpha-1 |
| Protein phosphatase 1 regulatory subunit 7 |
| Peflin |
| Protein/nucleic acid deglycase DJ-1 |
| Ras-related protein Rab-10 Ras-related protein Rap-2b |

**[Table 1-6]**

| |
|---|
| RNA transcription, translation and transport factor protein |
| Crk-like protein |
| Serpin B6 |
| 6-phosphogluconolactonase |
| Guanine nucleotide-binding protein G(I)/G(S)/G(T) subunit beta-1 |
| Lactadherin |
| Ras-related protein Ral-A |
| Protein transport protein Sec23A |
| Dihydropyrimidinase-related protein 2 |
| NCK-interacting protein with SH3 domain |
| Vesicle-associated membrane protein 5 |
| Protein phosphatase 1F |
| Nuclear receptor corepressor 2 |
| Ubiquitin-conjugating enzyme E2 D3 |
| WW domain-binding protein 2 |
| POTE ankyrin domain family member I |
| Copine-1 |
| Alpha-galactosidase A |
| Coronin-1B |
| ATP-dependent RNA helicase DDX1 |
| Ubiquitin-conjugating enzyme E2 K |
| Syntaxin-binding protein 1 |
| WD repeat-containing protein 37 |
| MOB kinase activator 1B |
| Nodal modulator 3 |
| Nucleoside diphosphate kinase B |
| Prostaglandin reductase 1 |
| Phosphoglycerate mutase 2 |
| TLD domain-containing protein 1 |
| Calcium-binding protein 39-like |
| Glutathione S-transferase LANCL1 |
| Prefoldin subunit 6 |
| Cytosolic phospholipase A2 |
| 14-3-3 protein zeta/delta |

(2) The method according to (1), in which the protein is one or more membrane proteins selected from the group of proteins shown in Table 2.

**[Table 2]**

| Protein Name |
|---|
| Band 4.1-like protein 2 |
| Attractin |
| Rho GTPase-activating protein 1 |
| Integrin alpha-V |
| Adhesion G-protein coupled receptor G1 |
| Integrin beta-5 |
| Transient receptor potential cation channel subfamily M member 4 |
| Insulin receptor |
| Sodium/potassium-transporting ATPase subunit alpha-1 |
| Major prion protein |
| Fructose-bisphosphate aldolase A |
| Neuroplastin |
| Sodium/potassium-transporting ATPase subunit beta-3 |
| Tetraspanin-15 |
| DnaJ homolog subfamily C member 5 |
| Cytochrome b5 type B |
| Progressive ankylosis protein homolog |
| Inactive tyrosine-protein kinase transmembrane receptor ROR1 |
| CD97 antigen |
| Extended synaptotagmin-2 |
| Leukocyte surface antigen CD47 |
| CD276 antigen |
| Protein phosphatase 1L |
| Trophoblast glycoprotein |
| Ephrin-B1 |
| Equilibrative nucleoside transporter 1 |
| Sodium/potassium-transporting ATPase subunit beta-1 |
| Endothelial cell-selective adhesion molecule |
| Peptidyl-prolyl cis-trans isomerase FKBP1A |
| Dystroglycan |
| Folate receptor alpha |
| Membrane cofactor protein |
| NCK-interacting protein with SH3 domain |
| Vesicle-associated membrane protein 5 |
| Syntaxin-binding protein 1 |
| Nodal modulator 3 |

(3) The method according to (1), in which the protein is one or more proteins selected from the group of proteins shown in Table 3.

**[Table 3]**

| Protein Name |
|---|
| Integrin beta-5 |
| Sorting nexin-9 |
| Phosphoacetylglucosamine mutase |
| Stathmin |
| Ran-specific GTPase-activating protein |
| EH domain-containing protein 3 |
| Adenine phosphoribosyltransferase |
| Protein phosphatase 1 regulatory subunit 7 |
| Nuclear receptor corepressor 2 |
| TLD domain-containing protein 1 |
| Glutathione S-transferase LANCL1 |

(4) The method according to any one of (1) to (3), in which the immune checkpoint inhibitor is an anti-PD-1 antibody or an anti-PD-L1 antibody.

(5) The method according to any one of (1) to (4), in which the immune checkpoint inhibitor is one or more selected from the group consisting of nivolumab, pembrolizumab, and atezolizumab.

(6) The method according to any one of (1) to (5), in which, as a method for isolating exosomes from the biological sample derived from the lung cancer patient, a mixture of magnetic particles bound to an anti-CD9 antibody, an anti-CD81 antibody, or an anti-CD63 antibody is used.

### Effects of the Invention

According to the aspects described above, it is possible to provide a novel method for predicting the efficacy of treatment of a lung cancer patient using an immune checkpoint inhibitor.

### EMBODIMENTS FOR CARRYING OUT THE INVENTION

### <Immune Checkpoint>

In the present specification, "immune checkpoint (system)" refers to a mechanism that suppresses immune responses by controlling the activity of immune-related cells (including T cells). This system suppresses inappropriate immune responses and excessive inflammatory responses to the cells and tissues of the self. Immune checkpoint molecules are molecules that are expressed on immune-related cells and that transmit signals that suppress immune responses to such immune-related cells by binding to ligands. Examples of immune checkpoint molecules and ligands thereof include molecules such as PD-1, CTLA-4, TIM-3 (T cell immunoglobulin and mucin containing protein-3), LAG-3 (lymphocyte activation gene-3), PD-L1, PD-L2, BTNL2, B7-H3, B7-H4, B7-H5 (VISTA), CD48, CD80, 2B4, BTLA, CD160, CD60, CD86, TIGIT (T cell Immunoreceptor with Ig and ITIM domain), without being limited thereto.

In the present specification, 'immune checkpoint inhibitor' refers to an agent that inhibits signal transmission by immune checkpoint molecules by inhibiting the binding of immune checkpoint molecules to the ligands thereof. In many cancer cells, PD-L1, PD-L2, and CD80, which are ligands for immune checkpoint molecules, are expressed on the cell surface and this leads to evasion of attacks by immune-related cells such as T cells, thus, it is considered that, in a normal state, the body's immune function alone is not able to eliminate cancer tissue. Immune checkpoint inhibitors enable efficient elimination of cancer by the body's immune function by inhibiting ligand-receptor interactions and the like that control the transmission of such suppressive signals from cancer tissue to the immune function.

### <Immune Checkpoint Inhibitors>

Examples of immune checkpoint inhibitors include PD-1 inhibitors, PD-L1 inhibitors, CTLA-4 inhibitors, and the like.

Examples of PD-1 inhibitors include anti-PD-1 antibodies such as Nivolumab (marketed as Opdivo (registered trademark)), Pembrolizumab (marketed as Keytruda (registered trademark)), and the like, without being limited thereto.

Examples of PD-L1 inhibitors include anti-PD-L1 antibodies such as Avelumab, Durvalumab, Atezolizumab (marketed as Tecentriq (registered trademark)), and the like, without being limited thereto.

Examples of CTLA-4 inhibitors include Anti-CTLA-4 antibodies such as Ipilimumab and Tremelimumab, without being limited thereto.

Examples of other immune checkpoint inhibitors include agents that target immune checkpoint proteins such as TIM-3, LAG-3, B7-H3, B7-H4, B7-H5 (VISTA), and TIGIT.

Among these, as the immune checkpoint inhibitors applied to the method of the present embodiment, PD-1 inhibitors or PD-L1 inhibitors are preferable and anti-PD-1 antibodies or anti-PD-L1 antibodies are more preferable. With anti-PD-1 antibodies or anti-PD-L1 antibodies, applying one or more selected from the group consisting of Nivolumab, Pembrolizumab, and Atezolizumab described in the present Examples is also preferable in the same manner.

### <Exosome-Derived Proteins for Determining Sensitivity to Immune Checkpoint Inhibitors>

The inventors analyzed exosomes in the blood of 10 responder patients with lung cancer with known sensitivity to immune checkpoint inhibitors and 10 non-responders, as shown in the Examples described below. As a result, 2406 proteins present in exosomes were identified and, among the above, exosome-derived proteins were found in which the value of the Area Under the Curve (AUC) of the ROC curve, which is an indicator of discriminatory ability in determining drug sensitivity, was 0.8 or higher. Furthermore, based on the AUC value of the ROC curve and the best cut-off value, an algorithm was constructed in Python and multi-markers having clinically usable sensitivity and specificity in which the proteins described above were combined were found.

### <Method for Predicting Efficacy of Treatment of Lung Cancer Patient using Immune Checkpoint Inhibitor>

A method for predicting the efficacy of treatment of a lung cancer patient using an immune checkpoint inhibitor according to one embodiment of the present invention (may be simply referred to below as "the method of the present embodiment") is a method including isolating exosomes from a biological sample derived from a lung cancer patient and determining the expression level of proteins present in the exosomes by a mass spectrometry method (may be referred to below as "the steps"). In the present specification and in the claims, "companion diagnostic agent" refers to a diagnostic agent used in a test performed before the actual initiation of medication to predict the effect of a drug for individual lung cancer patients, the risk of side effects, and the appropriate dosage of the medication.

The proteins are one or more proteins selected from the protein groups shown in Table 4-1 to Table 4-19 below. As shown in the Examples described below, these proteins have an AUC value of the ROC curve, which is an indicator of discriminatory ability in determining drug sensitivity, of 0.8 or higher and are biomarkers with excellent sensitivity and specificity for predicting the efficacy of treatment of a lung cancer patient using an immune checkpoint inhibitor. Therefore, according to the method of the present embodiment, it is possible to predict the efficacy of treatment of a lung cancer patient using an immune checkpoint inhibitor with high accuracy.

The protein groups shown in Table 4-1 to Table 4-19 below are identical to the protein groups shown in Table 1-1 to Table 1-6 above, and, for the protein groups shown in Table 1-1 to Table 1-6 described above, information is shown for the Genbank accession number of the mRNA base sequence encoding each protein in humans, the Genbank accession number of the amino acid sequence of each protein in humans, the name of the gene encoding each protein in humans, and the UniProt accession number of the amino acid sequence of each protein in humans.

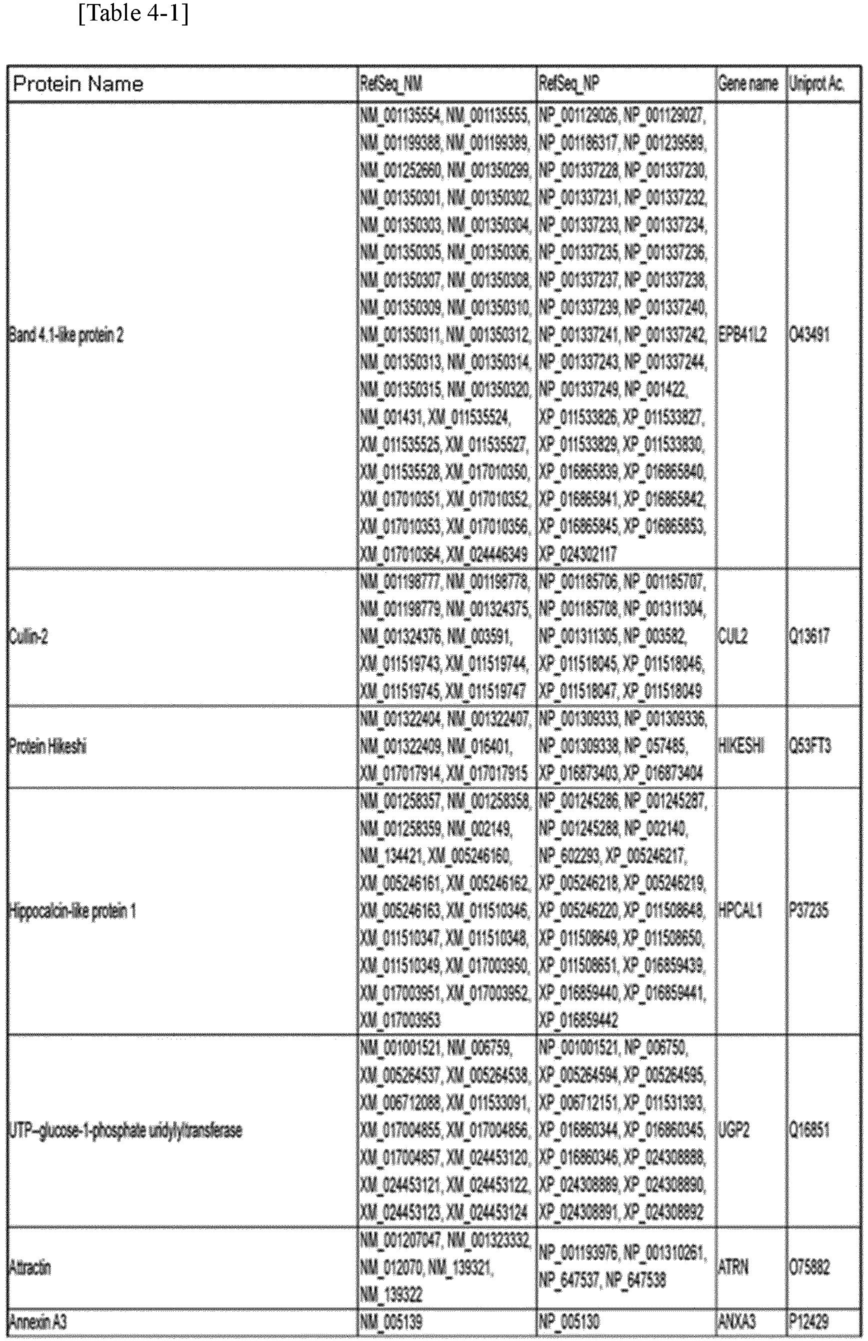

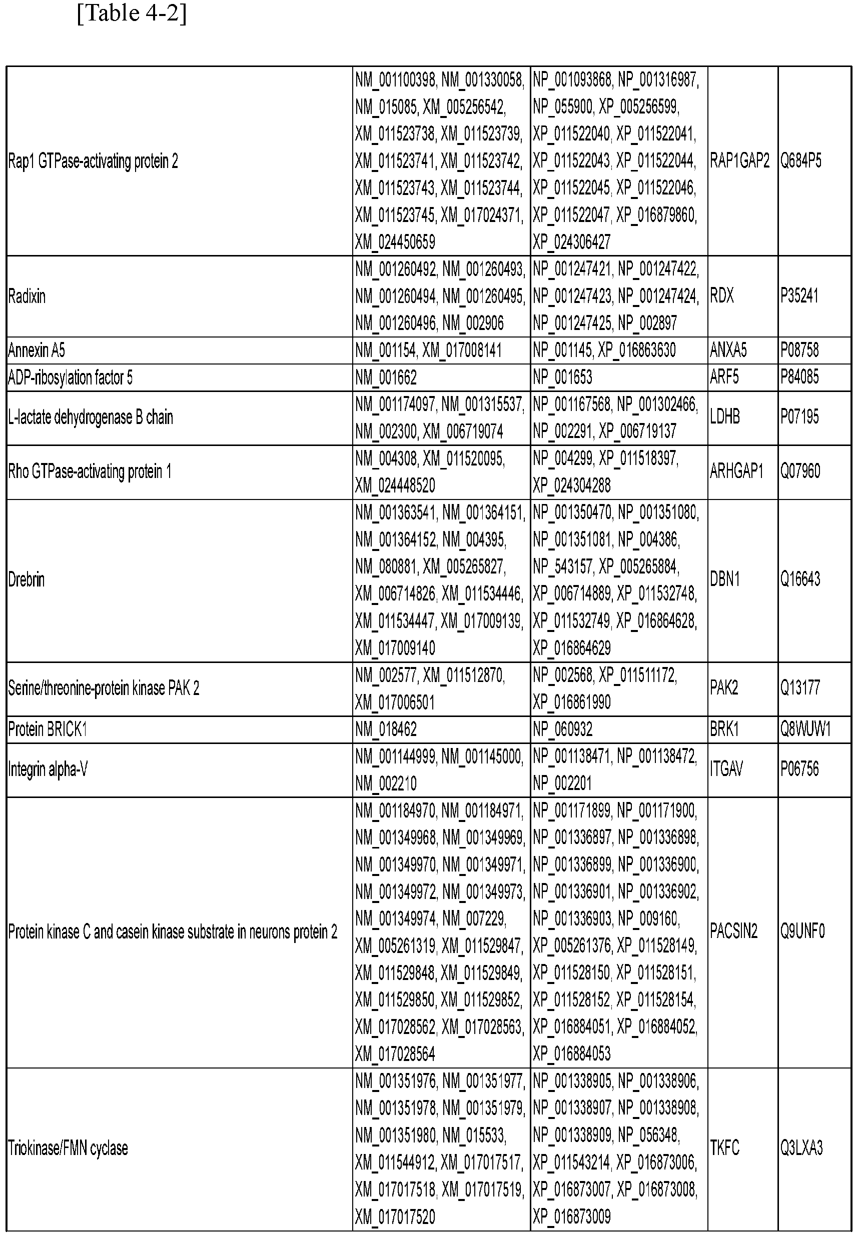

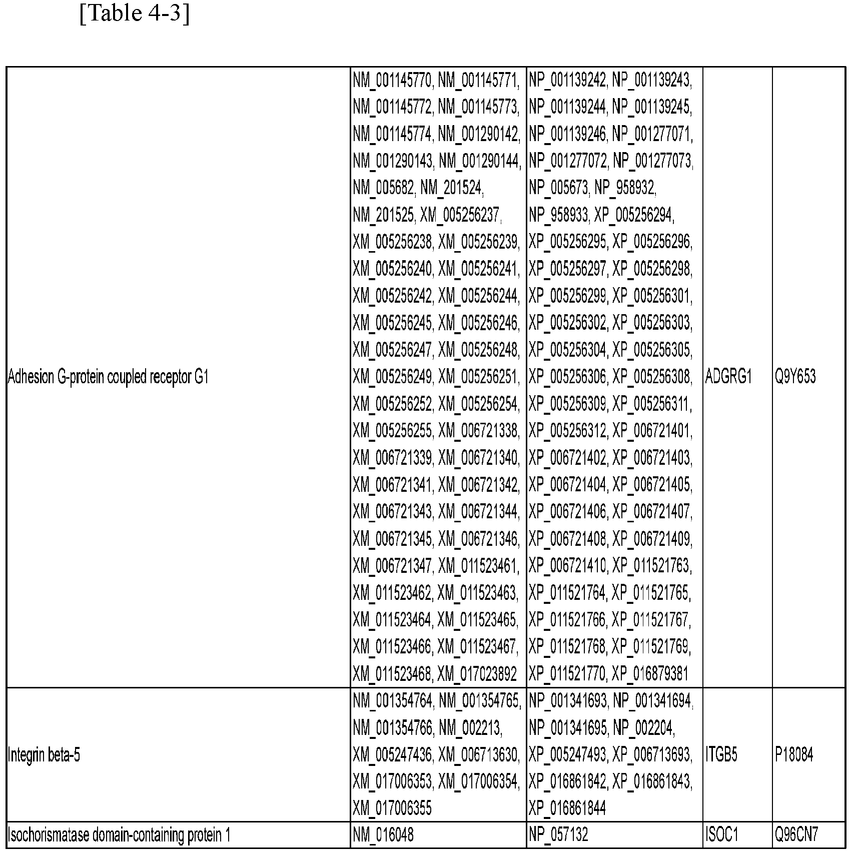

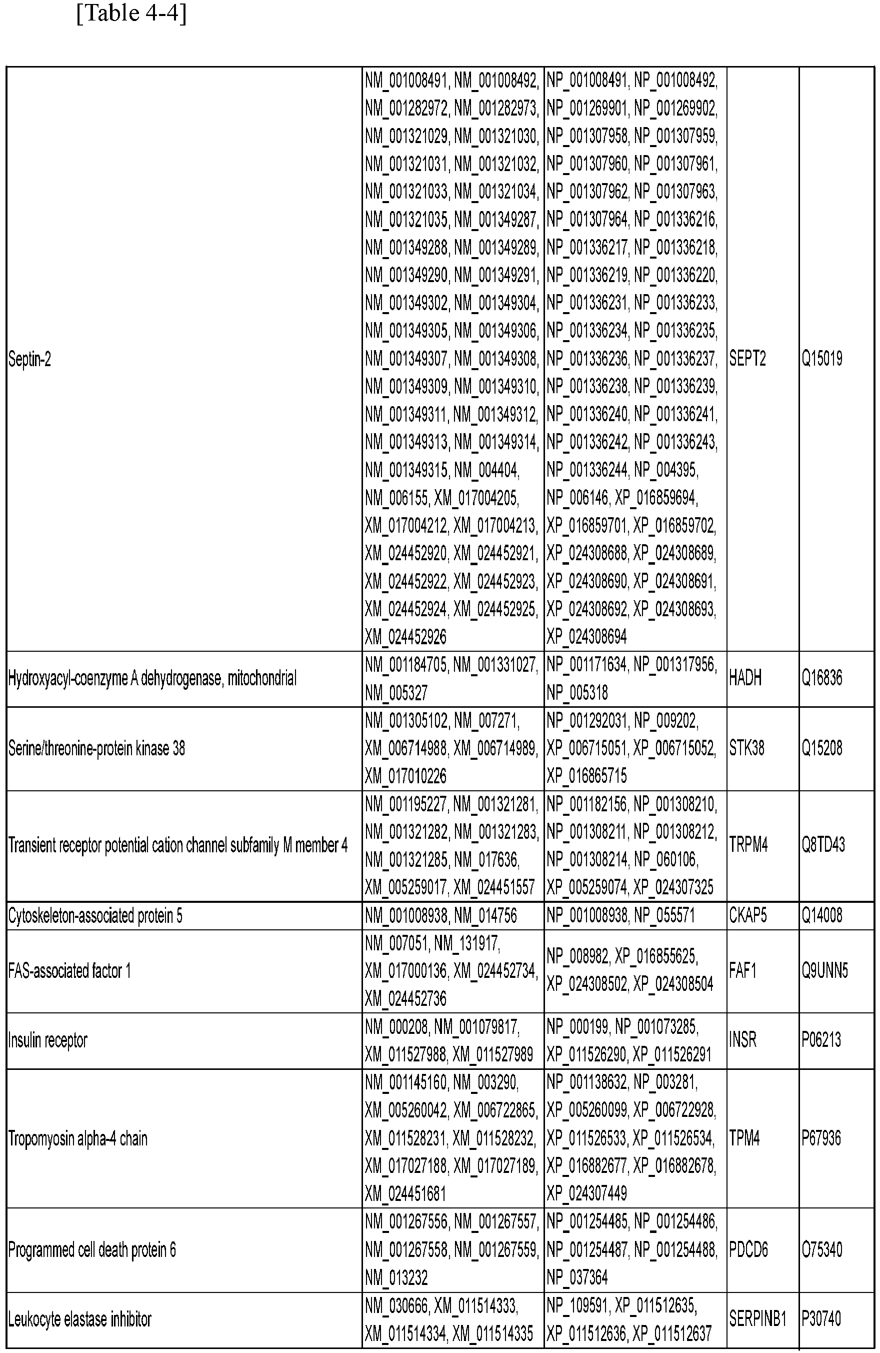

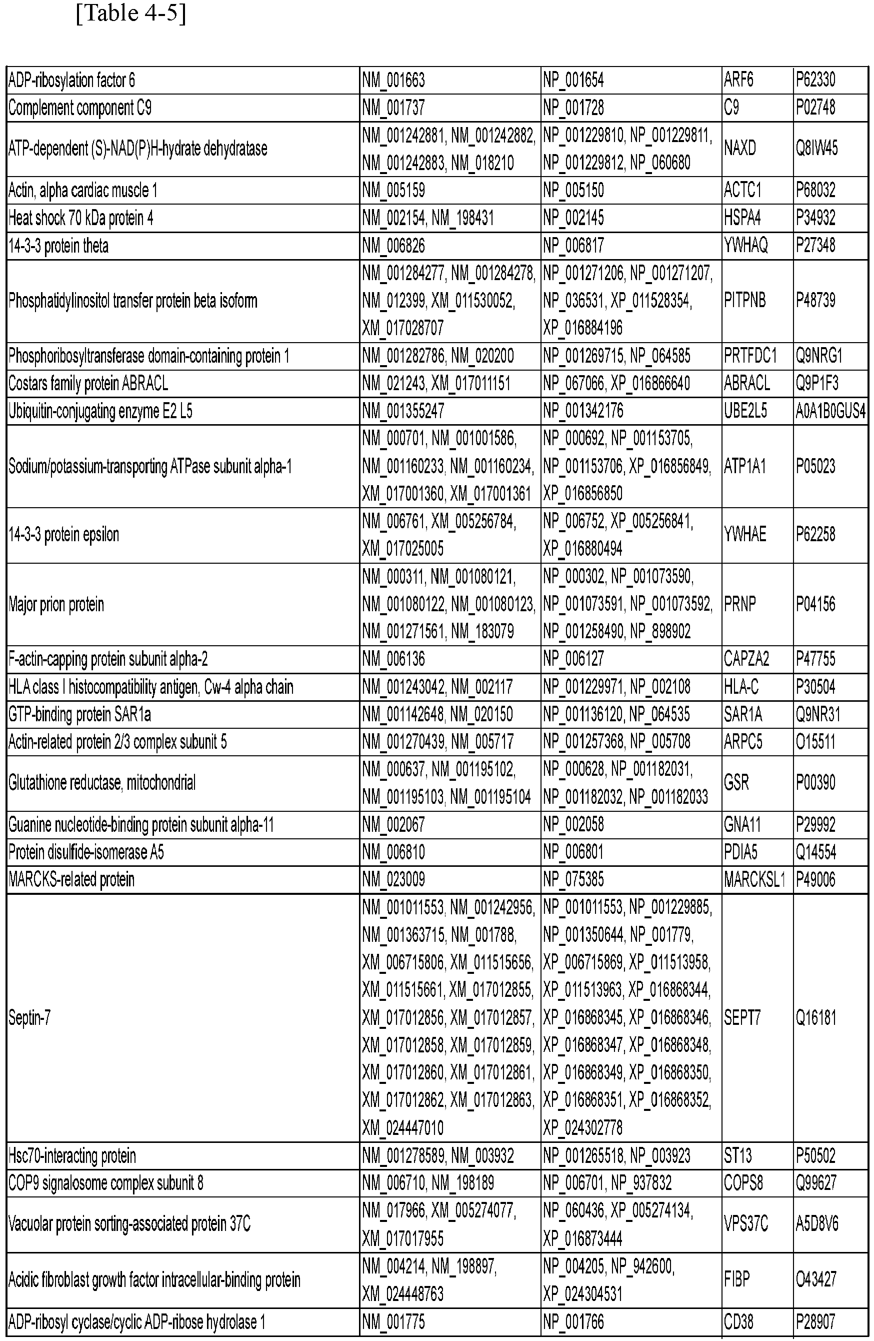

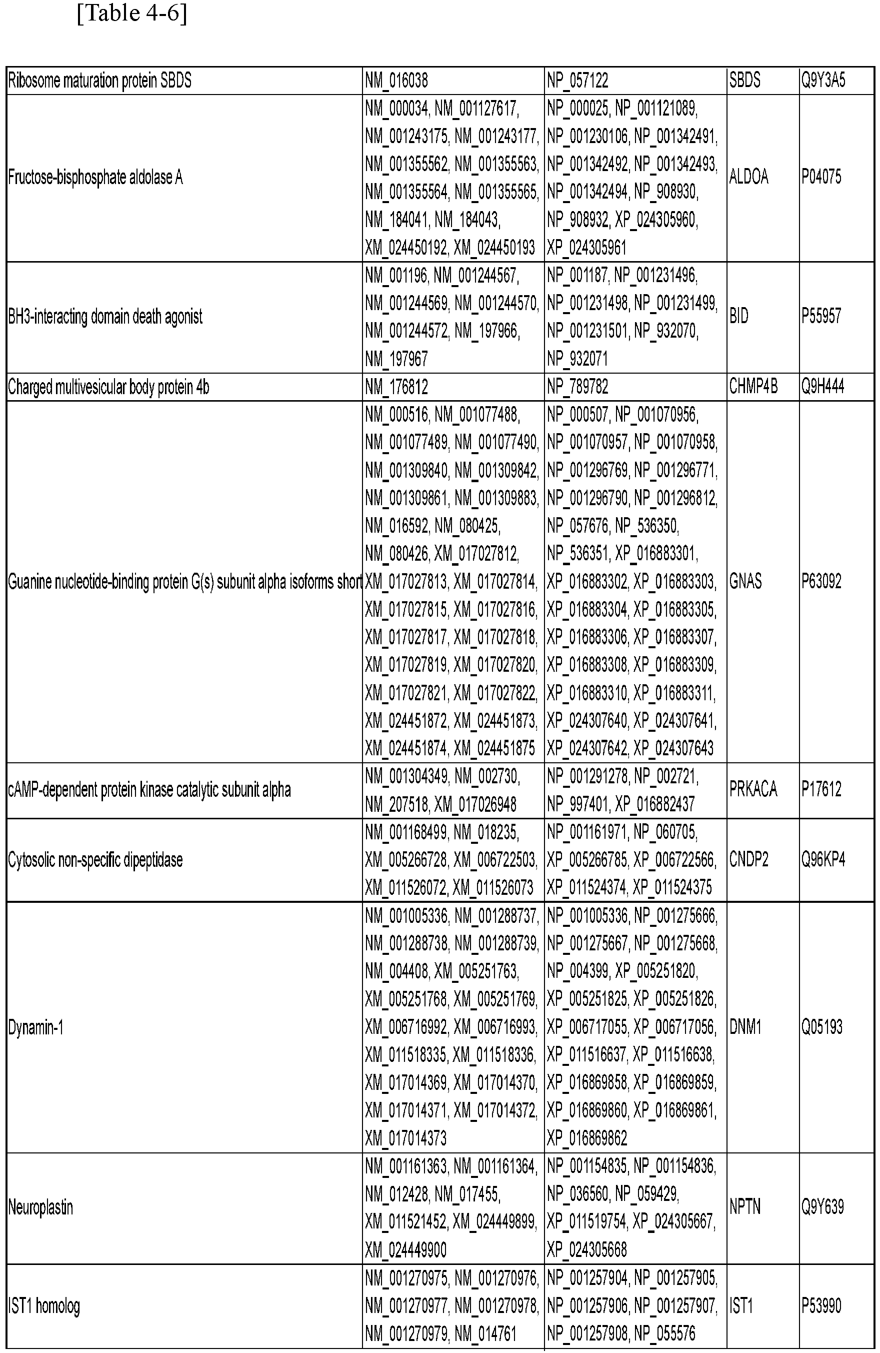

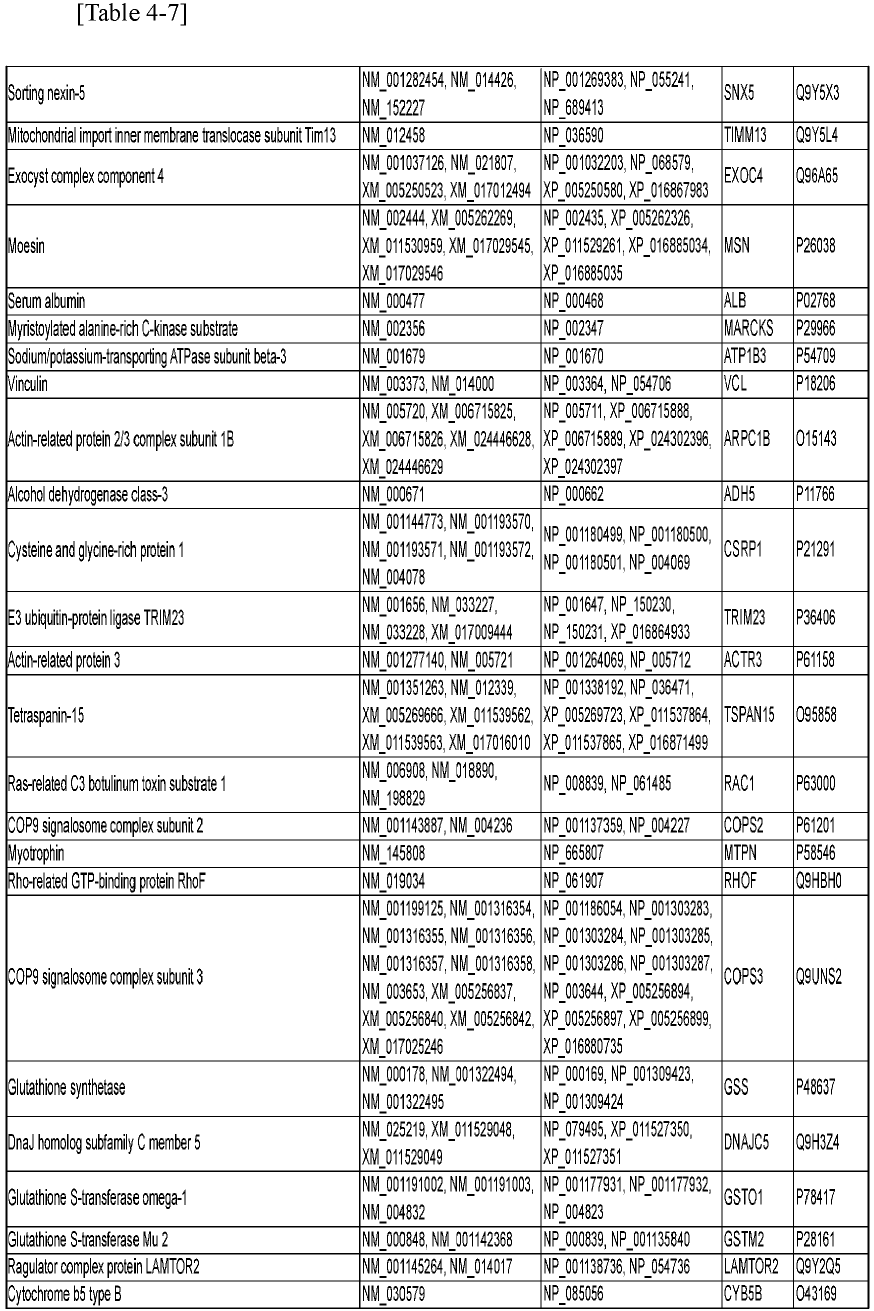

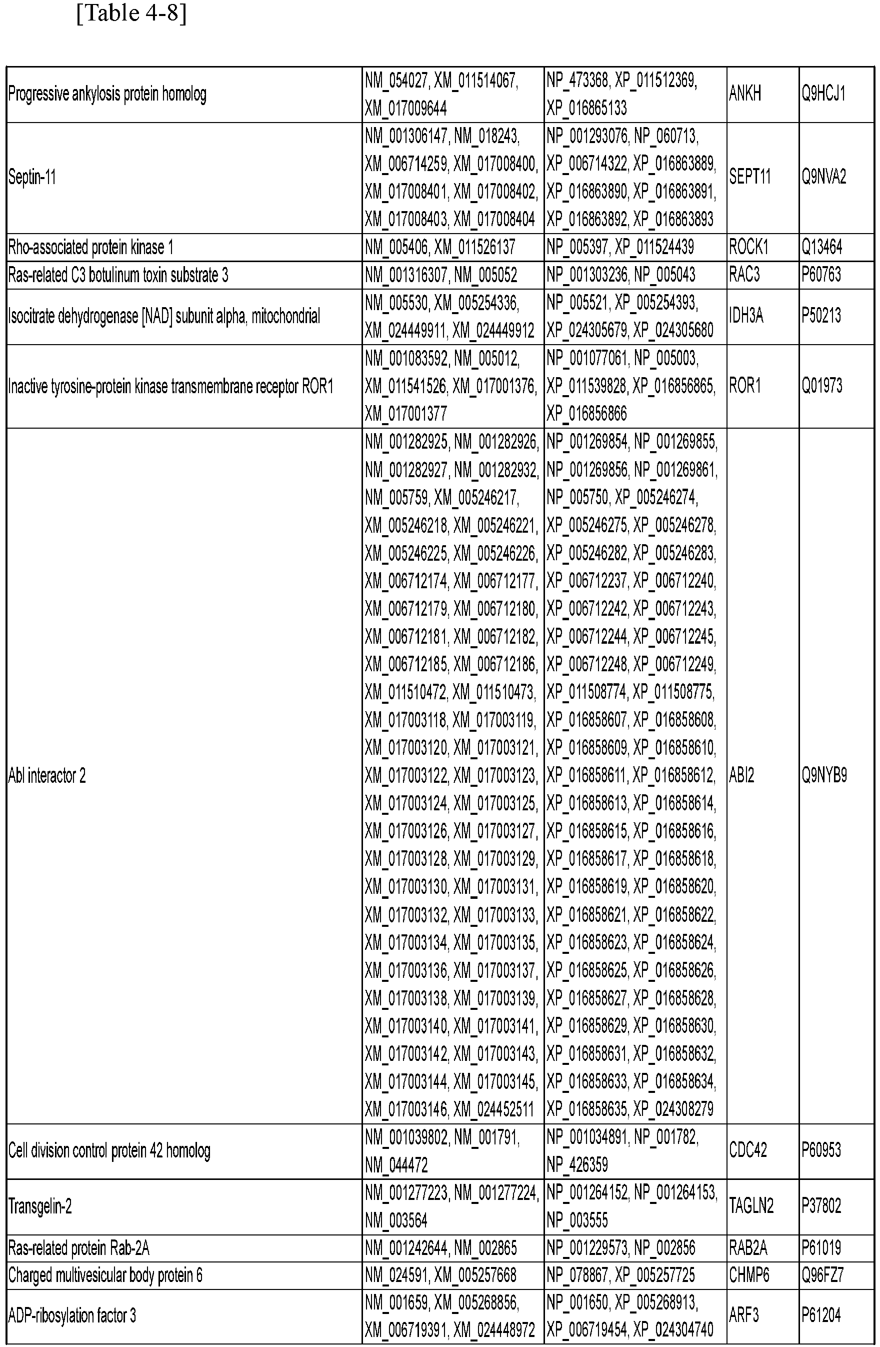

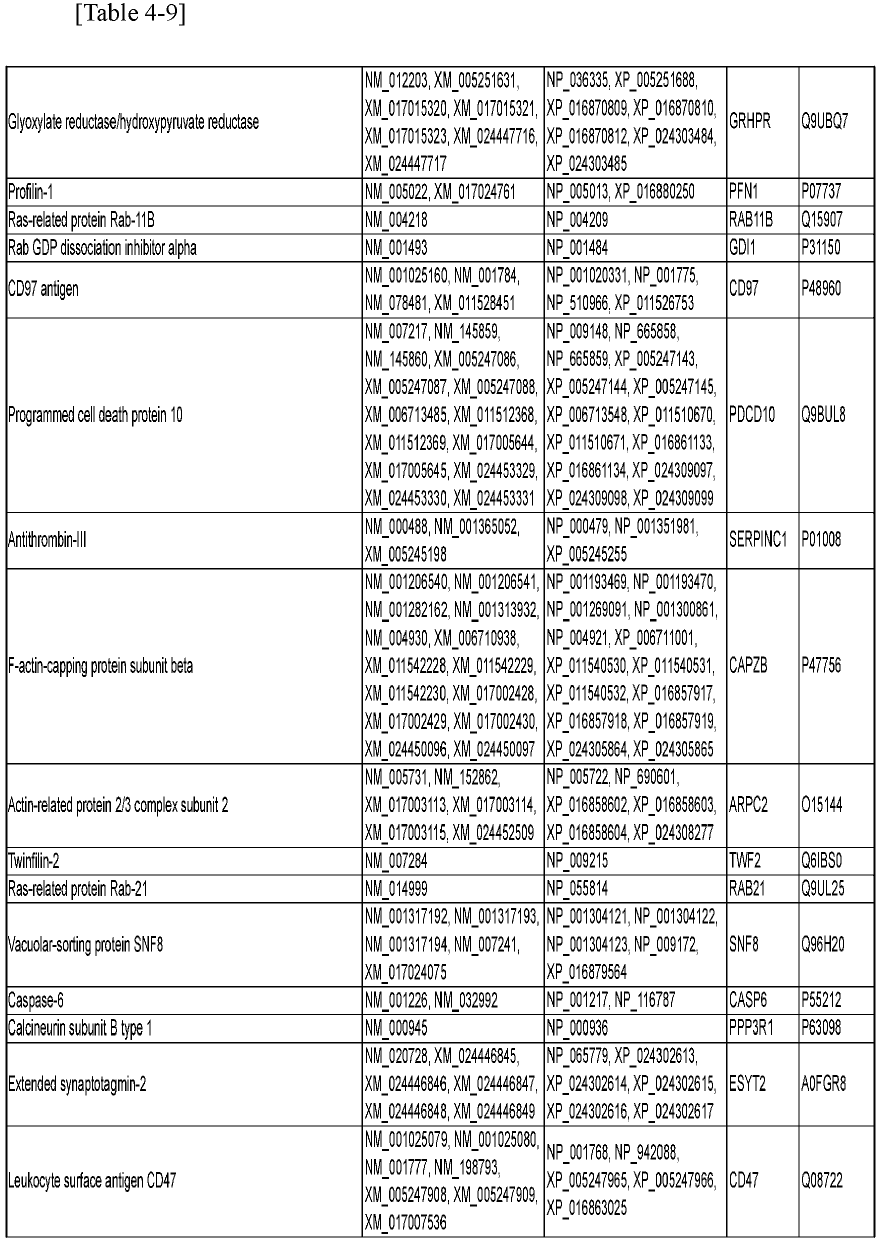

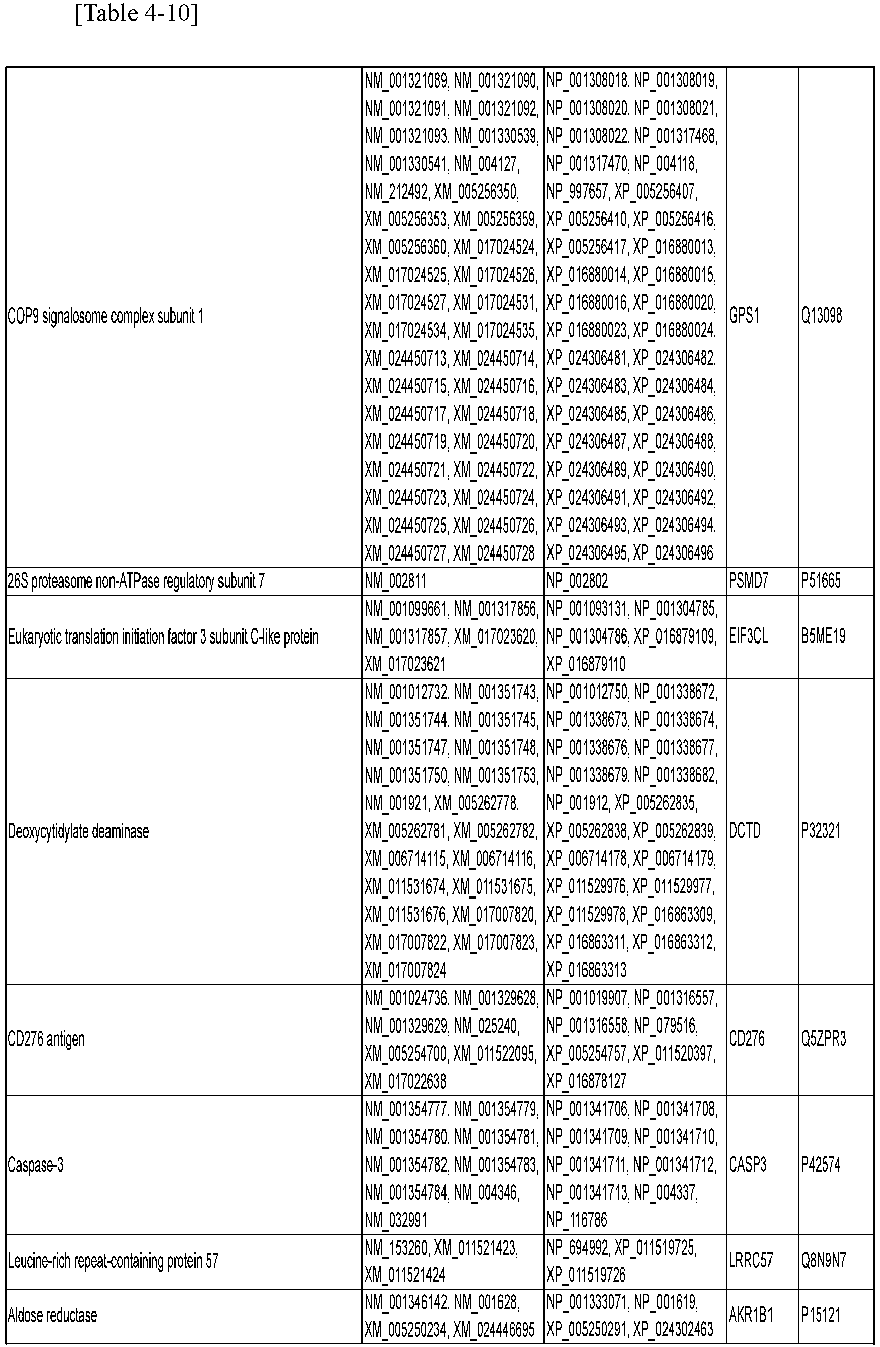

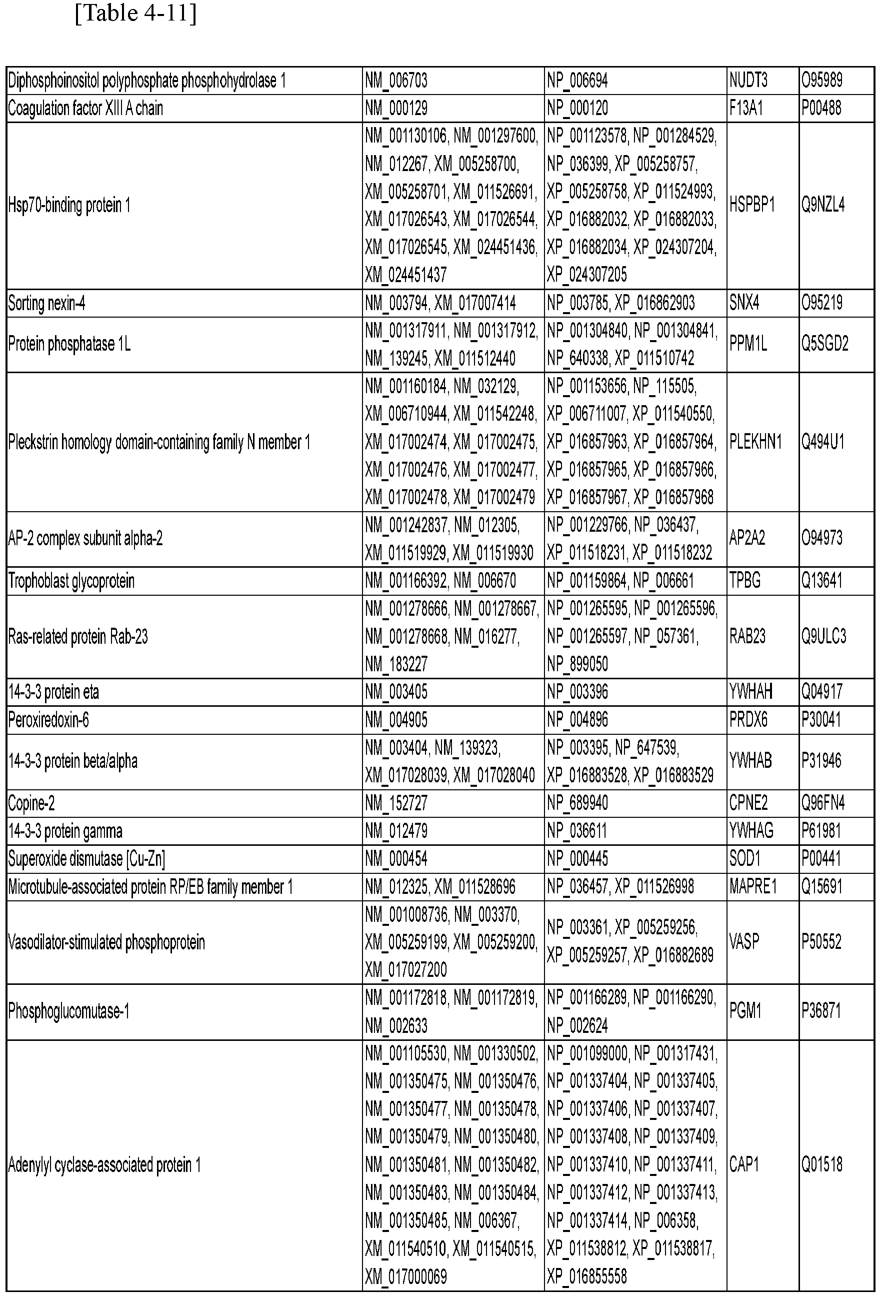

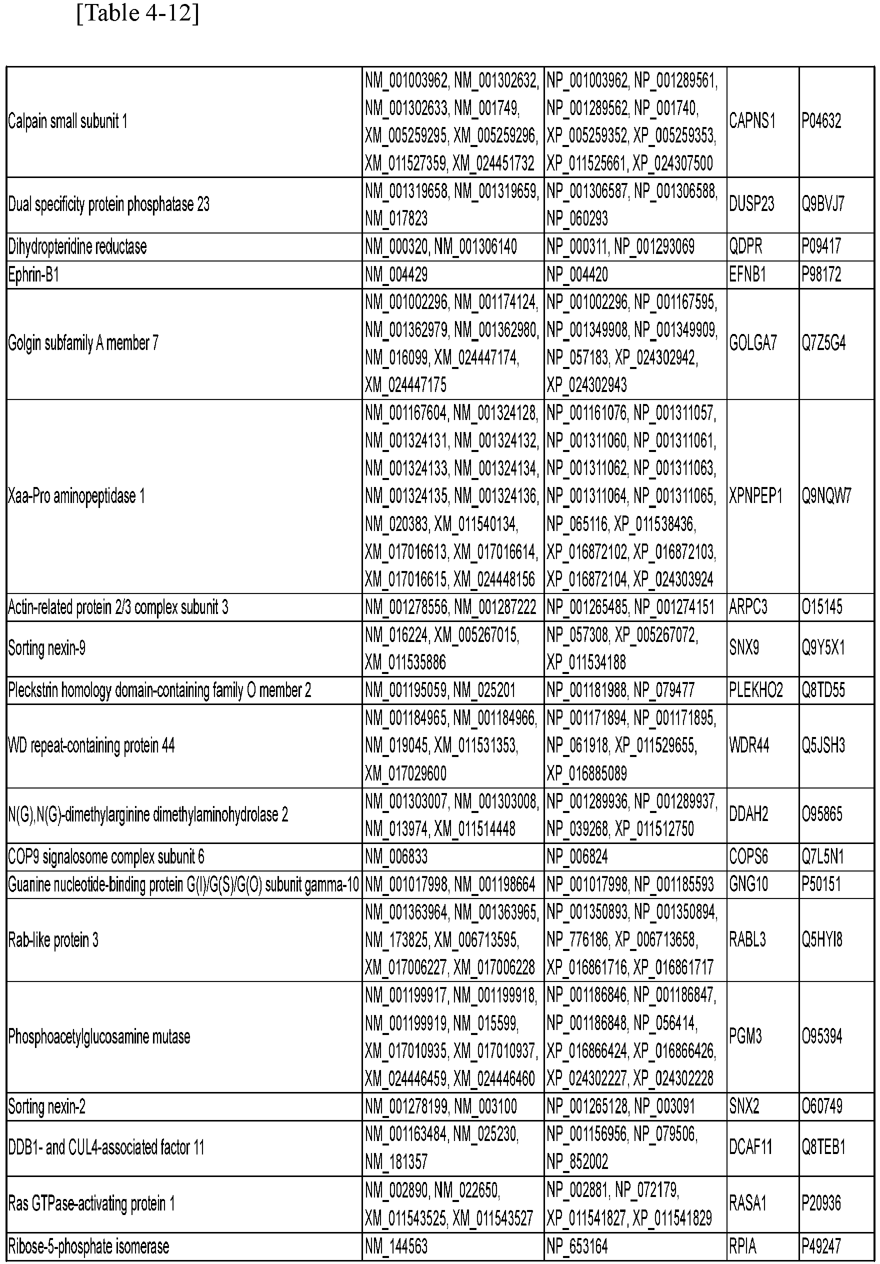

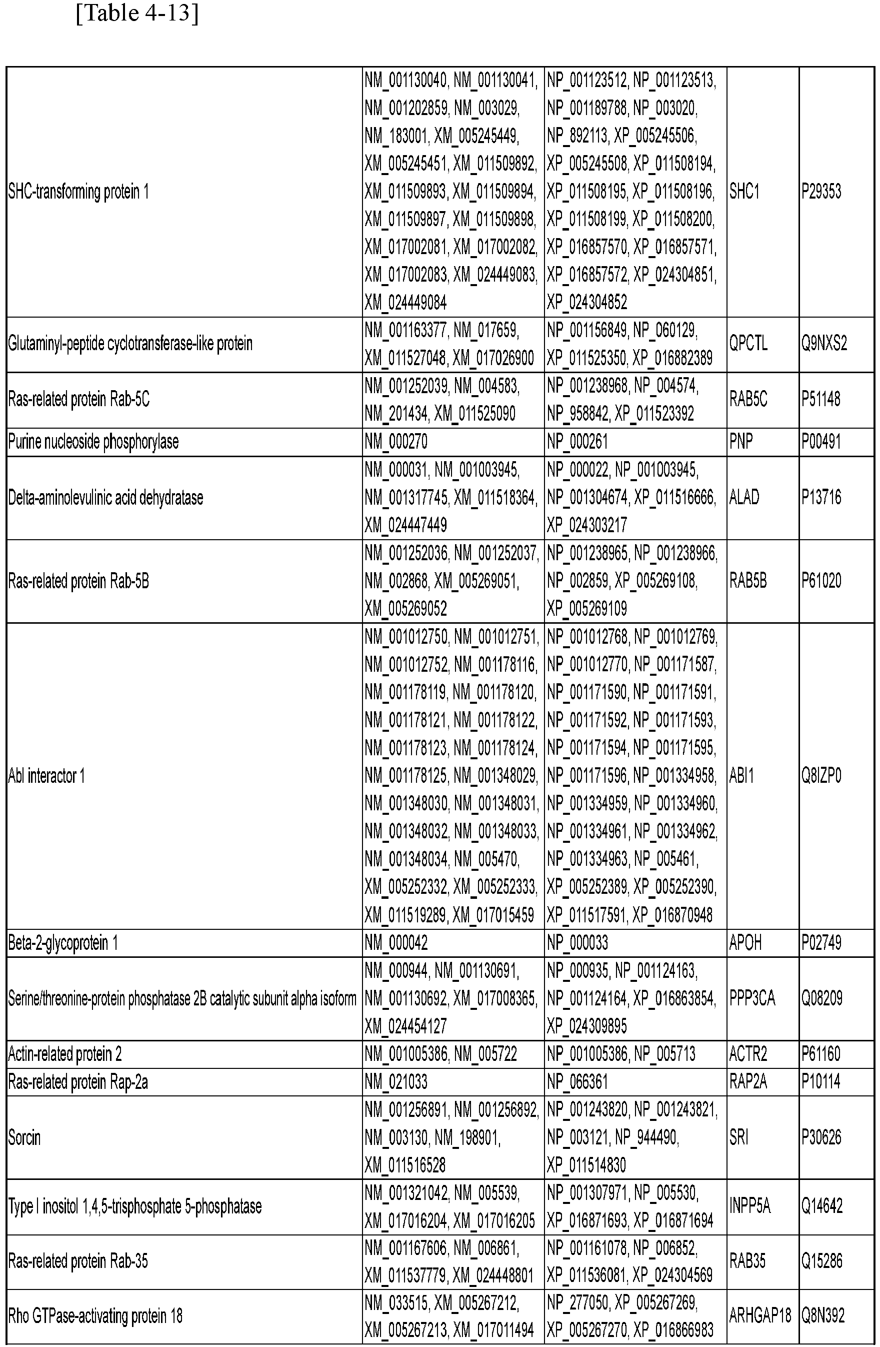

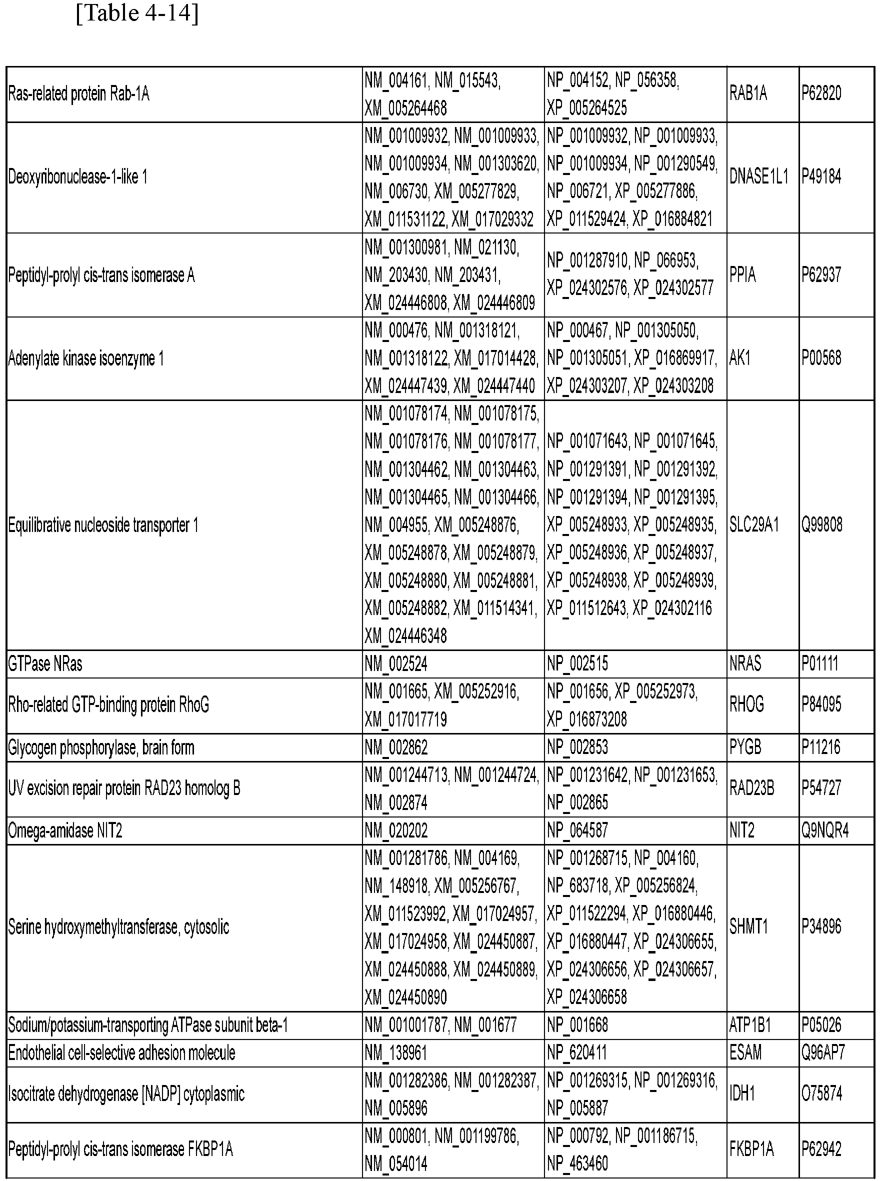

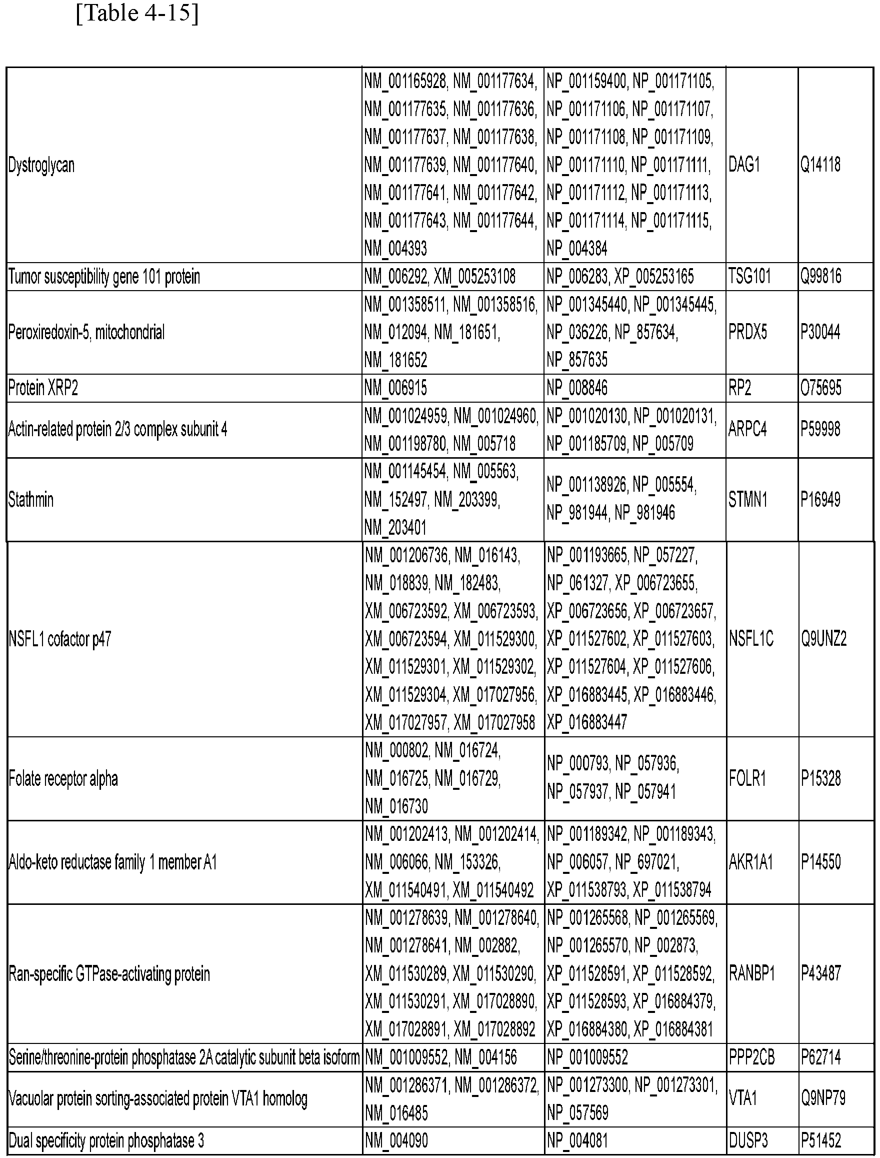

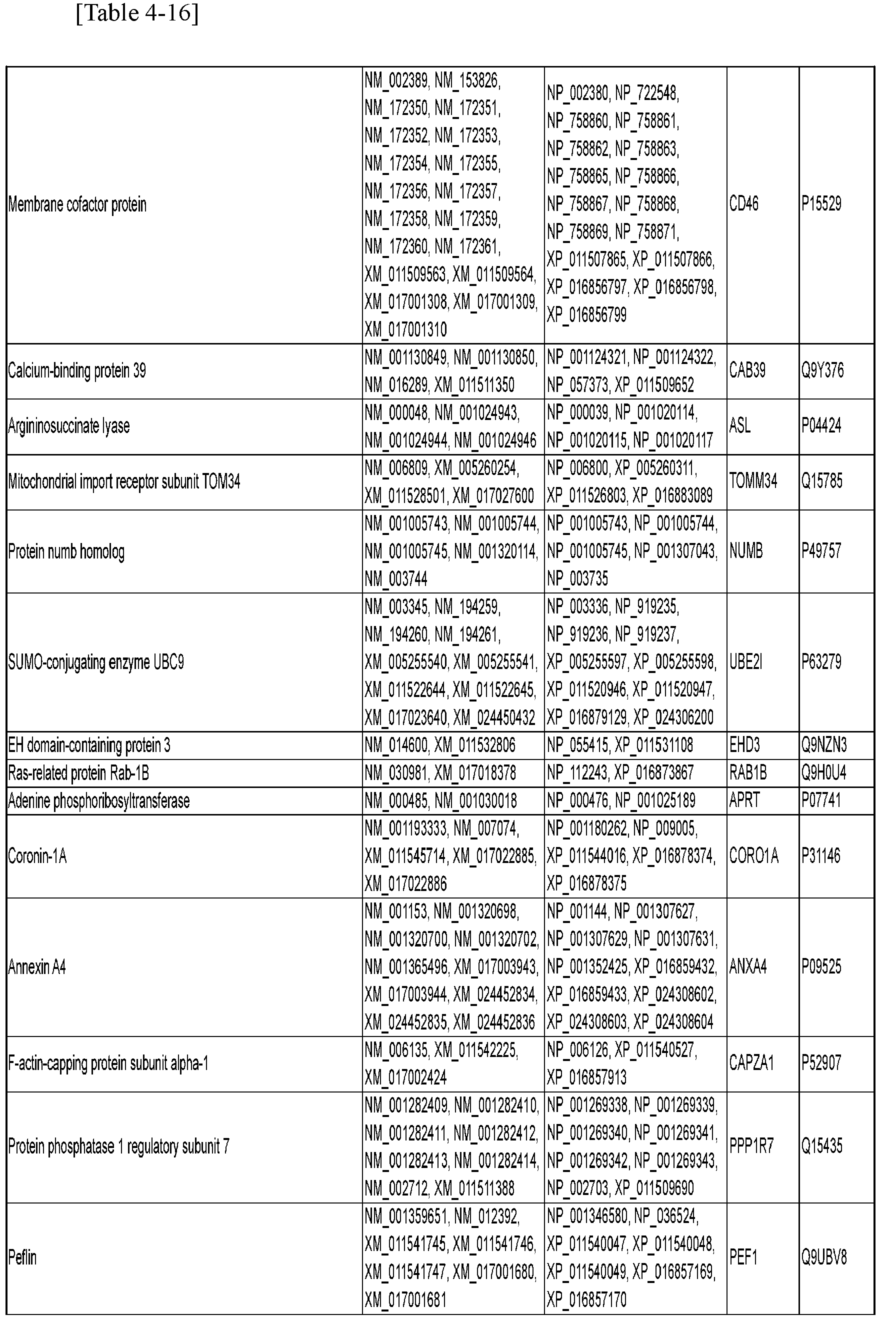

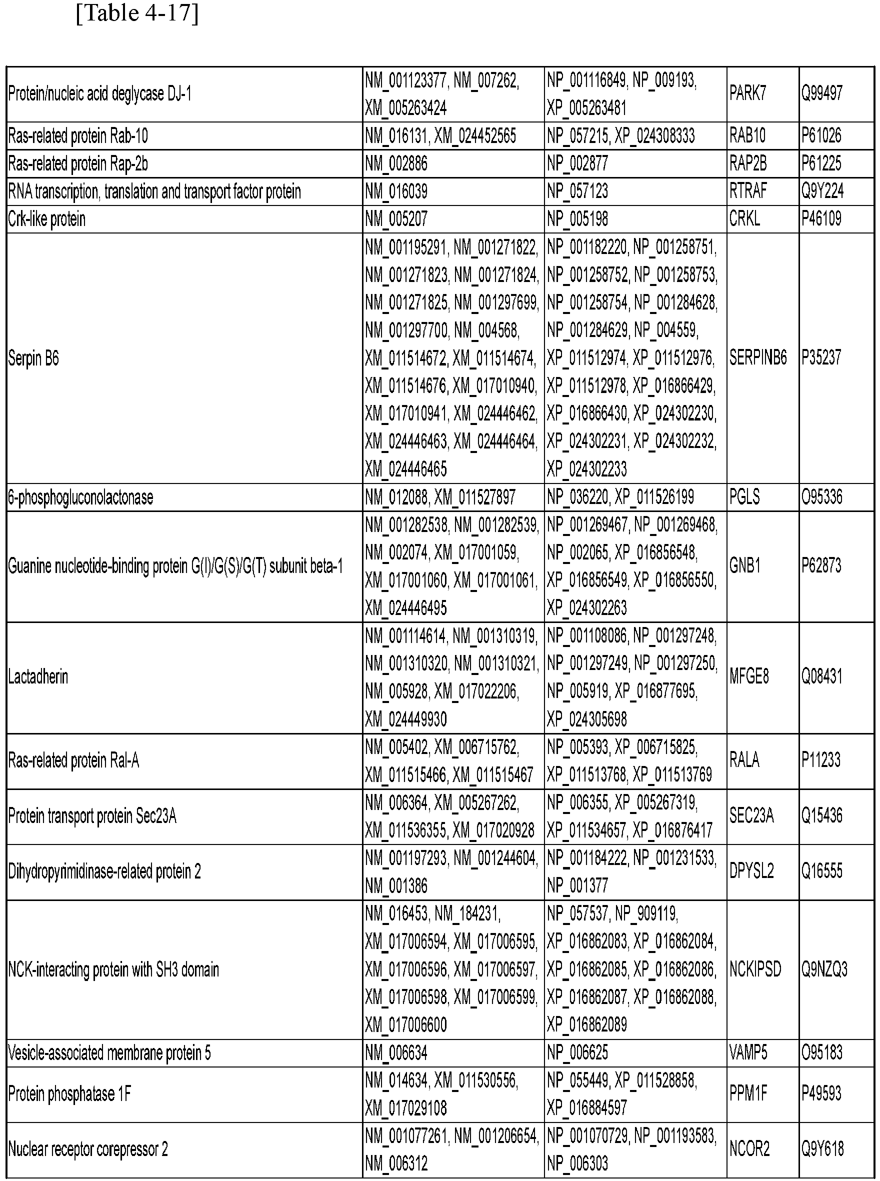

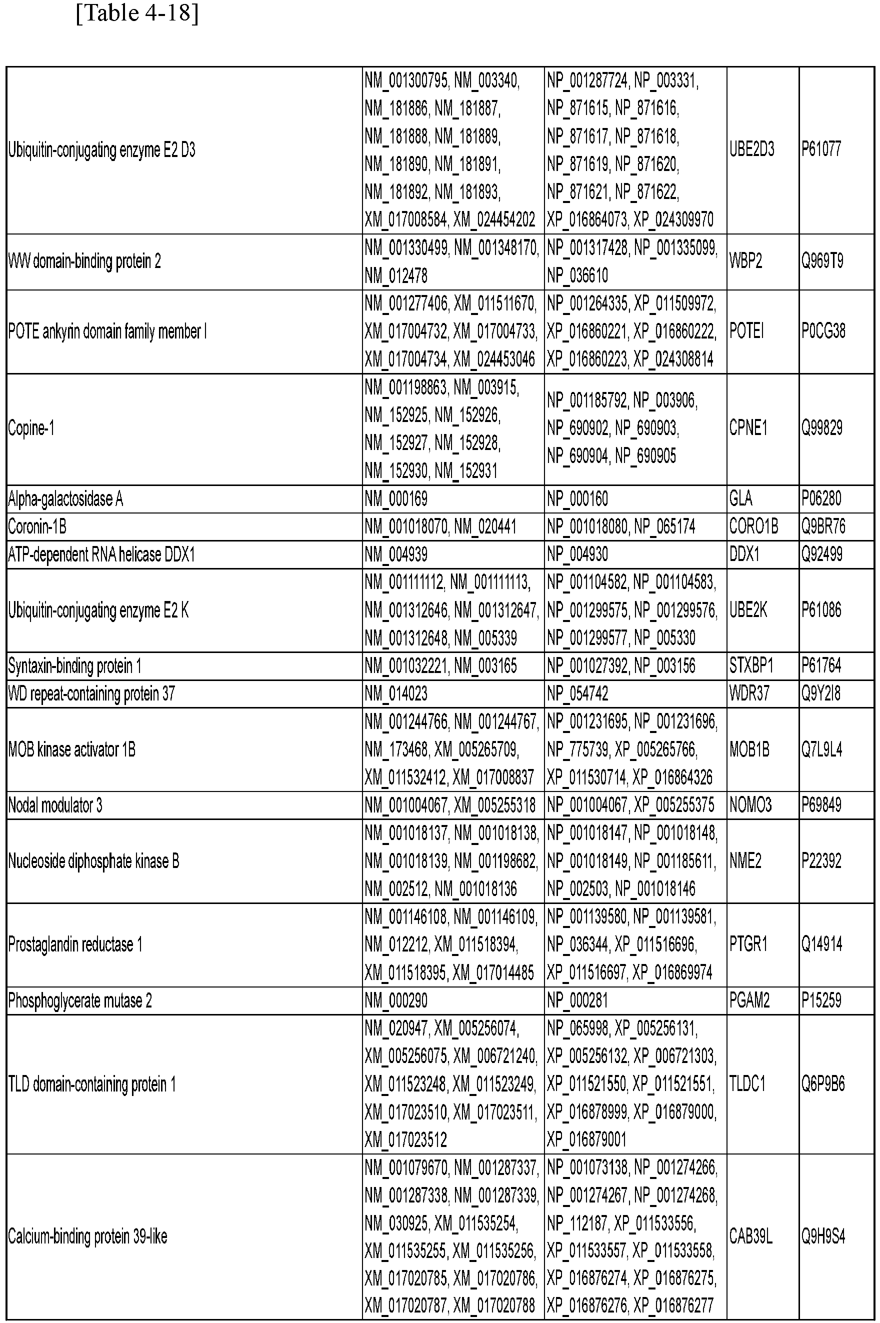

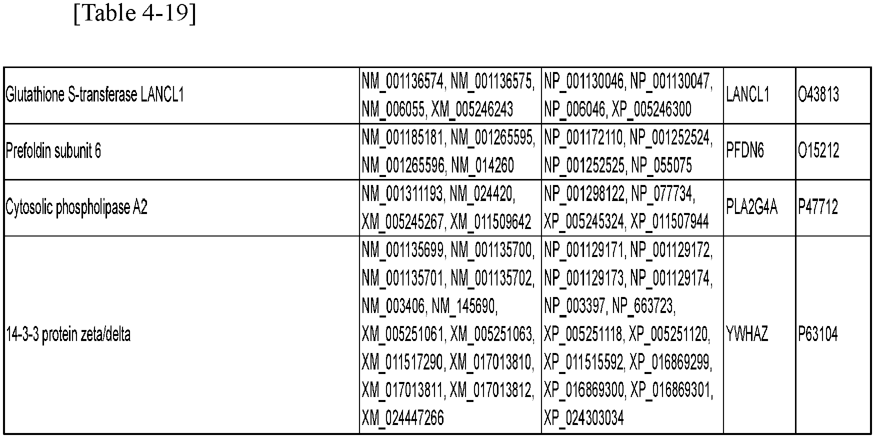

Among the protein groups shown in Table 4-1 to Table 4-19 described above, for example, it is possible to preferably use one or more membrane proteins selected from the protein groups shown in Table 2 described above in the method of the present embodiment. As shown in the examples described below, the protein groups shown in Table 2 above are groups formed of 36 membrane proteins with an AUC value of the ROC curve, which is an indicator of discriminatory ability in determining drug sensitivity, of 0.8 or higher.

In addition, among the protein groups shown in Table 4-1 to Table 4-19 described above, for example, it is possible to preferably use one or more proteins selected from the protein groups shown in Table 3 described above in the method of the present embodiment. As shown in the examples described below, the protein group shown in Table 3 described above is a group consisting of 11 proteins with an AUC value of the ROC curve, which is an indicator of discriminatory ability in determining drug sensitivity, of 0.8 or higher and for which, for patients having drug sensitivity (responders), false negatives are not produced based on a threshold calculated by Youden index.

A detailed description will be given below of the method of the present embodiment.

Examples of the biological samples used in the method of the present embodiment include various fluids, such as body fluids, cell culture supernatants, and crushed tissue cell fluids. Among the above, body fluids or cell culture supernatants are preferable. In addition to whole blood, serum, plasma, various blood cells, blood clots, platelets, and the like, examples of body fluids include urine, semen, breast milk, sweat, interstitial fluid, interstitial lymph fluid, bone marrow fluid, tissue fluid, saliva, gastric fluid, joint fluid, pleural fluid, bile, ascites fluid, amniotic fluid, and the like. Among the above, plasma is preferable. Plasma may be treated with anticoagulants such as citric acid, heparin, and EDTA.

Biological samples may be pre-treated, such as by being added to a known buffer, and it is also possible to use samples ingested from a living organism as they are.

It is possible to appropriately adjust the amount of the sample, for example, the amount may be 1 µL or more and 1000 µL or less or, for example, the amount may be 1 µL or more and 500 µL or less.

Examples of methods for isolating exosomes from biological samples include a PEG precipitation method, an isolation method using an ultracentrifuge or the like, an immunoprecipitation method using molecules for exosome capture, and the like, without being limited thereto. Among the above, the immunoprecipitation method using molecules for exosome capture is convenient and enables the selective separation of exosomes without performing any pre-treatment and is thus preferable.

The molecules for exosome capture are formed of specific binding substances for antigens expressed on the exosome surface.

Examples of antigens expressed on the exosome surface include CD9, CD63, and CD81, without being limited thereto.

The molecules for exosome capture are formed of specific binding substances for these antigens.

Examples of specific binding substances include substances that bind specifically to the antigens. Examples of specific binding substances to antigens include antibodies and aptamers.

The antibodies may be any having a binding ability to antigenic sequences (epitopes) on the exosome surface and examples thereof include anti-CD9 antibodies, anti-CD63 antibodies, and anti-CD81 antibodies. These antibodies may be monoclonal antibodies or may be polyclonal antibodies, but monoclonal antibodies are preferable. In addition, it is also possible to use a mixture of a plurality of antibodies. Furthermore, these antibodies may be bispecific antibodies. Examples of the classes of antibodies include IgG and IgM and IgG is preferable. In addition, the antibodies may also be chimeric antibodies, such as humanized antibodies. Antibodies may be derived from animal species such as mammals such as humans, mice, rabbits, cattle, pigs, horses, sheep, goats, and the like, or birds such as chickens, without being particularly limited. Antibodies may, for example, be prepared from serum derived from the animal species described above by methods known in the related art, antibodies synthesized from known antibody gene sequence information may be used, or commercially available antibodies may be used.

It is also possible to use antibody fragments instead of antibodies. Examples of antibody fragments include Fv, Fab, Fab', F(ab')2, rIgG, and scFv.

Aptamers are substances having a specific binding ability with respect to a target substance. Examples of aptamers include nucleic acid aptamers, peptide aptamers, and the like. It is possible to sort nucleic acid aptamers having a specific binding ability to antigens, for example, by a systematic evolution of ligands by exponential enrichment (SELEX) method. In addition, it is possible to sort peptide aptamers having a specific binding ability to antigens, for example, by the Two-Hybrid method using yeast.

The molecules for exosome capture are preferably fixed on a solid-phase carrier.

Examples of the substances of solid-phase carriers include polymer compounds such as polystyrenes, polyethylenes, polypropylenes, polyesters, poly(meth)acrylonitrile, styrene-butadiene copolymers, poly(meth)acrylic esters, fluorine resins, cross-linked dextrans, and polysaccharides; glass; metals; magnetic materials; resin compositions including magnetic materials; and combinations thereof.

In addition, the shape of the solid-phase carrier is not particularly limited and examples thereof include tray shapes, sphere shapes, particle shapes, fiber shapes, rod shapes, plate shapes, container shapes, cells, microplates, microfluidic devices, and test tubes.

In this step, magnetic particles are preferable.

Examples of the magnetic particles described above include metals such as iron tetroxide (Fe₃O₄), iron sesquioxide (γ-Fe₂O₃), various ferrites, iron, manganese, nickel, cobalt, and chromium; magnetic material fine particles formed of alloys of cobalt, nickel, manganese, and the like; and magnetic particles including these magnetic materials in a resin. Examples of the resins include hydrophobic polymers, hydrophilic polymers, and the like.

Among the above, magnetic particles including magnetic materials in resin are preferable and magnetic particles in which a polymer layer is formed on the surface of a mother particle including superparamagnetic fine particles are more preferable. Examples thereof include magnetic particles in which, as described in Japanese Unexamined Patent Application, First Publication No. 2008-32411 (Patent Document 1), a hydrophobic first polymer layer is formed on the surface of a mother particle including superparamagnetic fine particles, a second polymer layer having at least a glycidyl group on the surface thereof is formed on the first polymer layer, and polar groups are introduced by chemical modification of the glycidyl group.

Here, as superparamagnetic fine particles, iron oxide-based fine particles with a particle diameter of 20 nm or less (preferably a particle diameter of 5 nm or more and 20 nm or less) are representative and examples thereof include ferrite expressed as XFe₂O₄ (X = M n, Co, Ni, Mg, Cu, Li_{0.5}Fe_{0.5}, and the like), magnetite expressed as Fe₃O₄, and γ Fe₂O₃, and, in terms of strong saturation magnetization and low residual magnetization, including either γ-Fe₂O₃ or Fe₃O₄, is preferable.

In addition, the monomers for forming the hydrophobic first polymer layer described above are broadly classified as monofunctional monomers and cross-linking monomers.

Examples of the monofunctional monomers described above include aromatic vinyl monomers such as styrene, α-methylstyrene, and halogenated styrene; and ethylenically unsaturated carboxylic acid alkyl ester monomers such as methyl acrylate, methyl methacrylate, ethyl acrylate, ethyl methacrylate, stearyl acrylate, stearyl methacrylate, cyclohexyl acrylate, cyclohexyl methacrylate, isobonyl acrylate, and isobonyl methacrylate.

In addition, examples of the cross-linking monomers described above include polyfunctional (meth)acrylates such as ethylene glycol diacrylate, ethylene glycol dimethacrylate, trimethylolpropane triacrylate, trimethylolpropane trimethacrylate, pentaerythritol triacrylate, pentaerythritol trimethacrylate, dipentaerythritol hexaacrylate, and dipentaerythritol hexamethacrylate; conjugated diolefins such as butadiene and iprene, as well as divinylbenzene, diarylphthalate, allyl acrylate, and allyl methacrylate.

In addition, the monomers for forming the second polymer layer described above are mainly intended for introducing functional groups to the particle surface and include glycidyl group-containing monomers. As for the content of the glycidyl group-containing monomer, 20% by mass or more in the monomer for forming the second polymer layer is preferable. Here, examples of copolymerizable monomers including glycidyl groups include glycidyl acrylate, glycidyl methacrylate, and allyl glycidyl ether.

The polar group introduced by chemical modification of the glycidyl group in the second polymer layer is preferably a functional group capable of reacting with specific binding substances for antigens expressed on the surface of cancer cells and is more preferably a polar group including at least one or more atoms selected from the group consisting of oxygen atoms, nitrogen atoms, and sulfur atoms. Among the above, amino groups, aldehyde groups, carboxy groups, or active ester groups are more preferable. In particular, in a case where the second polymer layer of the magnetic particles has the polar group and 2,3-dihydroxypropyl group, the binding property to specific binding substances for antigens expressed on the exosome surface (in particular, antibodies for antigens expressed on the exosome surface) is favorable.

As methods for binding specific binding substances for antigens expressed on the exosome surface to solid-phase carriers, it is possible to use physical adsorption methods, chemical binding methods such as covalent binding methods and ionic binding methods, and the like. Examples of physical adsorption methods include a method for directly fixing the specific binding substance for the antigen expressed on the exosome surface on the solid-phase carrier, a method for chemically binding to another protein such as albumin and then fixing by adsorption, and the like. Examples of chemical binding methods include a method for direct binding on a solid-phase carrier using a functional group introduced into the surface of the solid-phase carrier which is able to react with the specific binding substance for the antigen expressed on the exosome surface, a method for introducing and binding spacer molecules (such as a carbodiimide compound) between the solid-phase carrier and the specific binding substance for the antigen expressed on the exosome surface by chemical binding, a method for binding a specific binding substance for the antigen expressed on the exosome surface to another protein such as albumin and then carrying out chemical binding of the protein to the solid-phase carrier, and the like.

Immunoprecipitation methods using molecules for exosome capture may be performed using salts, proteins such as albumin, surfactants, and the like as necessary, in addition to each of the components described above.

The reaction temperature in the immunoprecipitation method using molecules for exosome capture is usually 2°C or higher and 42°C or lower and the reaction time is usually 5 minutes or more and 24 hours or less. In a case where the reaction is performed at 2°C or higher and 8°C or lower, the reaction time is preferably 8 hours or more and 30 hours or less and, in a case where the reaction is carried out at 8°C or higher and 42°C or lower, the reaction time is preferably 5 minutes or more and 24 hours or less.

In the immunoprecipitation method using molecules for exosome capture, the pH in the system is not particularly limited, but is preferably in a range of pH 5 or more and 10 or less and more preferably in a range of pH 6 or more and 8 or less. To maintain the desired pH, buffer solutions are usually used, for example, a phosphate buffer solution, a tris(hydroxymethyl) aminomethane buffer solution, a HEPES buffer solution, and an MES buffer solution may be used.

In addition, in a case where one or more types of solid-phase carrier with fixed molecules for exosome capture are used, the usage amount and the mixing ratio are not particularly limited, but 0.00001 to 10 w/v% with respect to the total amount of the liquid phase in the system is preferable, 0.00001 to 5 w/v% is more preferable, and 0.001 to 0.1 w/v% is even more preferable.

Next, a mass spectrometry method is used to determine the expression level of the proteins present in the isolated exosomes.

The proteins for which the expression levels are to be determined are one or more proteins selected from the group consisting of the groups of proteins shown in Table 4-1 to Table 4-19 described above.

In a case where a mass spectrometry method is used, a pre-treatment may be performed to separate the substances present in the exosomes before mass spectrometry. It is possible to use various chromatographic separation techniques as separation methods, more specifically, examples thereof include liquid chromatography (LC), high-performance liquid chromatography (HPLC), gas chromatography (GC), thin layer chromatography, and size exclusion chromatography, without being limited thereto. Among the above, liquid chromatography or high-performance liquid chromatography is preferable.

Examples of mass spectrometry methods include a tandem quadrupole mass spectrometry (MS/MS) method, a direct injection mass spectrometry method, a Fourier transform ion cyclotron resonance mass spectrometry (FT-ICR-MS) method, a capillary electrophoresis mass spectrometry (CE-MS) method, an inductively coupled plasma mass spectrometry (ICP-MS) method, a pyrolysis mass spectroscopy (Py-MS) method, an ion mobility mass spectrometry method, a time-of-flight mass spectrometry (TOF MS) method, and the like.

Among the above, as a mass spectrometry method, a mass spectrometry method coupled with the separation methods described above is preferable and examples thereof include a GC-MS method, a LC-MS method, a HPLC-MS method, a LC-MS/MS method, and a HPLC-MS/MS method, without being limited thereto.

It is possible to determine protein expression levels, for example, by analysis using a DIA (data-independent acquisition) method in a mass spectrometry method. The DIA method is a data-independent analysis method, which is an exhaustive analysis method that analyzes all ions included in a certain mass range by MS/MS analysis. Among the above, the SWATH method, which is one DIA method, for example, repeats an operation of acquiring MS/MS 10 times while shifting a 100 Da width window and records the MS/MS spectra of all ions within the total 1,000 Da width (the window width and number of acquisitions are variable).

The method of the present embodiment predicts the efficacy of treatment using an immune checkpoint inhibitor for lung cancer patients from whom the biological sample which is the evaluation target is derived, based on the determined protein expression levels.

Specifically, in a case where the protein expression level is a predetermined reference value or less, it is possible to evaluate or predict that the lung cancer patient from whom the biological sample which is the evaluation target is derived is potentially sensitive to the immune checkpoint inhibitor, that is, that the treatment with the immune checkpoint inhibitor is potentially effective. On the other hand, in a case where the protein expression level is over a predetermined reference value, it is possible to evaluate or predict that the lung cancer patient from whom the biological sample which is the evaluation target is derived is potentially not sensitive to the immune checkpoint inhibitors, that is, that the treatment with the immune checkpoint inhibitor is potentially not effective. The reference value is for distinguishing between a group of lung cancer patients who are sensitive to immune checkpoint inhibitors (responder group) and a group of lung cancer patients who are not sensitive to immune checkpoint inhibitors (non-responder group).

It is possible to experimentally determine the reference value, for example, as a threshold value able to distinguish between the two groups by measuring the expression levels of a specific protein present in the exosomes of the responder groups and non-responder groups. The method for determining the reference value for the expression level of a specific protein in the method of the present embodiment is not particularly limited and determination is possible, for example, using general statistical methods. Here, the specific protein means one or more proteins selected from the group of proteins shown in Tables 4-1 to 4-19 above.

Specifically, as a method for determining the reference value, for example, the expression levels of a specific protein present in exosomes in biological samples collected in advance before administration of an immune checkpoint inhibitor are measured for patients diagnosed as having lung cancer by a commonly performed method such as imaging diagnosis. After measurement for a plurality of patients, the expression levels of the specific proteins present in the exosomes in these biological samples are calculated from the mean values, the median values, or the like and it is possible to use values included therein as reference values. In addition, it is also preferable that the reference value is described in the instructions or the like of the kit relating to the evaluation or prediction method.

In addition, for a plurality of responder groups and a plurality of non-responder groups, the expression levels of a specific protein present in exosomes in biological samples collected in advance before administration of the immune checkpoint inhibitor are measured, respectively, and, from the mean values, the median values, or the like, the expression levels of the specific protein present in exosomes in biological samples of the responder group and non-responder group and variations thereof are each calculated, then, a threshold value able to distinguish between the two values, also taking into account variations, is determined and it is possible to use this as the reference value.

### <Other Embodiments>

In one embodiment, the present invention provides a biomarker for predicting or diagnosing the efficacy of treatment of a lung cancer patient using an immune checkpoint inhibitor, which is formed of one or more proteins selected from the protein groups shown in Tables 1-1 to 1-6 described above.

In one embodiment, the present invention provides a biomarker for predicting or diagnosing the efficacy of treatment of a lung cancer patient using an immune checkpoint inhibitor, which is formed of one or more membrane proteins selected from the group of proteins shown in Table 2 described above.

In one embodiment, the present invention provides a biomarker for predicting or diagnosing the efficacy of treatment of a lung cancer patient using an immune checkpoint inhibitor, which is formed of one or more proteins selected from the group of proteins shown in Table 3 described above.

In one embodiment, the present invention provides a method for diagnosing the efficacy of treatment of a lung cancer patient using an immune checkpoint inhibitor, that is, a method for companion diagnosis using an immune checkpoint inhibitor for a lung cancer patient, the method including isolating exosomes from a biological sample derived from the lung cancer patient, and determining an expression level of a protein present in the exosomes by a mass spectrometry method, in which the protein is one or more proteins selected from the groups of proteins shown in Table 1-1 to Table 1-6 described above.

In one embodiment, the present invention provides a method for diagnosing the efficacy of treatment of a lung cancer patient using an immune checkpoint inhibitor, that is, a method for companion diagnosis using an immune checkpoint inhibitor for a lung cancer patient, the method including isolating exosomes from a biological sample derived from the lung cancer patient, and determining an expression level of a protein present in the exosomes by a mass spectrometry method, in which the protein is one or more proteins selected from the group of proteins shown in Table 2 above.

In one embodiment, the present invention provides a method for diagnosing the efficacy of treatment of a lung cancer patient using an immune checkpoint inhibitor, that is, a method for companion diagnosis using an immune checkpoint inhibitor for a lung cancer patient, the method including isolating exosomes from a biological sample derived from the lung cancer patient, and determining an expression level of a protein present in the exosomes by a mass spectrometry method, in which the protein is one or more proteins selected from the group of proteins shown in Table 3 described above.

### EXAMPLES

A description will be given below of the present invention using Examples, but the present invention is not limited to the following Examples.

### [Example 1]

### 1. LC-MS Analysis of Exosomes

### (1) Preparation of Biological Samples

EDTA plasma (before administration of nivolumab, Pembrolizumab, or atezolizumab) from 20 Japanese lung cancer patients was used in the following test. For the lung cancer patient group, according to RECIST guidelines, after administration of nivolumab (12 patients), pembrolizumab (6 patients), or atezolizumab (2 patients), 10 patients determined to be partial response (PR) (5 patients administered with nivolumab, 4 patients administered with pembrolizumab, and 1 patient administered with atezolizumab) were set as a responder group and 3 patients determined to be stable (SD) (3 patients administered with nivolumab) and 7 patients determined to be progressive (PD) (4 patients administered with nivolumab, 2 patients administered with pembrolizumab, and 1 patient administered with atezolizumab) were set as a non-responder group.

### (2) Antibody-Bound Particles for Exosome Capture

Exosome-capturing antibodies (Anti-CD9 mAb (MEX001-3 manufactured by MBL Co., Ltd.), Anti-CD63 (LAMP-3) mAb (MEX002-3 manufactured by MBL Co., Ltd.), or Anti-CD81 (TAPA1) mAb (MEX003-3 manufactured by MBL Co., Ltd.)) were bound to magnetic particles (Magnosphere^{™} MS300/Carboxyl manufactured by JSR Life Sciences Corporation) to produce three types of antibody-bound particles. Next, the three types of antibody-bound particles were mixed in equal amounts and a solution with a final particle concentration of 0.06% by mass was used as the antibody-bound particle solution for the following tests.

### (3) Isolation of Exosomes

Freeze-thawed EDTA plasma was filtered through a membrane filter with a pore diameter of 0.22 µM (SLGV033RS manufactured by Merck Millipore) and 0.5 mL thereof was added to a 15 mL low-adsorption tube (MS-52150 manufactured by Sumitomo Bakelite Co., Ltd.). Next, 4.5 mL of TBS was added and diluted 10-fold and 5 mL of the antibody-bound particle solution was added and then stirred overnight at room temperature.

The antibody-bound particles after the reaction were transferred to a 2 mL low-adsorption tube (MS-4220M manufactured by Sumitomo Bakelite Co., Ltd.) and washed four times with a washing solution (ExoCap^{™} Ultracentrifugation/Storage Booster (MEX-USB manufactured by MBL Co., Ltd.). The antibody-bound particles were transferred to a new 2 mL low-adsorption tube and washed twice with TBS. Thereafter, 50 µL of a 2% by mass sodium dodecyl sulfate (SDS) solution was added and shaken for 10 minutes at 25°C. The supernatant after the reaction was added to a 100 K ultrafiltration spin column (OD100 C34 manufactured by PALL), centrifuged at 10,000 x g for 5 minutes and then the filtrate was recovered in a 2 mL low-adsorption tube and used as an exosome extract.

### (4) LC-MS/MS Analysis of Exosome Proteins

25 µL of the exosome extract was transferred to a 1.5 mL tube (0030123328 manufactured by Eppendorf) and an 8-fold volume of cold acetone was added thereto and allowed to stand at -20°C for 2 hours. Next, after centrifugation at 15,000 x g for 15 minutes at 4°C, 20 µL of a 0.5% by mass sodium dodecanoate-containing 100 mM Tris (pH 8.5) solution was added to the precipitate and the protein was dissolved by a sealed ultrasonic crusher. To cleave the S-S bonds of the protein, dithiothreitol (DTT) was added to a final concentration of 10 mM and allowed to react at 50°C for 30 min. To alkylate the cysteine residues, iodoacetamide was added to a final concentration of 30 mM and allowed to react for 30 minutes at room temperature under light-shielding conditions. To stop the reaction, cysteine was added to a final concentration of 60 mM and allowed to stand for 10 minutes at room temperature. Thereafter, 150 µL of 50 mM ammonium bicarbonate was added thereto, then 250 ng of Lys-C (125-05061 manufactured by Fujifilm Wako Pure Chemical Corporation) and 250 ng of trypsin (V5113 manufactured by Promega) were added and reacted overnight at 37°C to break down the protein into peptides. After acidifying the solution by adding 5% by mass of trifluoroacetic acid (TFA) to remove sodium dodecanoate, the result was centrifuged at 15,000 x g for 10 minutes at room temperature and the supernatant was recovered. After desalting using a C18 column, the result was dried and solidified using a centrifugal evaporator, a solution containing 3% by mass acetonitrile-0.1% by mass formic acid was added thereto, the peptides were dissolved in a sample sealed ultrasonic crusher and LC-MS analysis was performed. LC-MS analysis was performed using the Q Exactive^{™} HF-X, manufactured by Thermo Fisher Scientific and Scaffold DIA software to identify proteins and calculate relative quantification values.

### 2. Calculation of Area Under Curve (AUC) of Receiver Operating Characteristic (ROC)

For the proteins analyzed by the Scaffold DIA software in "1. (4)", proteins where the Protein FDR was 1% or less were extracted and normalization of the relative quantification values was performed after excluding the immunoglobulin groups and keratin groups, which were considered to be contaminants. 2406 proteins were identified from lung cancer patients. For these proteins, Scikit-Leam, which is an open-source library of the Python programming language, was used to calculate the AUC as an indicator of discriminatory ability and a method using the Youden index was used to calculate the threshold for discriminating between the two groups. The 825 proteins with AUC values of 0.7 or higher and the AUC values thereof are shown in Table 5-1 to Table 5-54. The amino acid sequence of 'Putative nucleoside diphosphate kinase' in Table 5-19 is shown in Sequence No. 1.

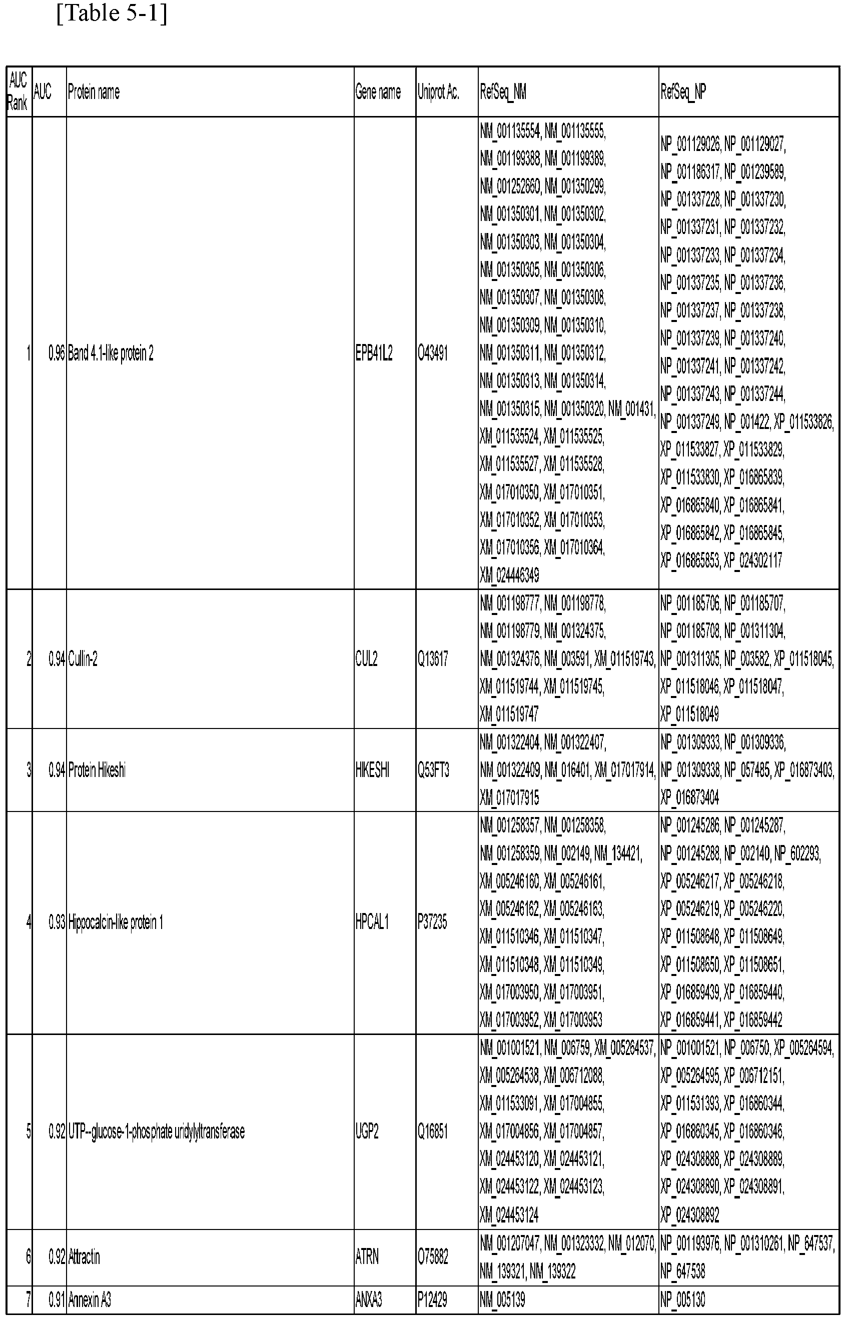

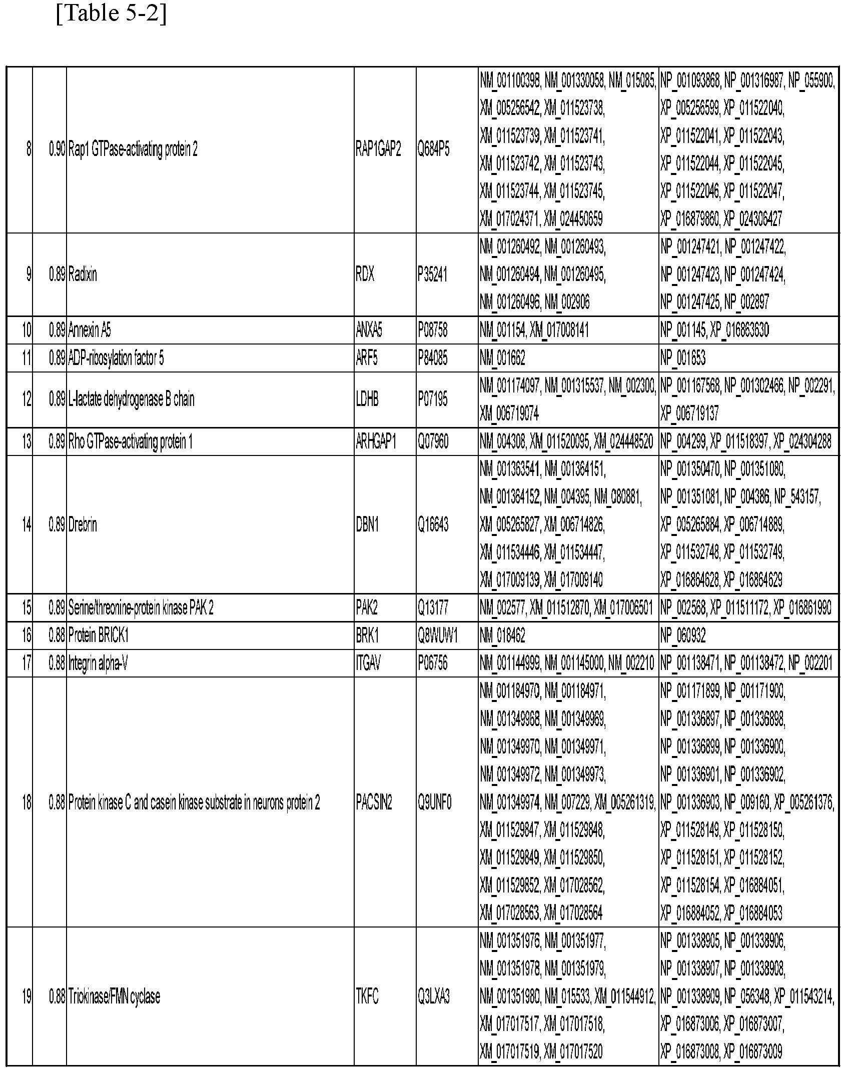

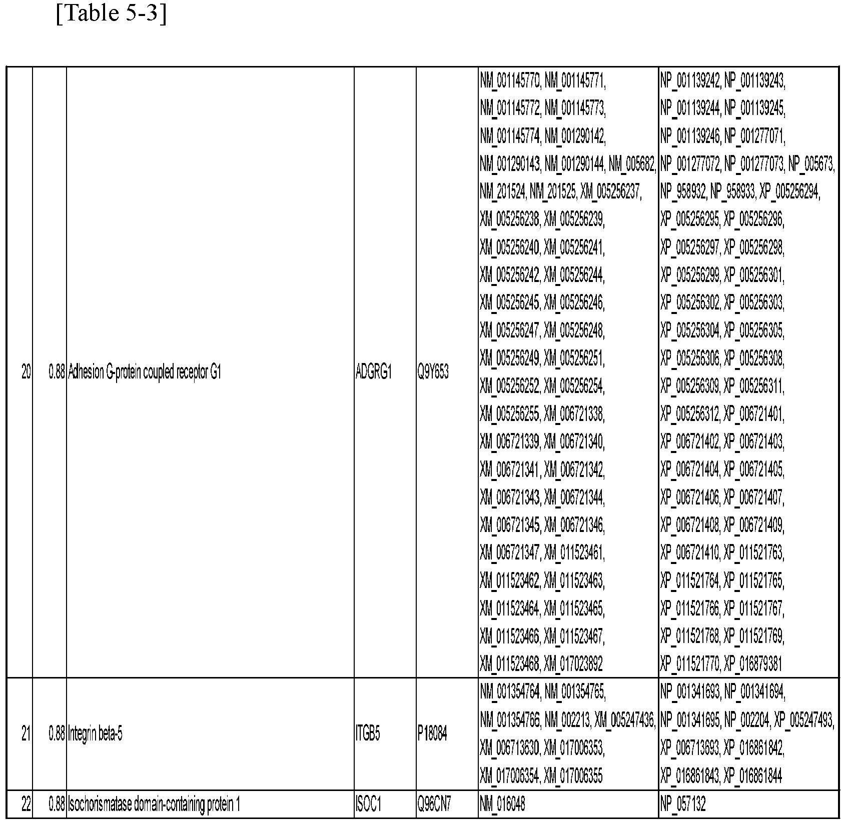

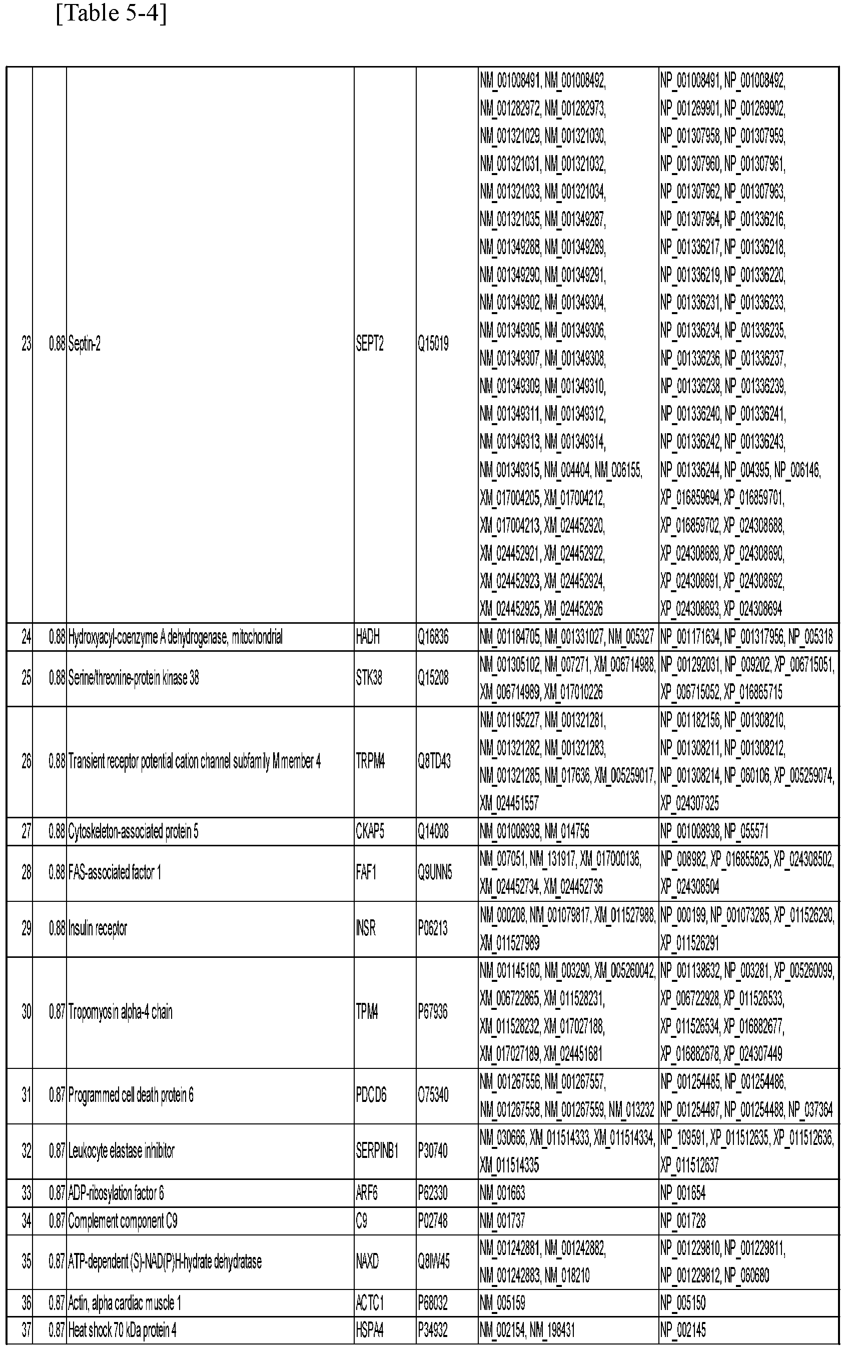

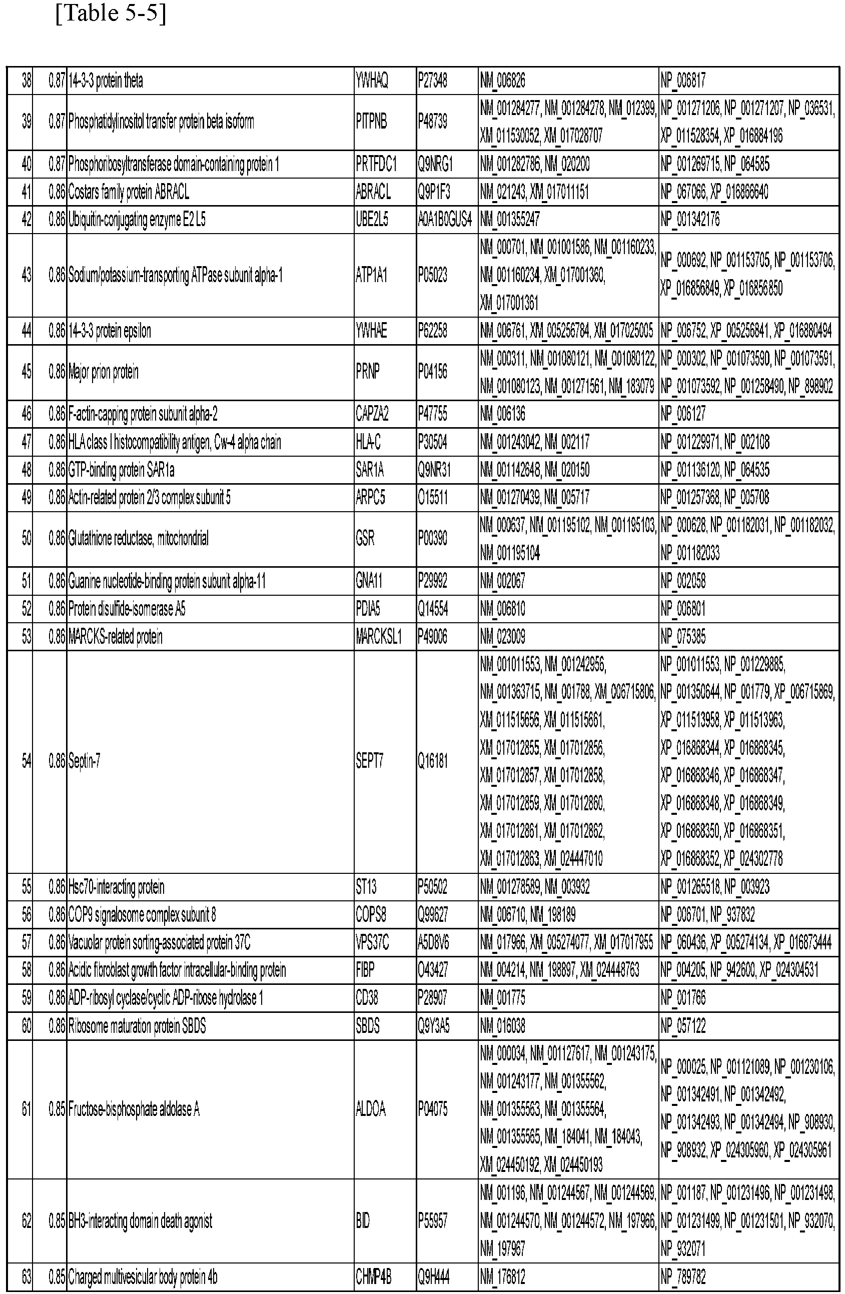

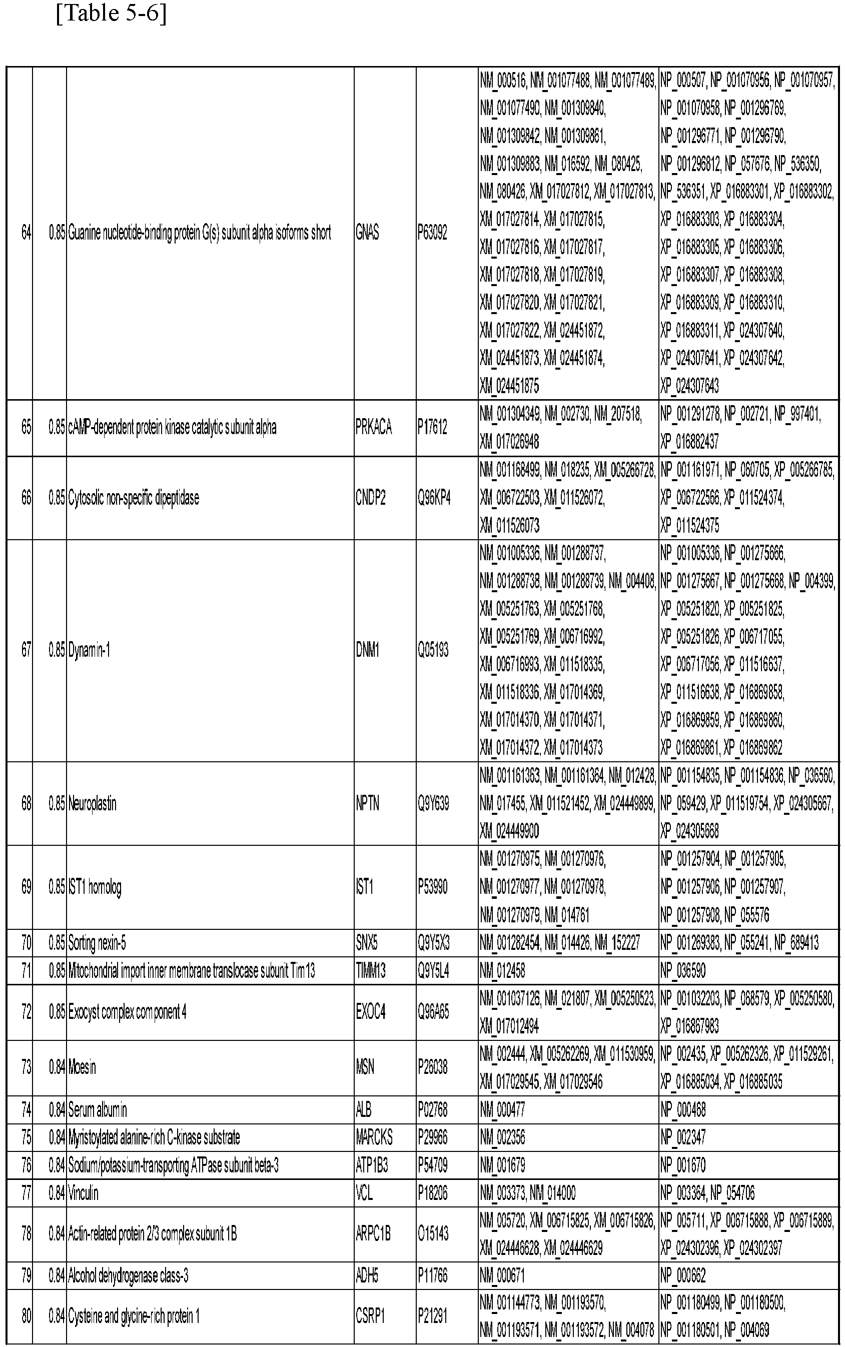

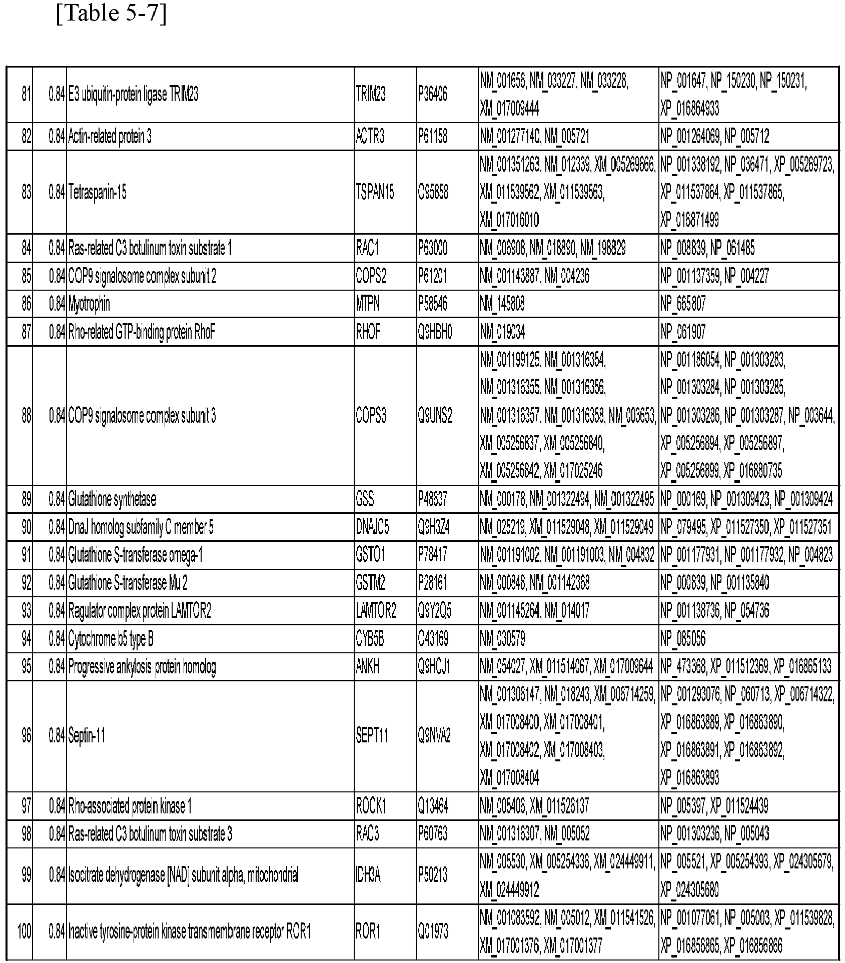

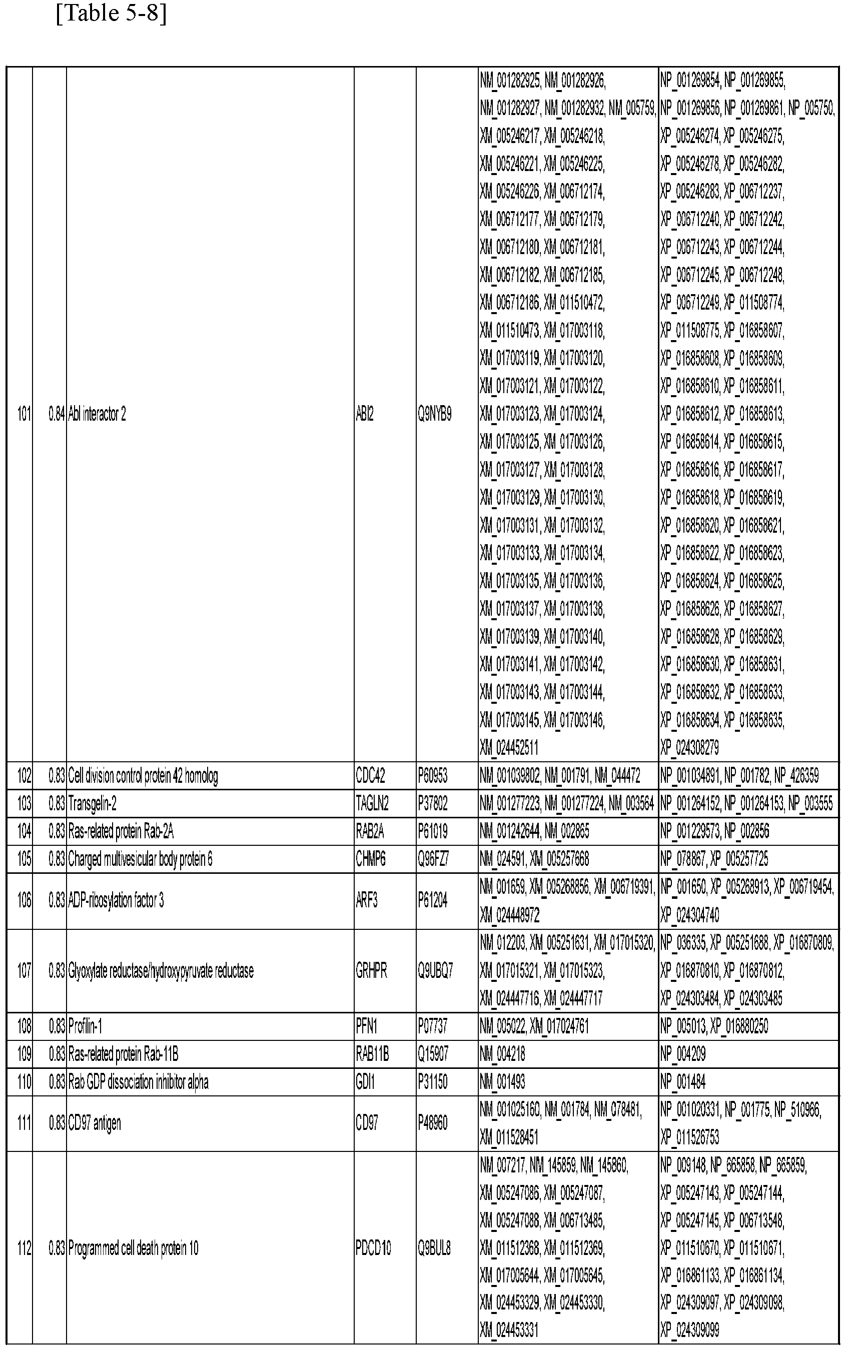

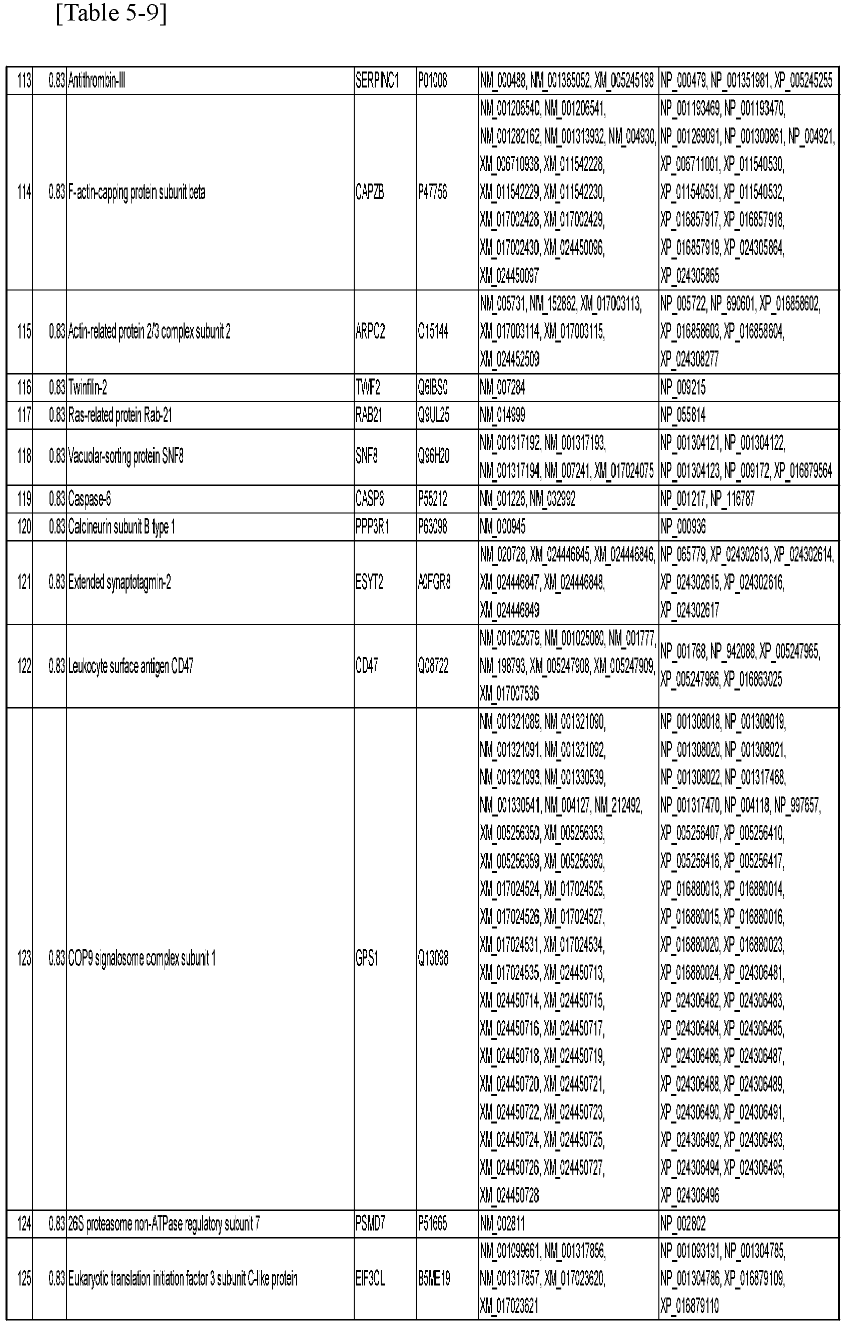

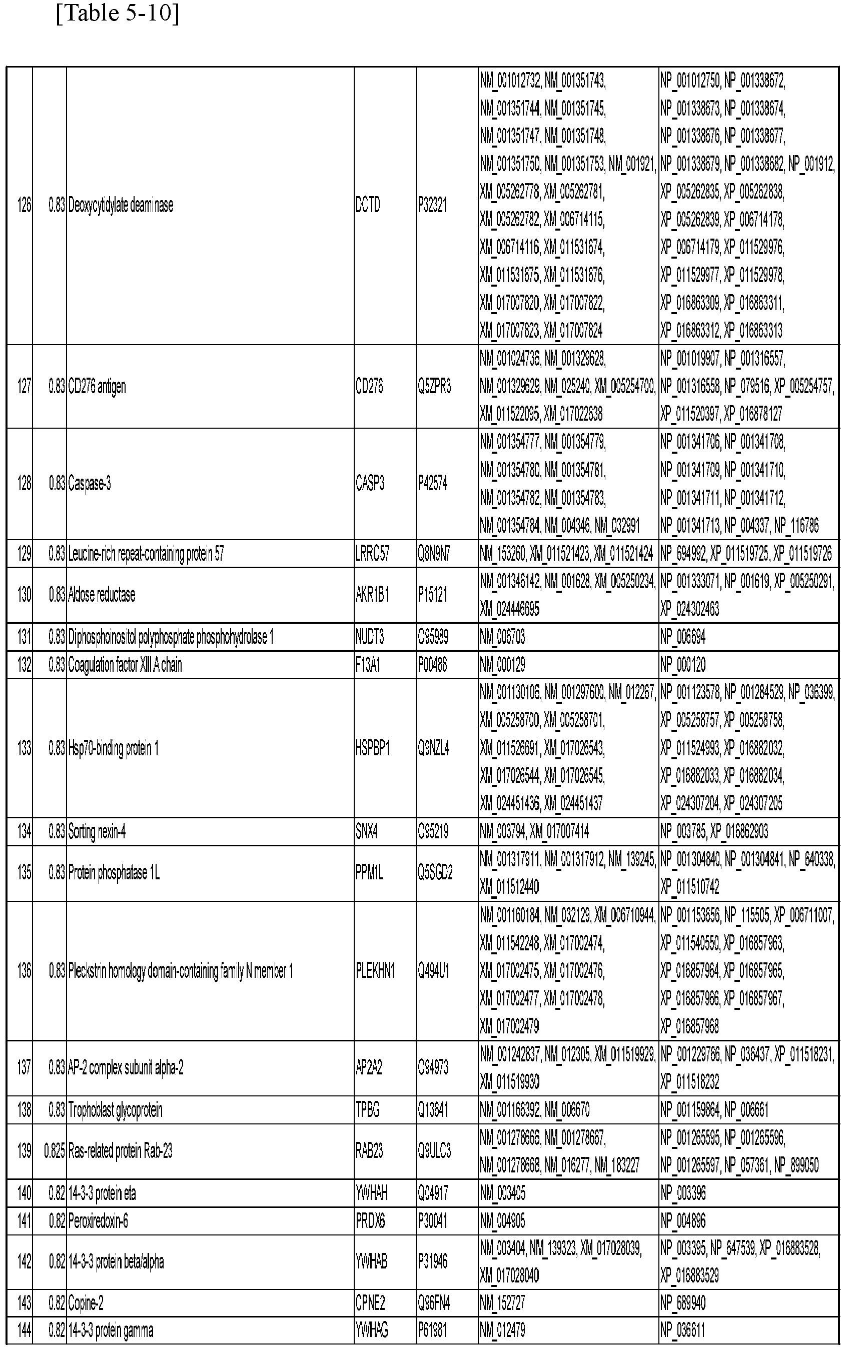

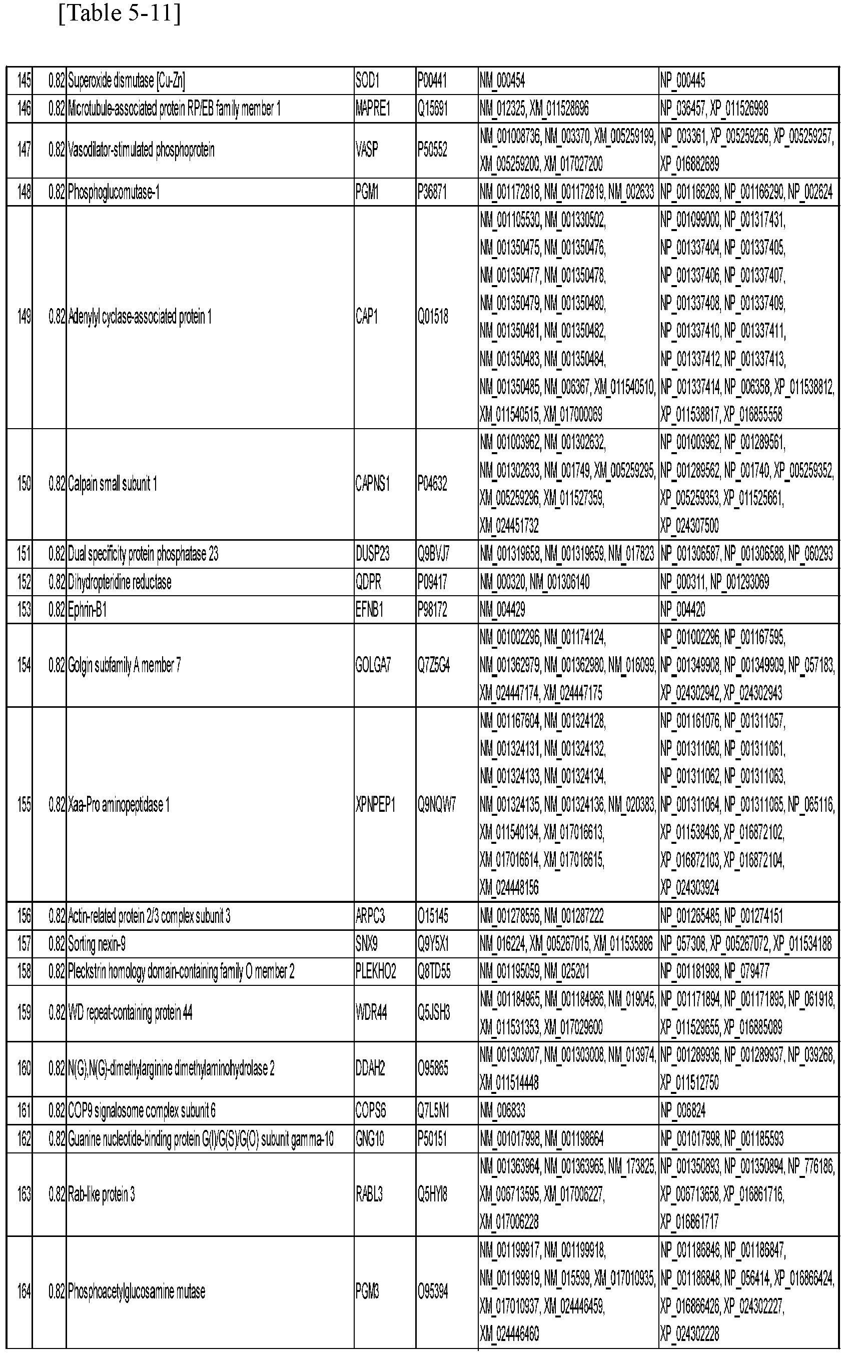

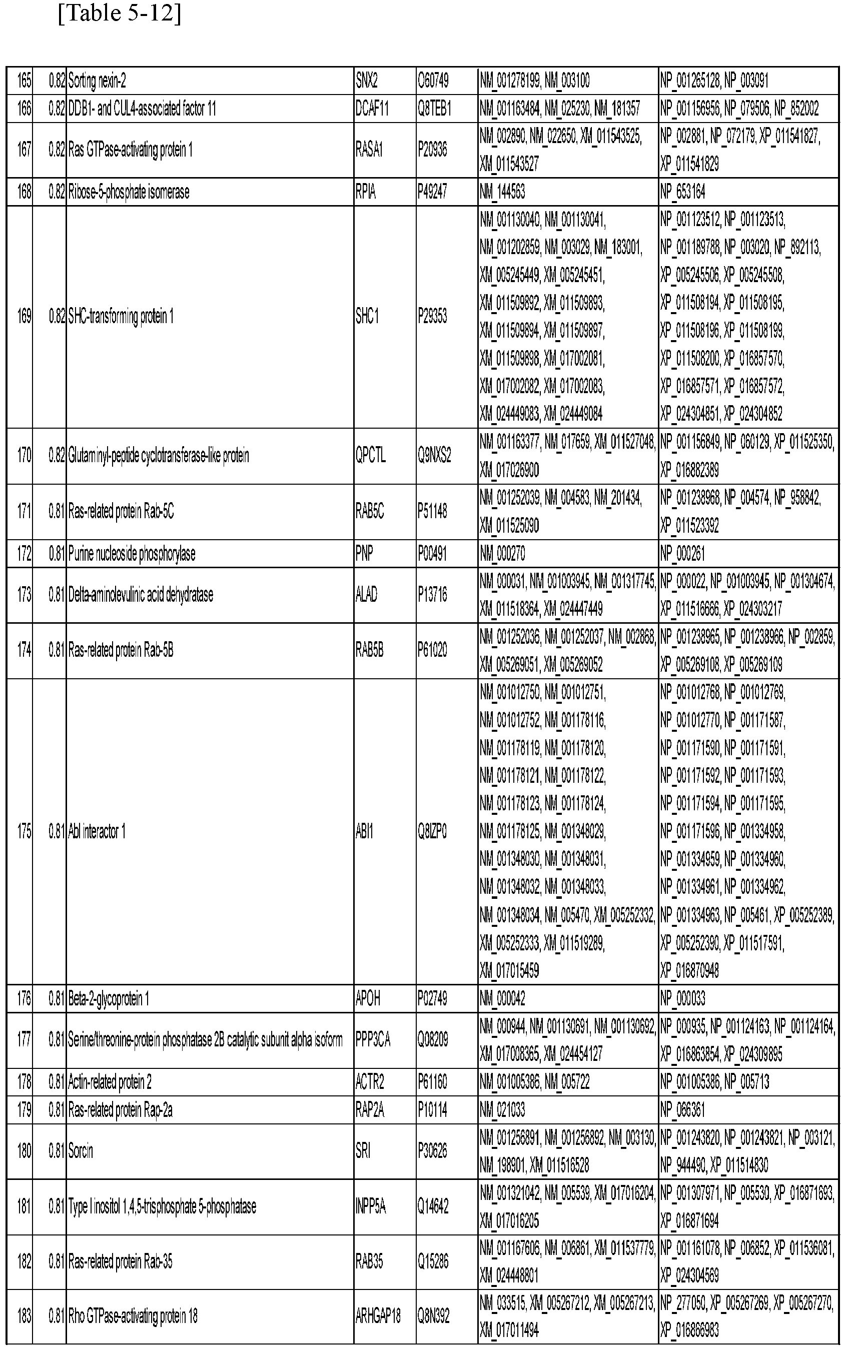

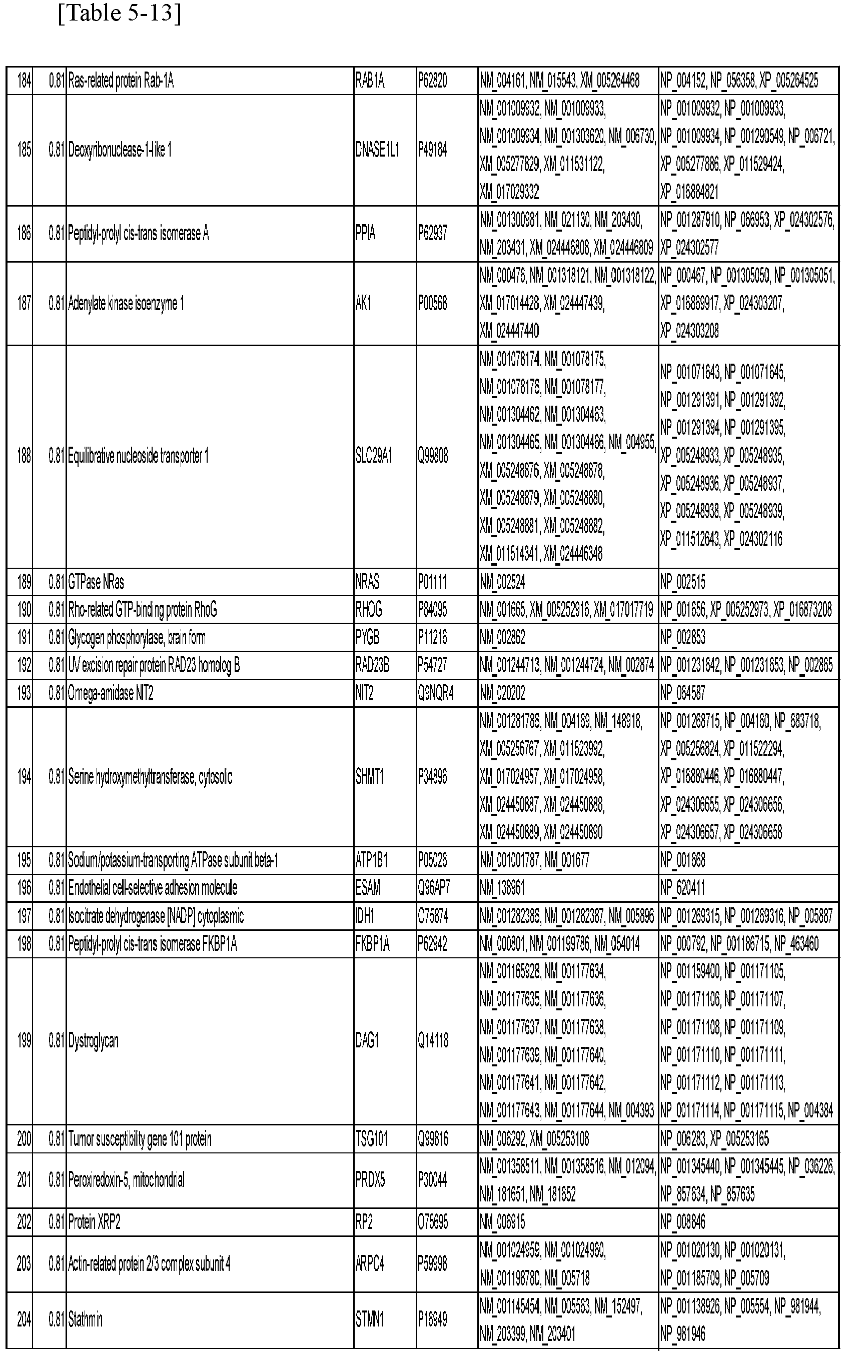

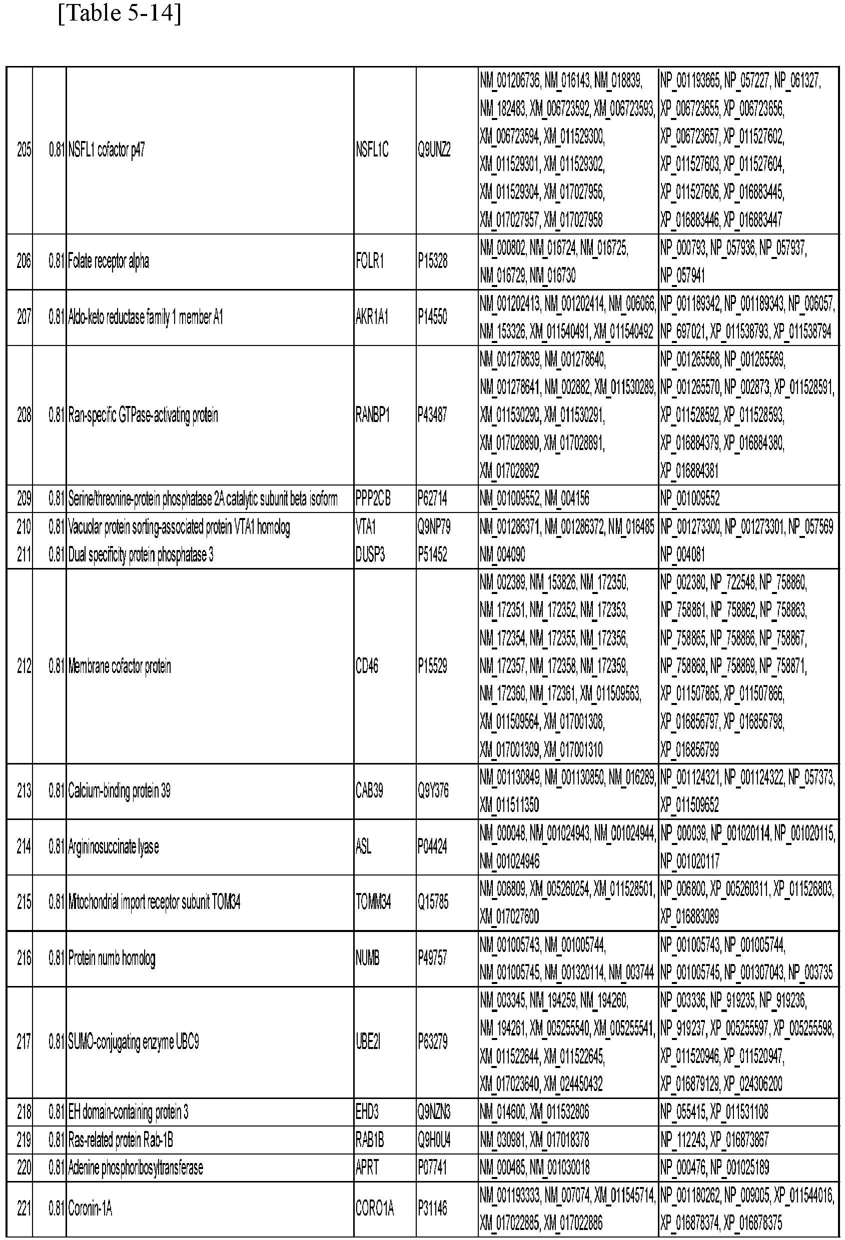

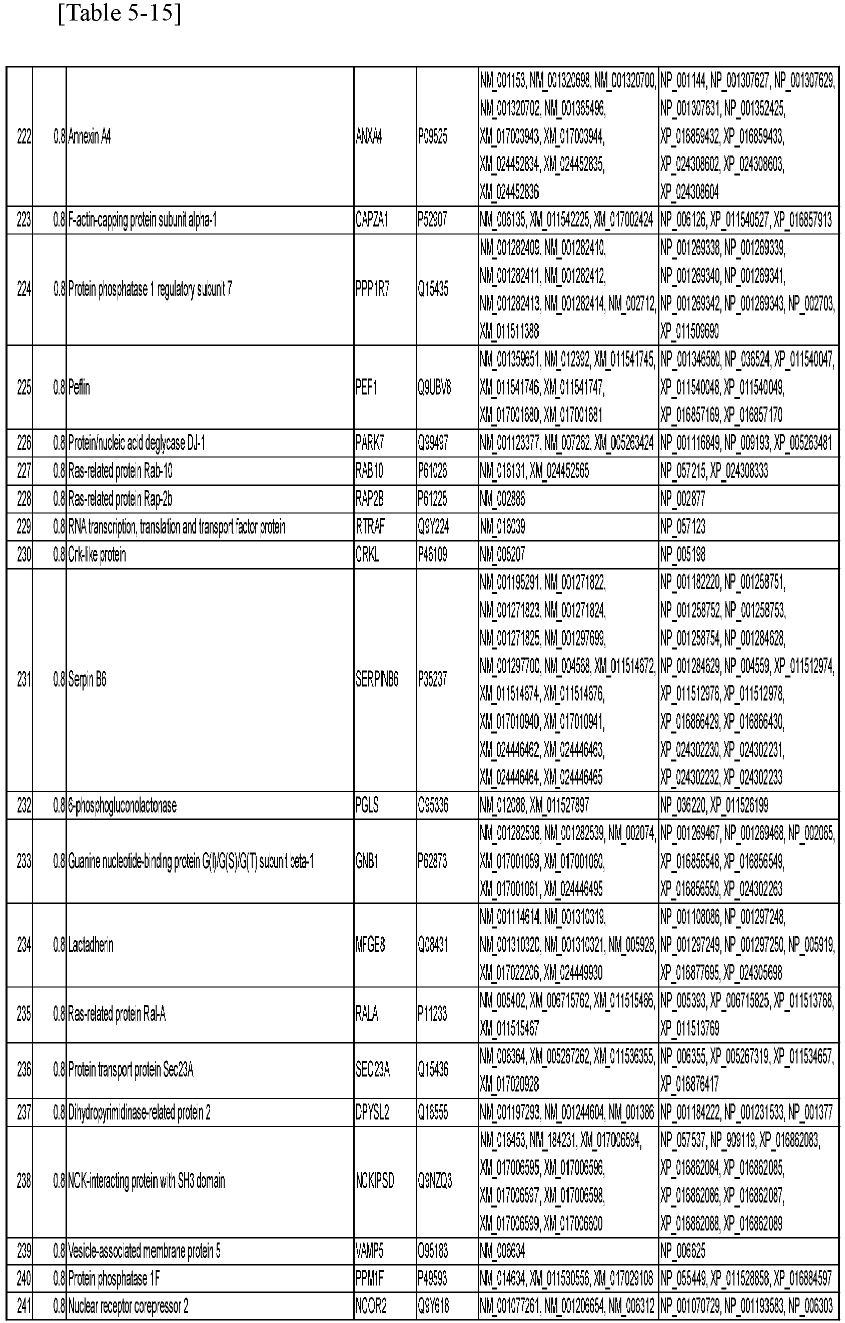

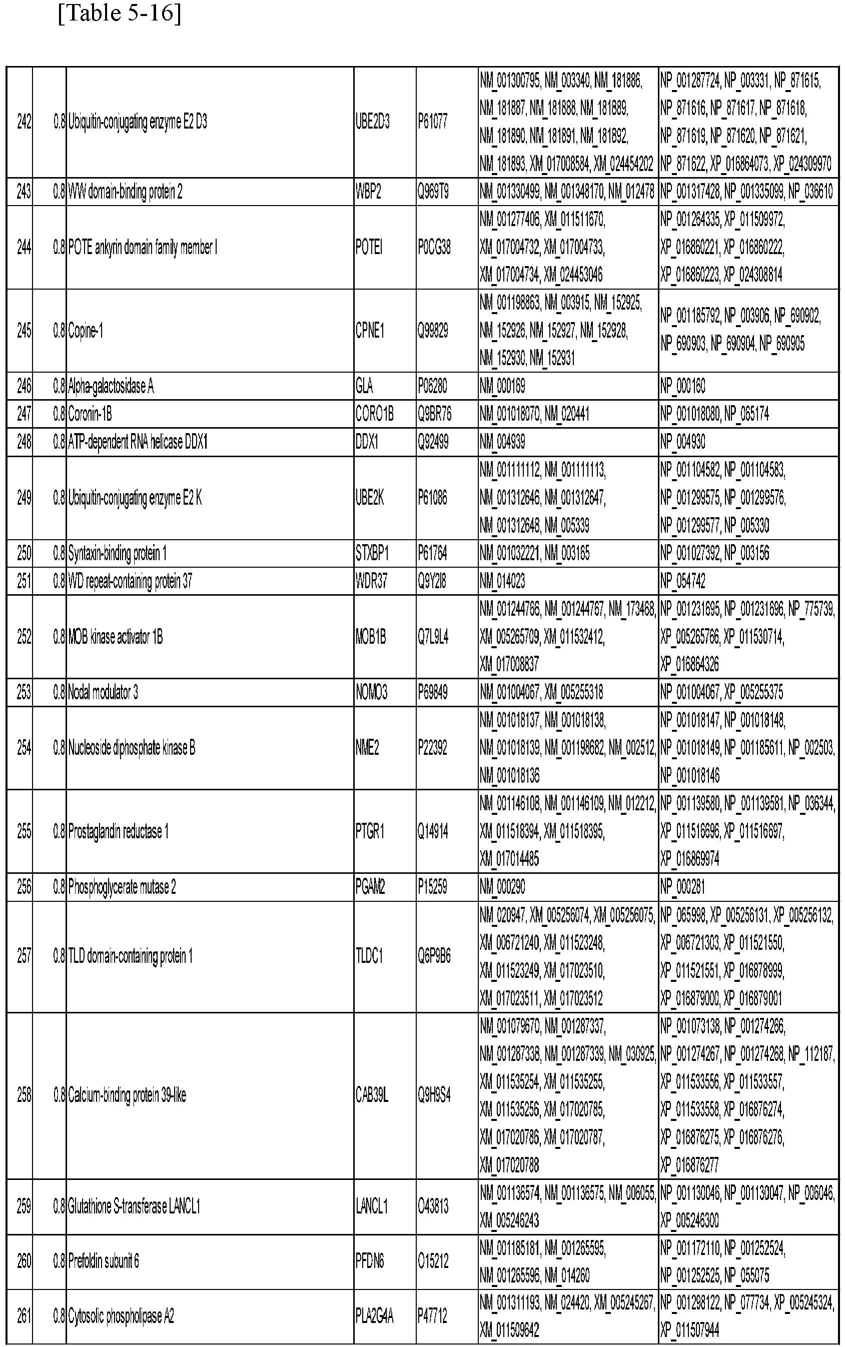

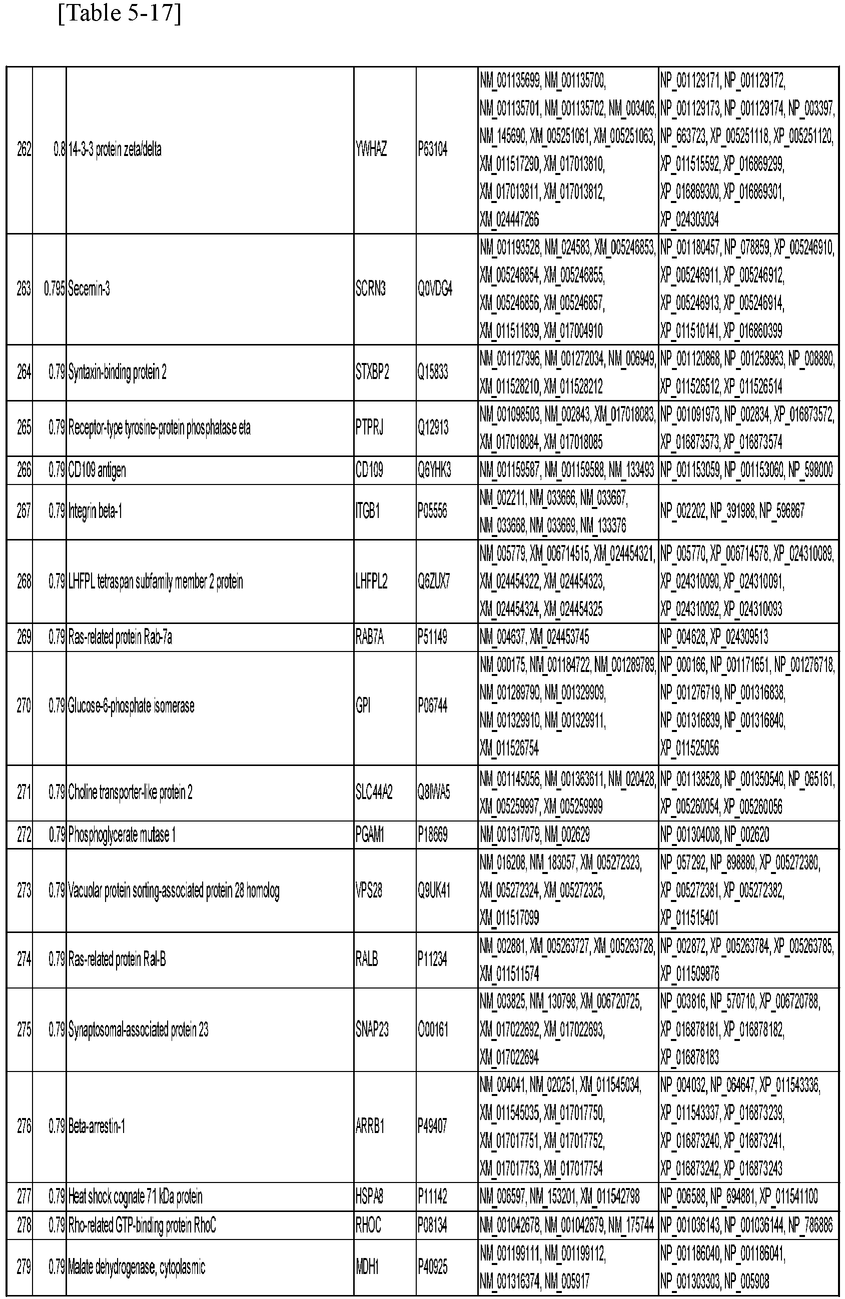

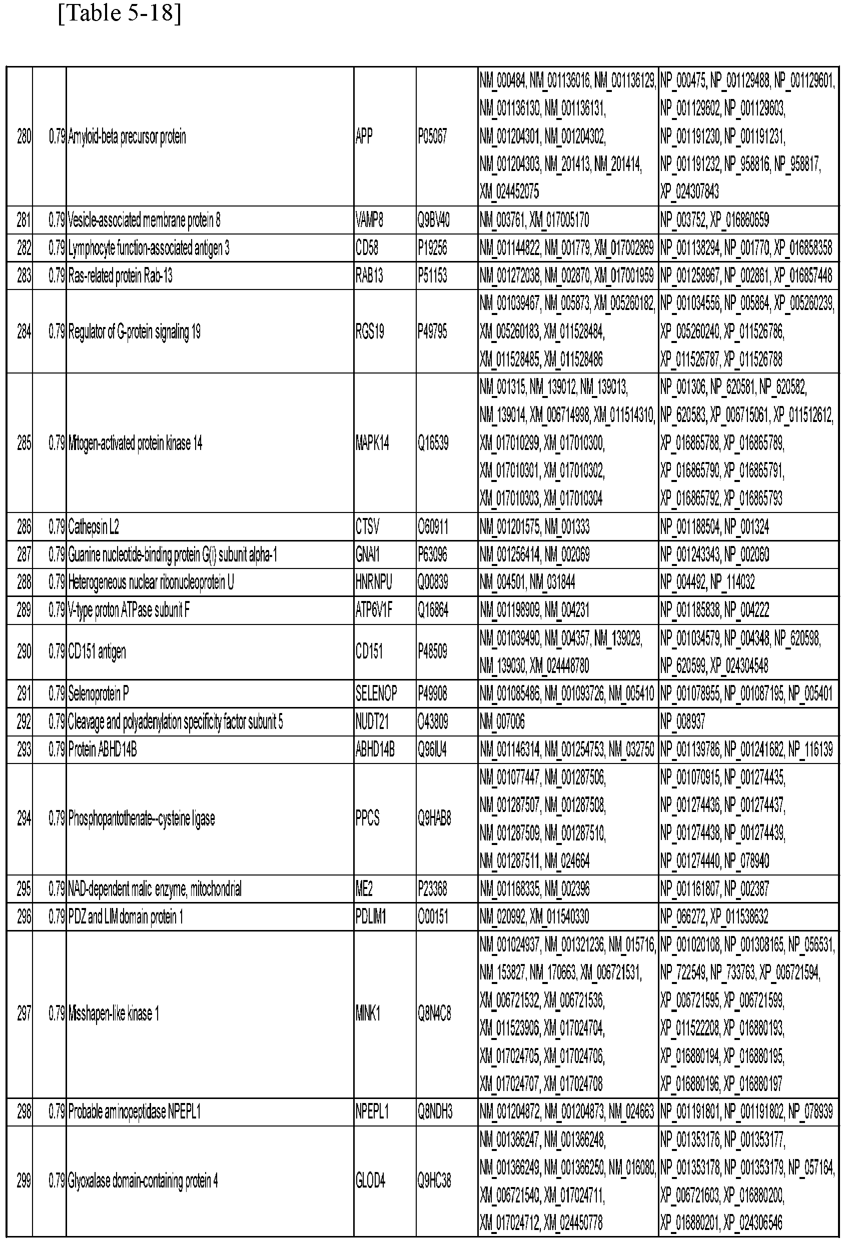

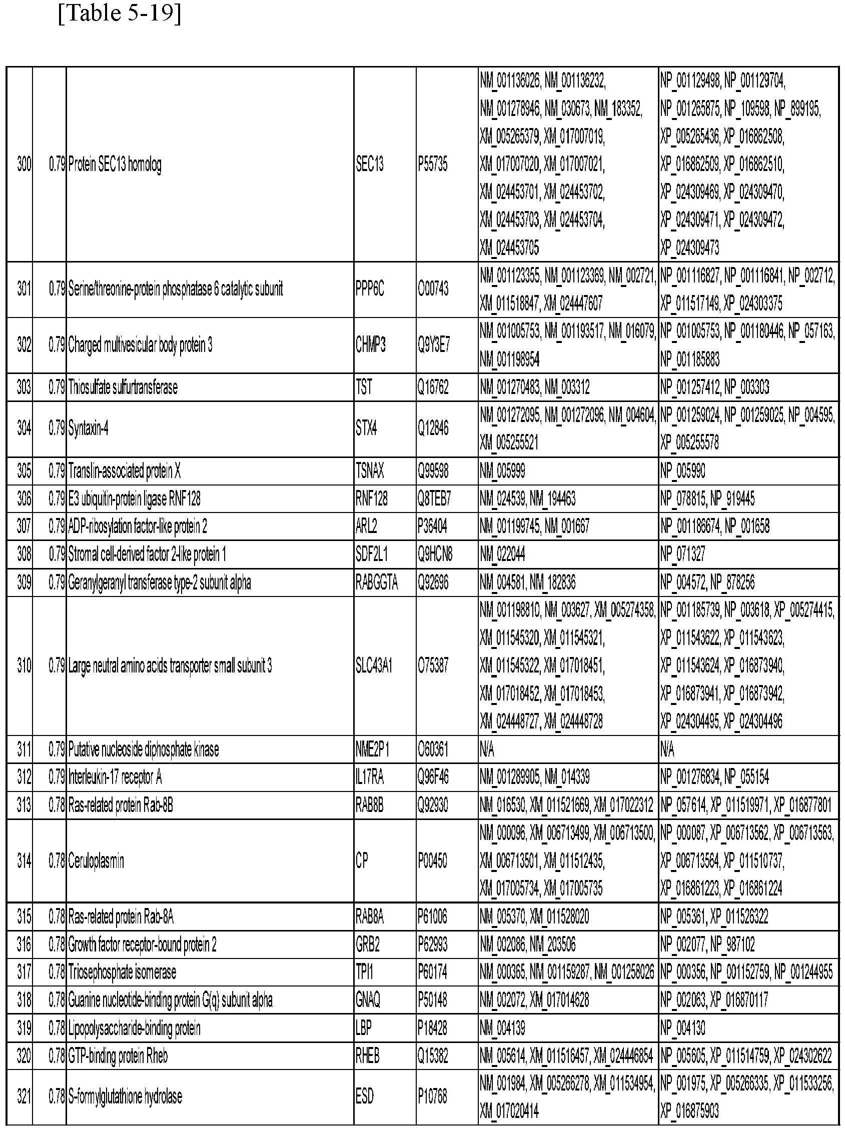

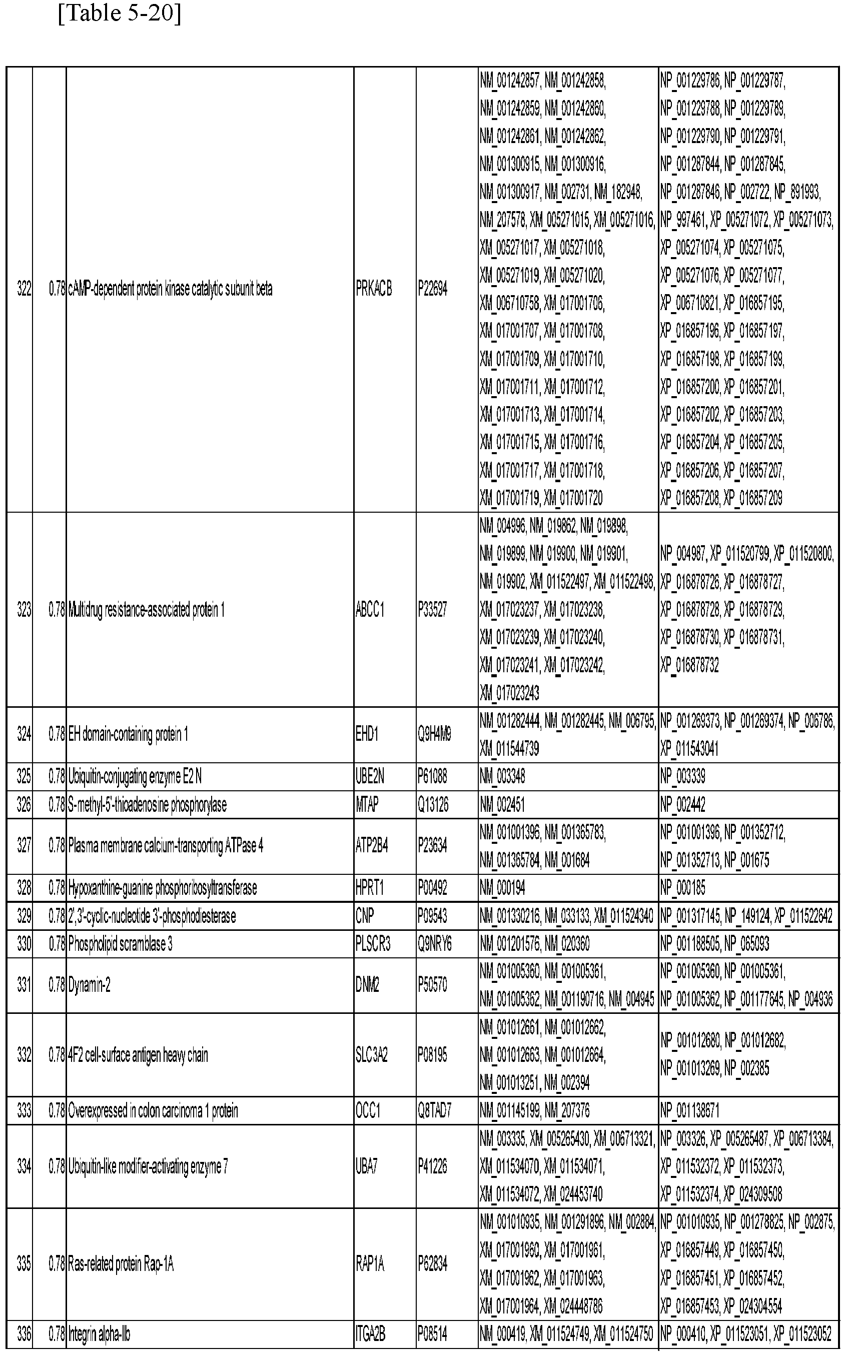

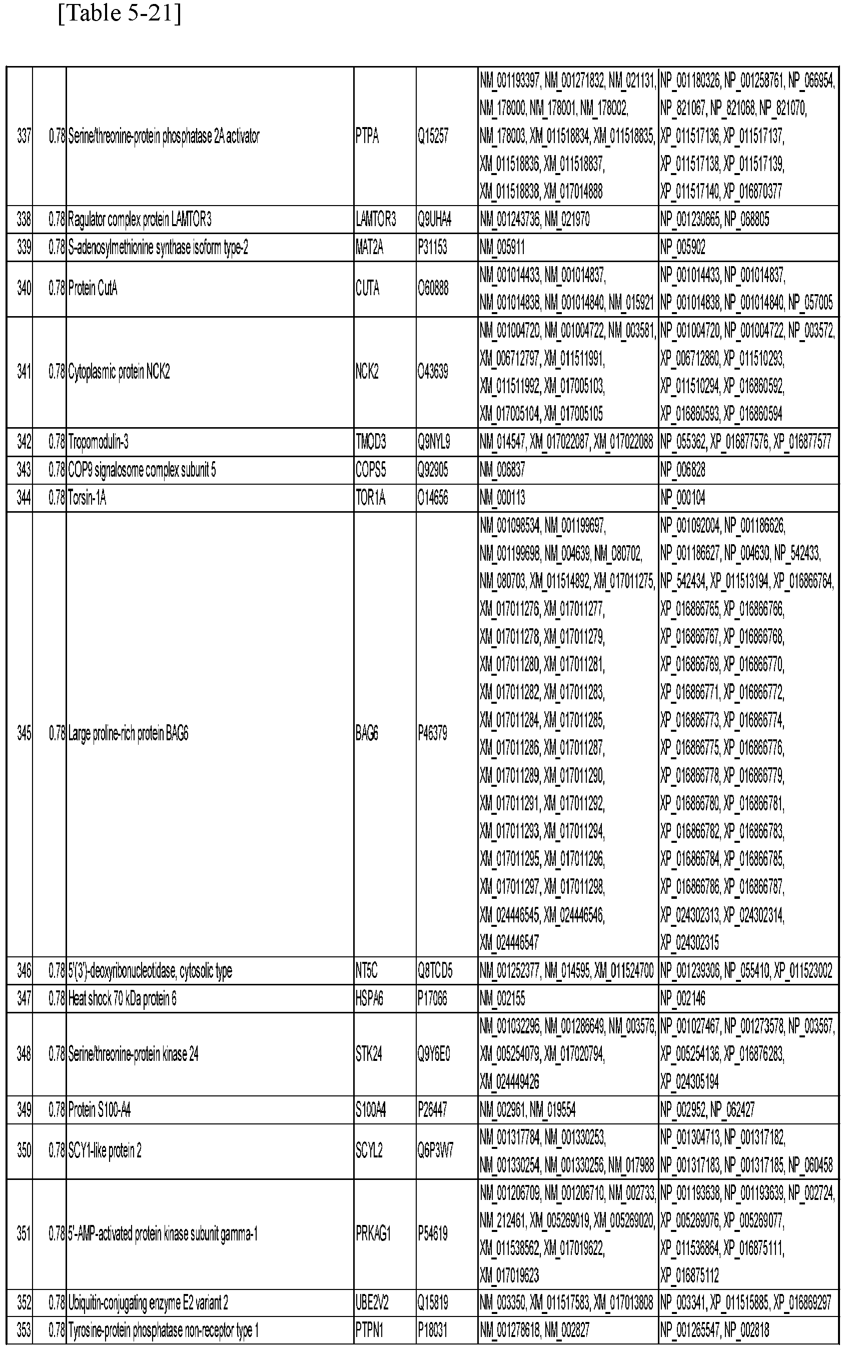

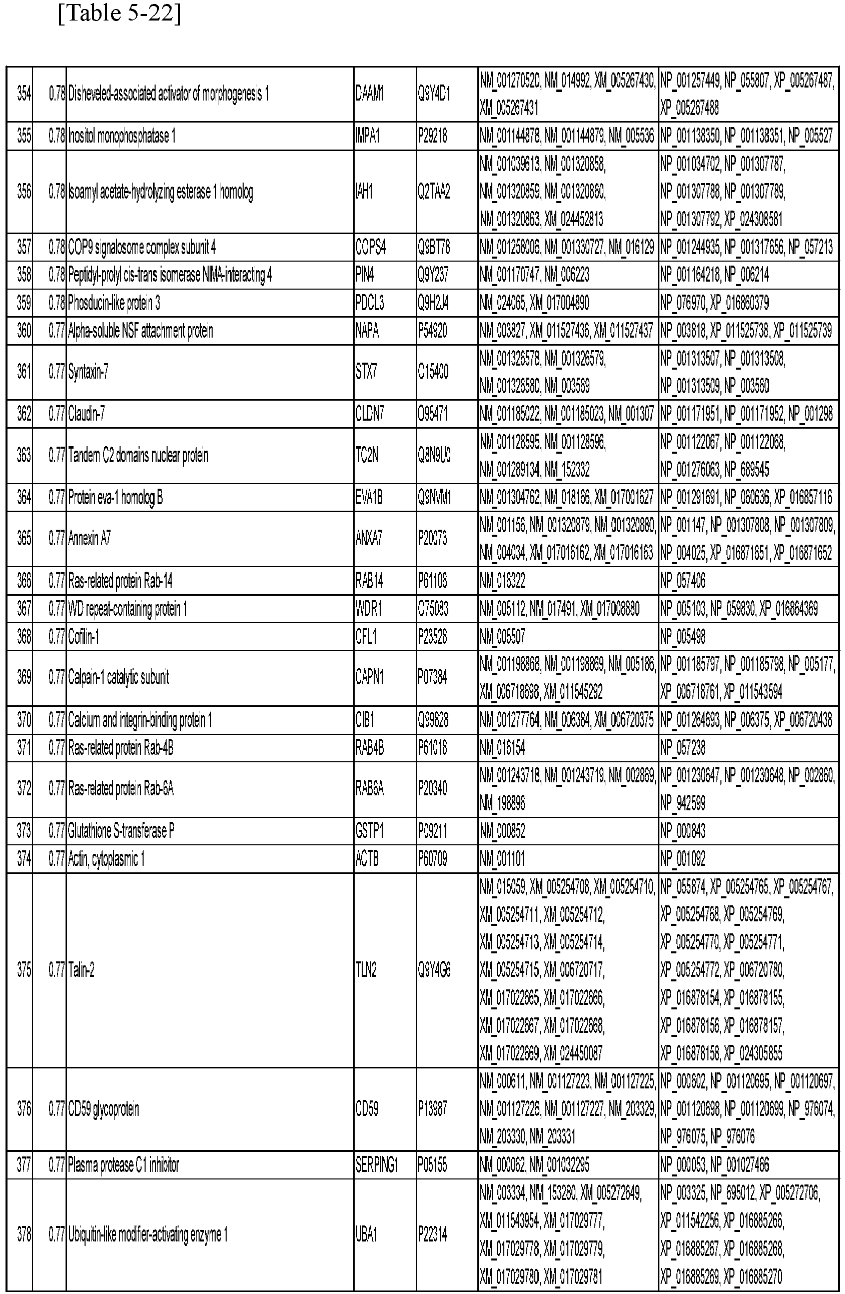

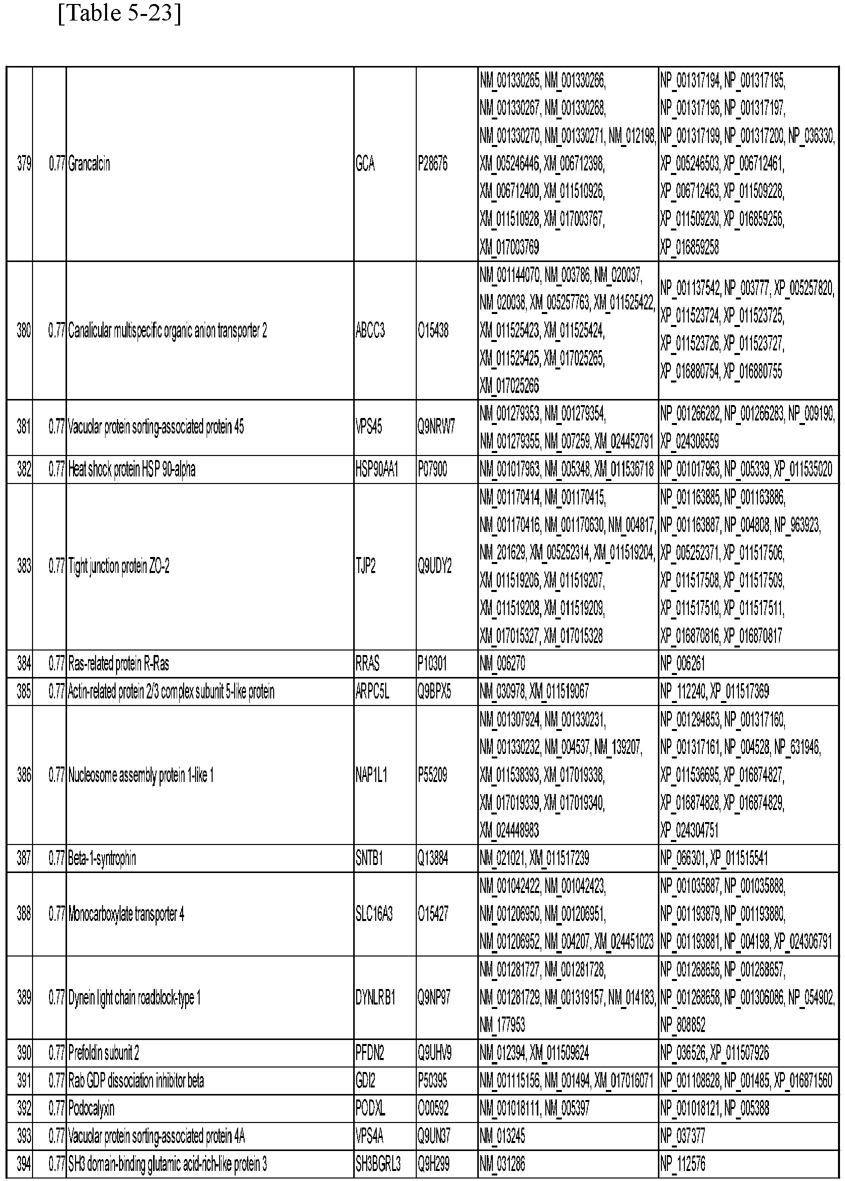

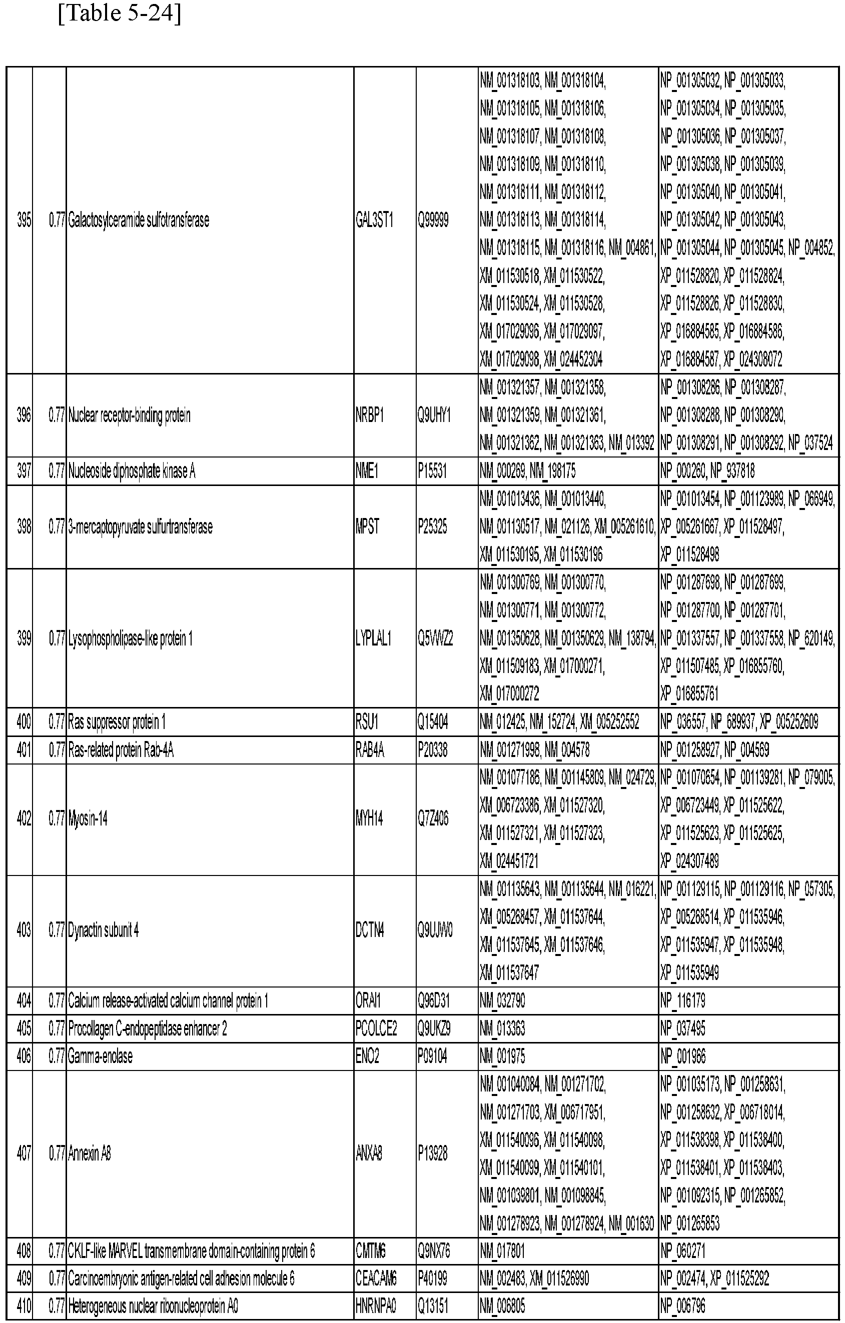

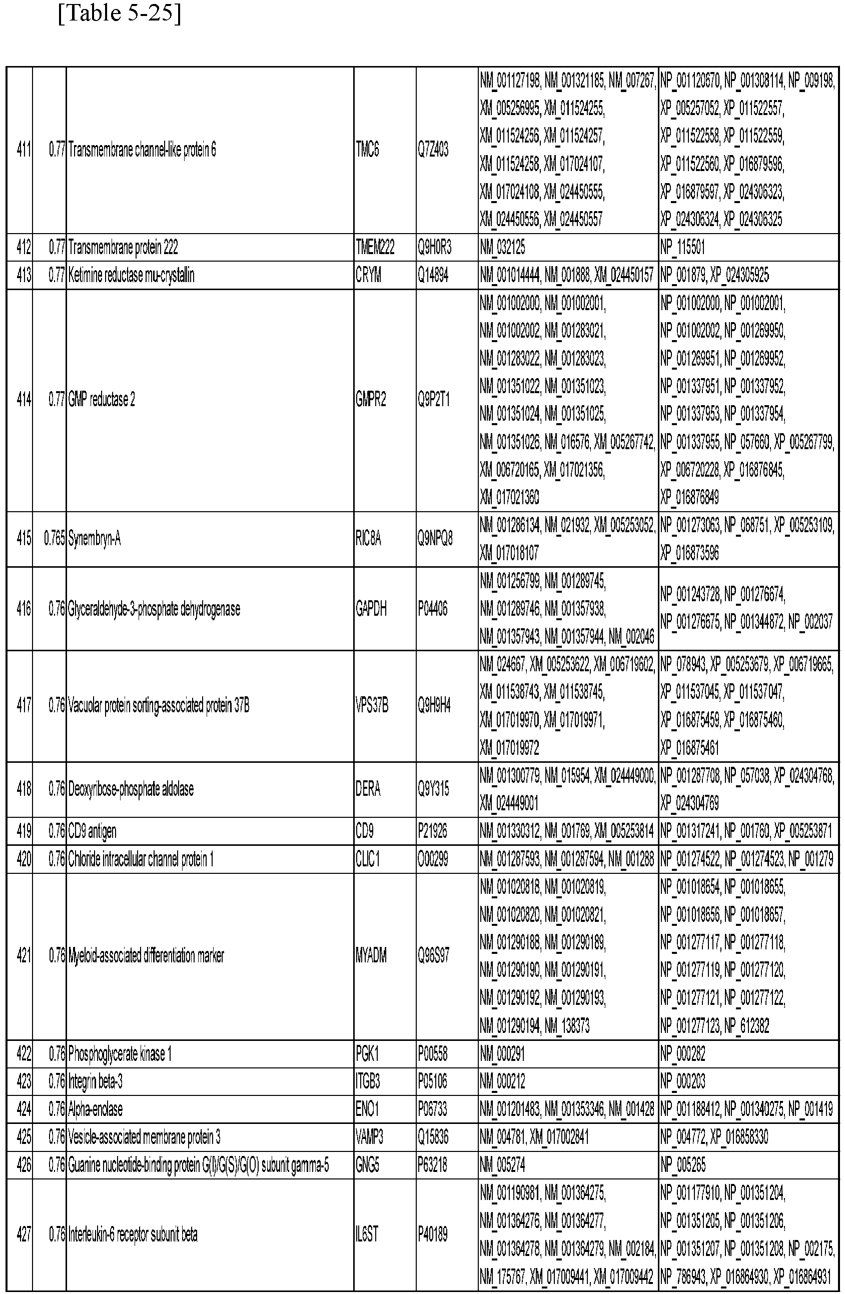

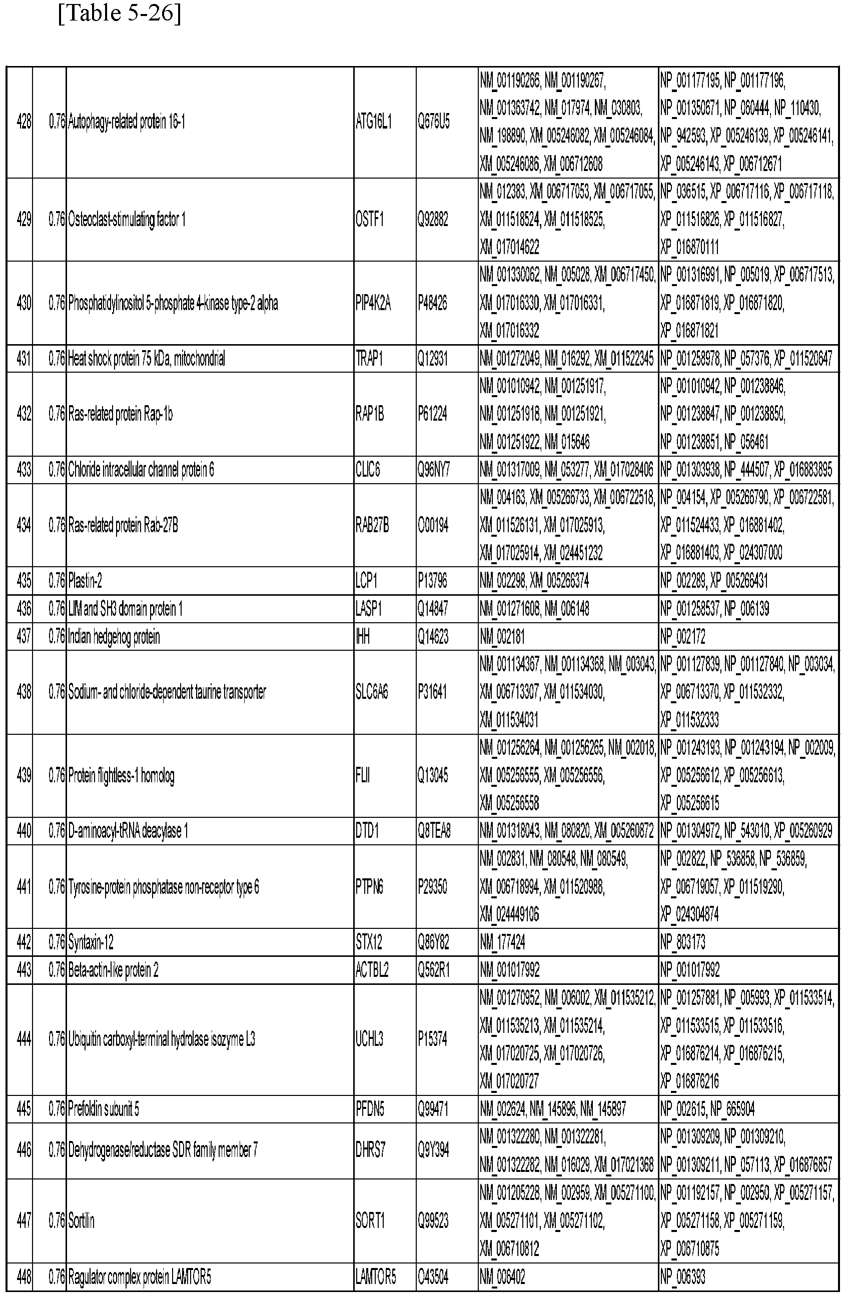

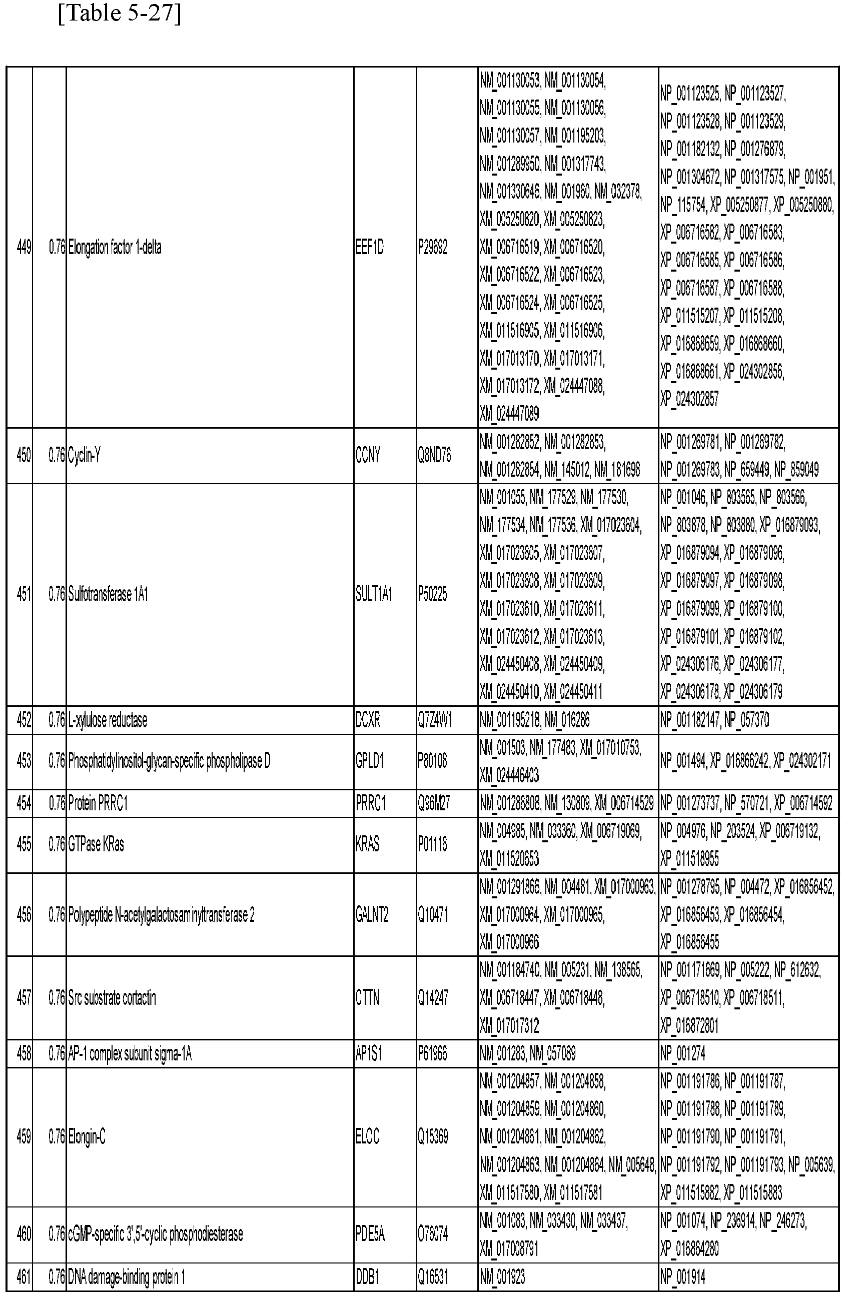

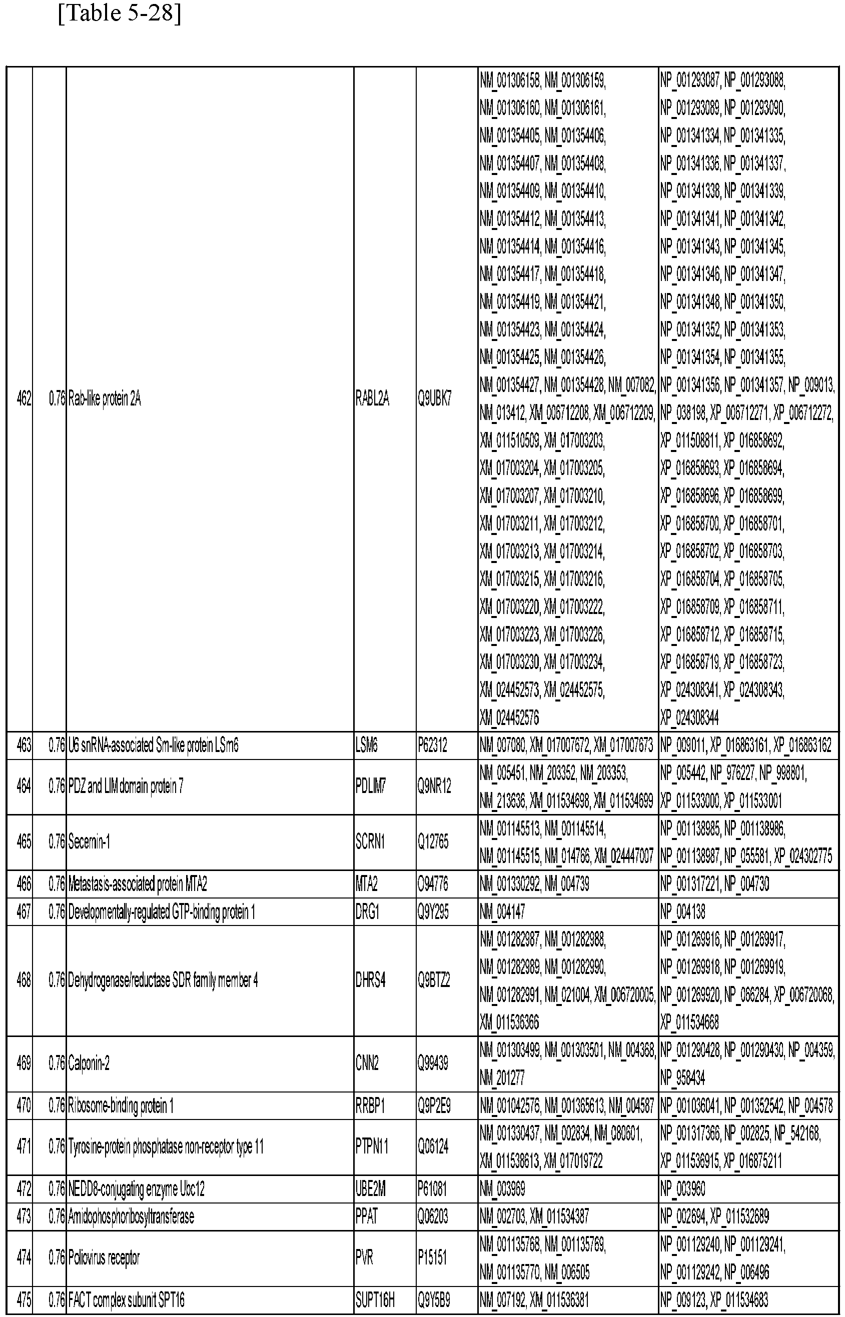

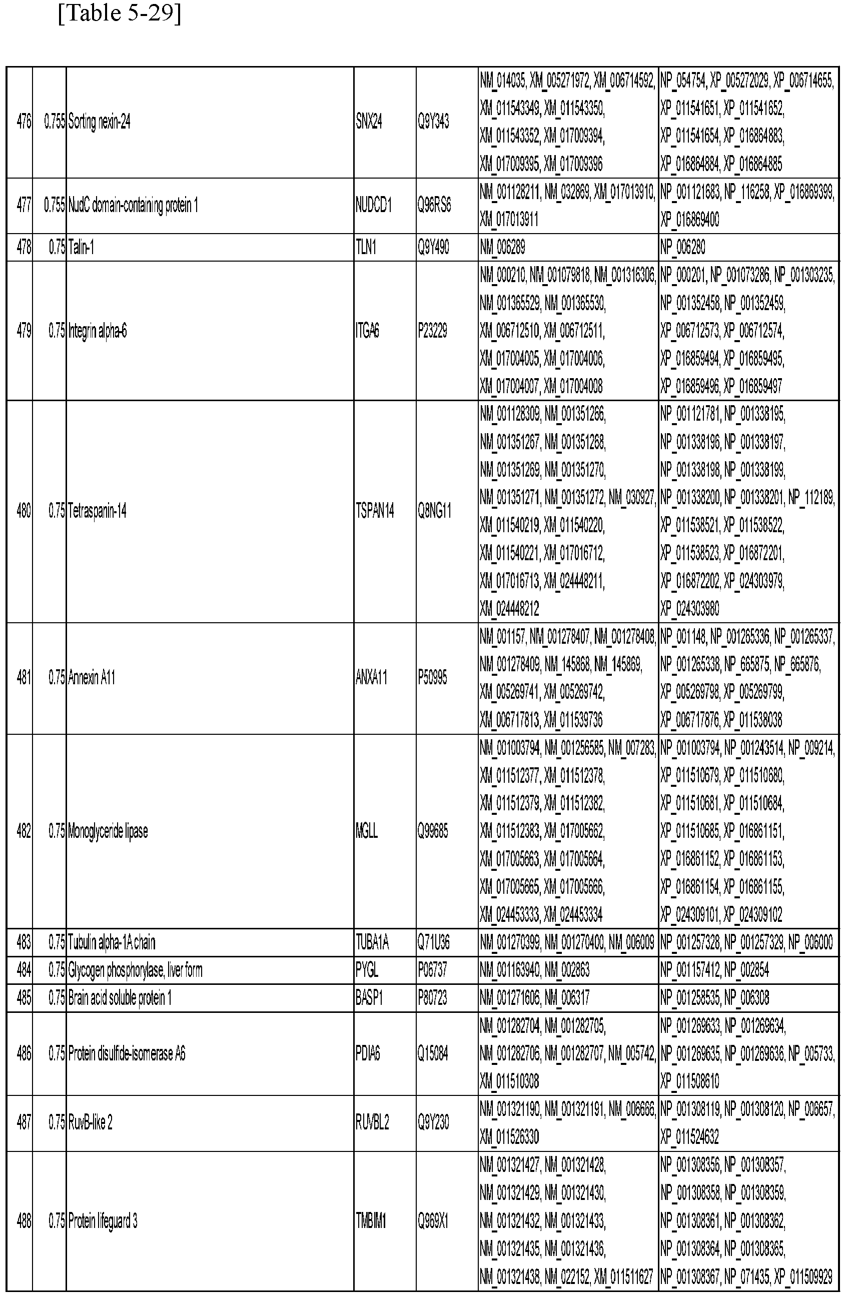

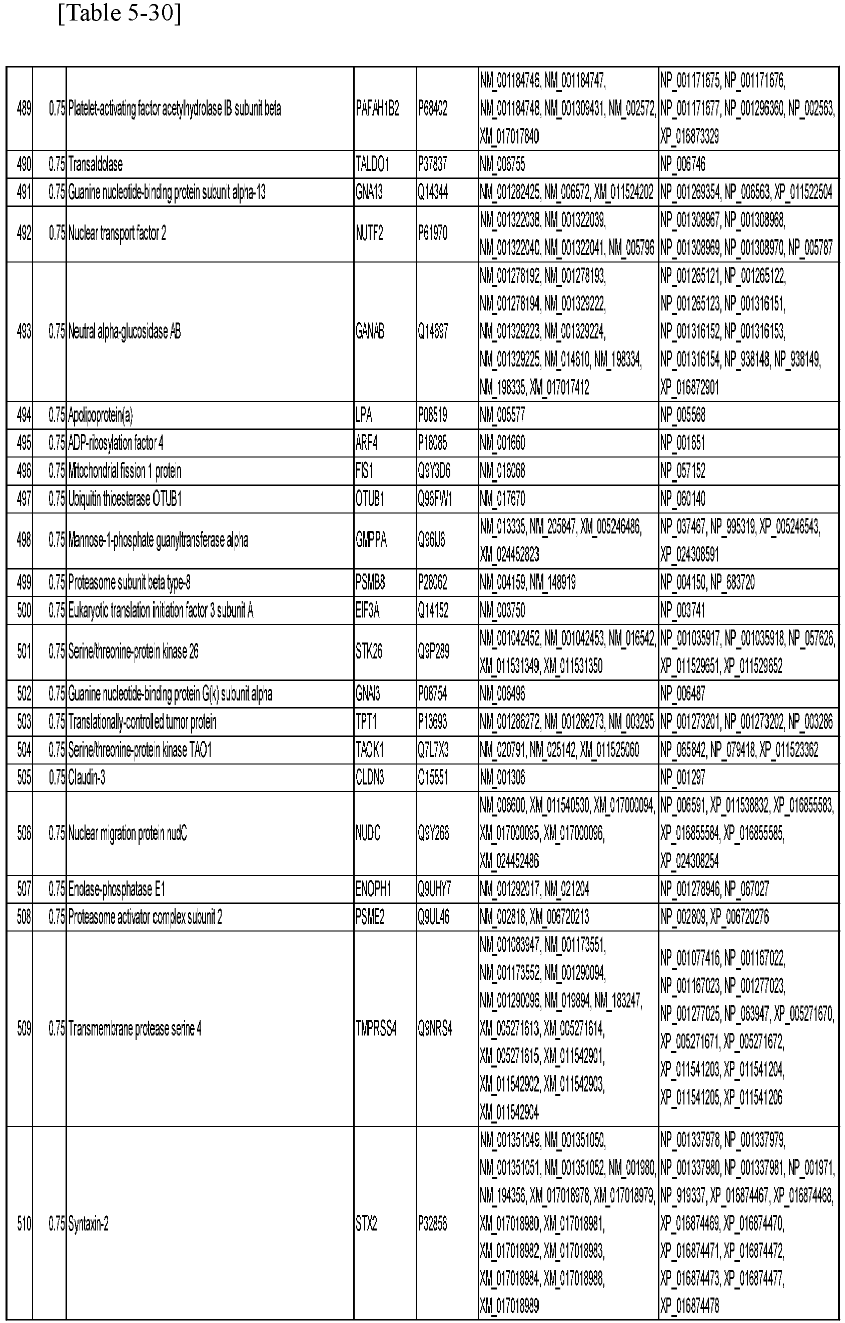

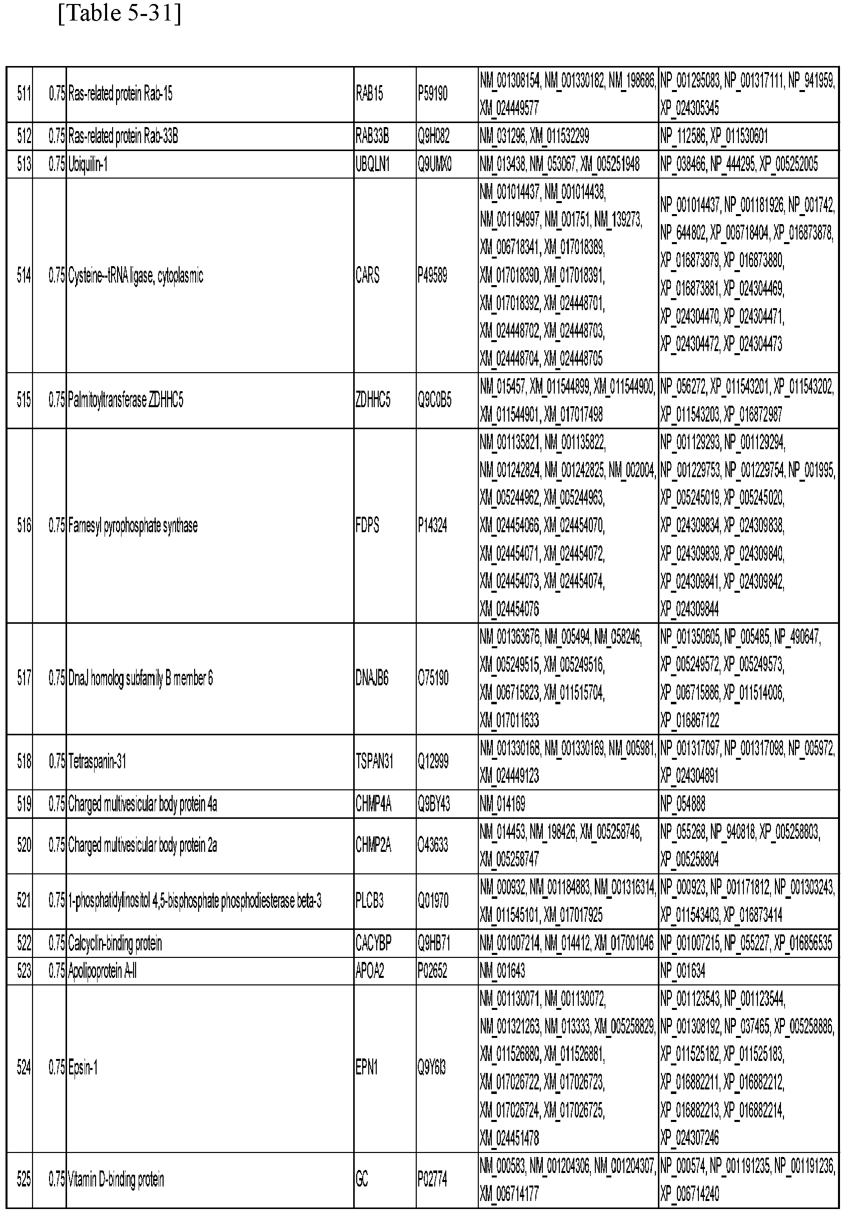

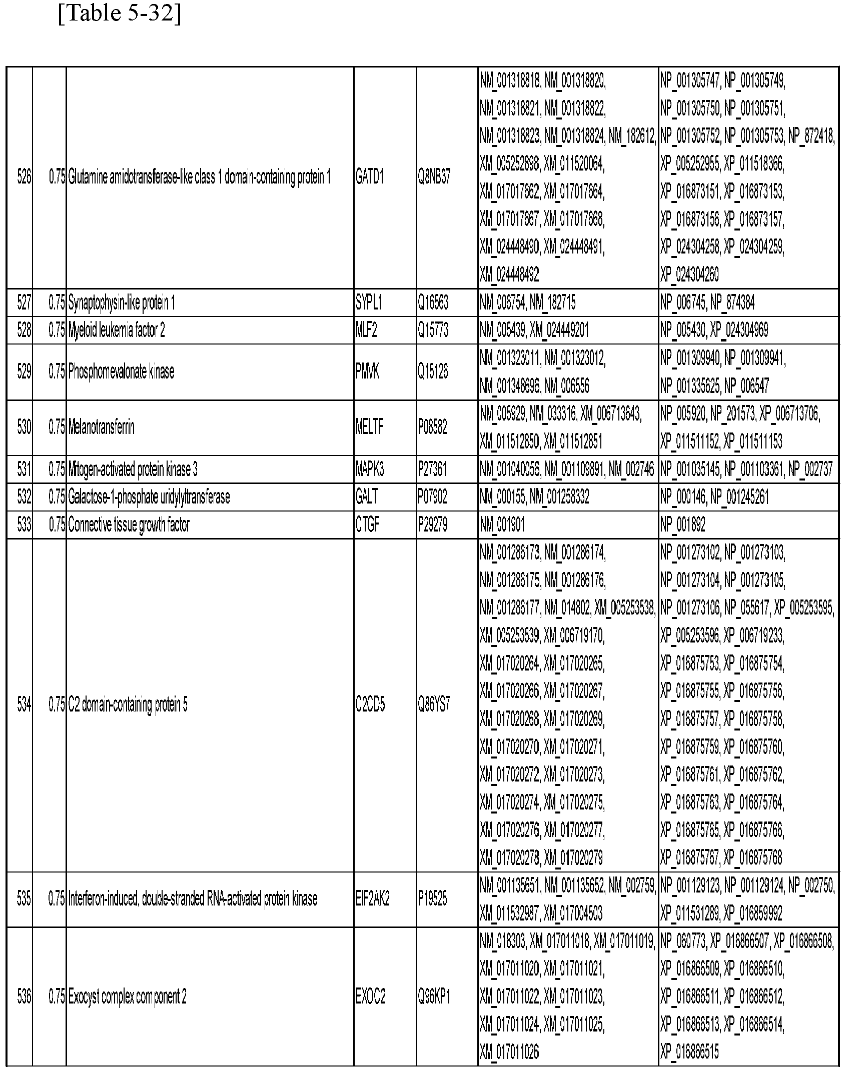

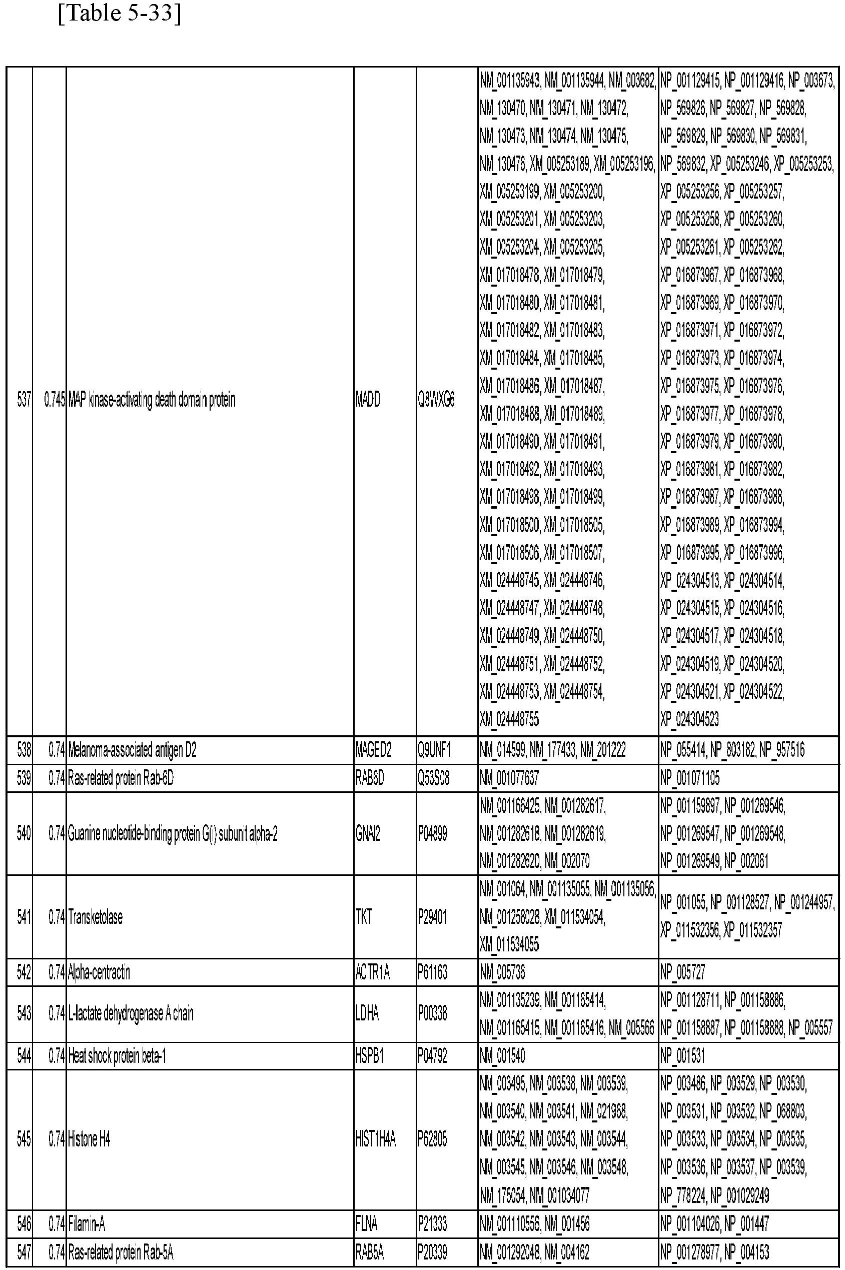

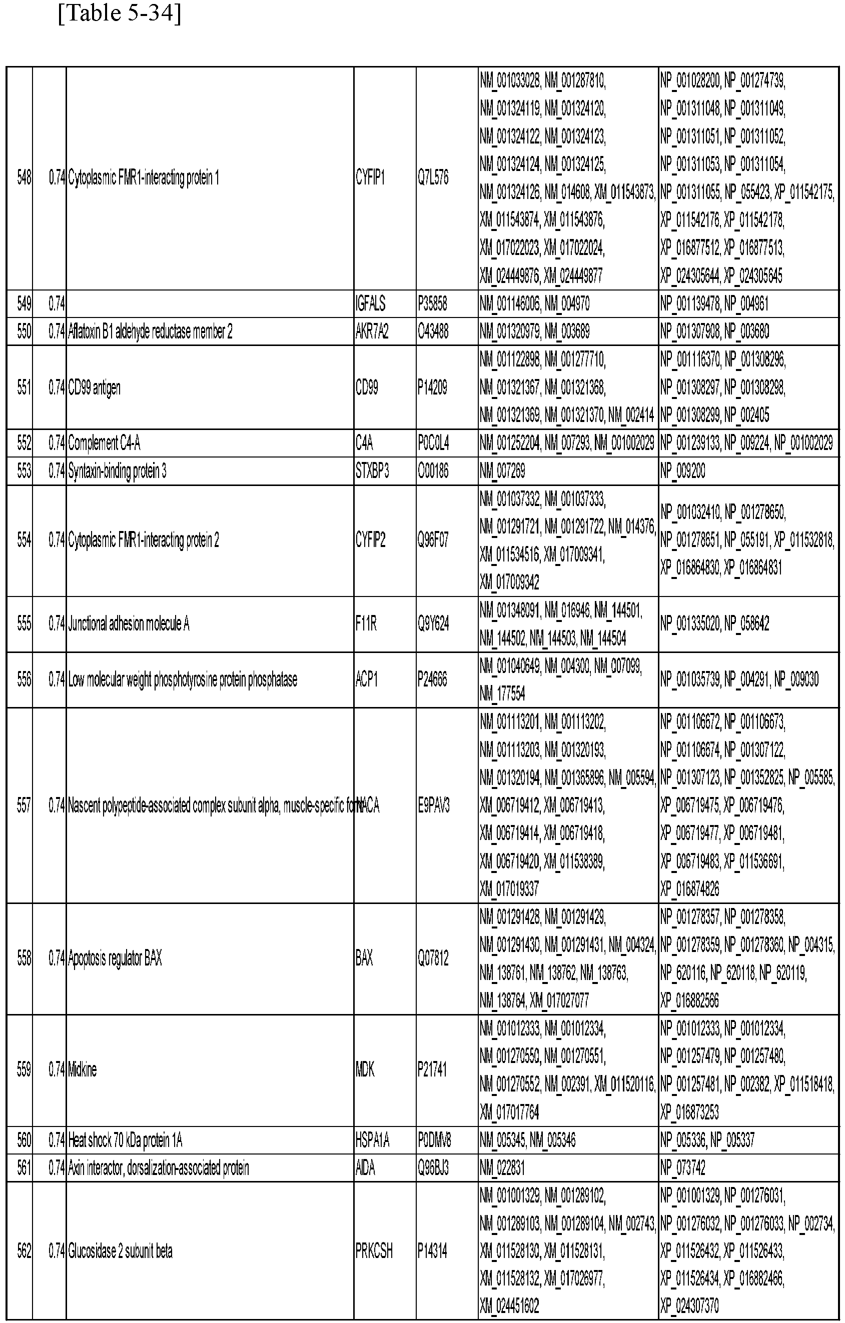

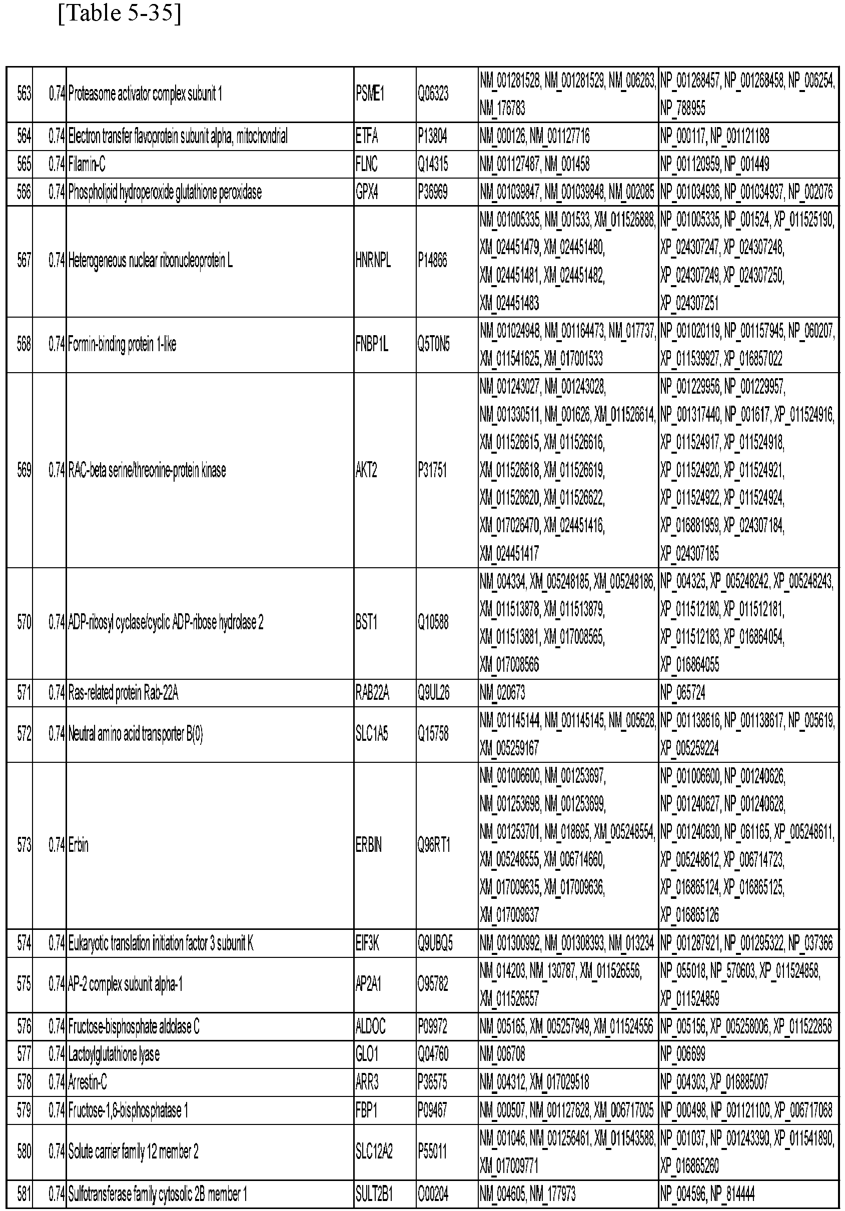

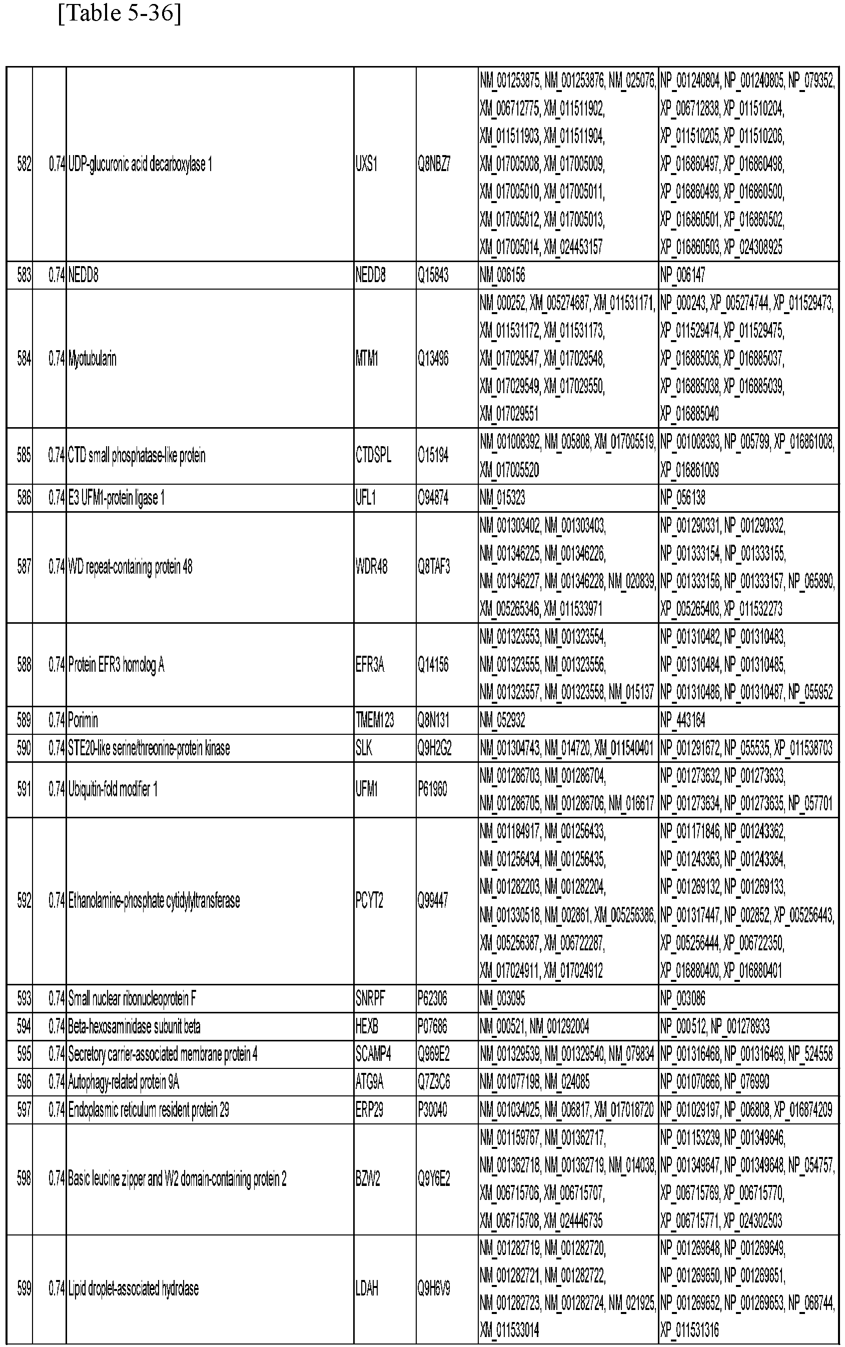

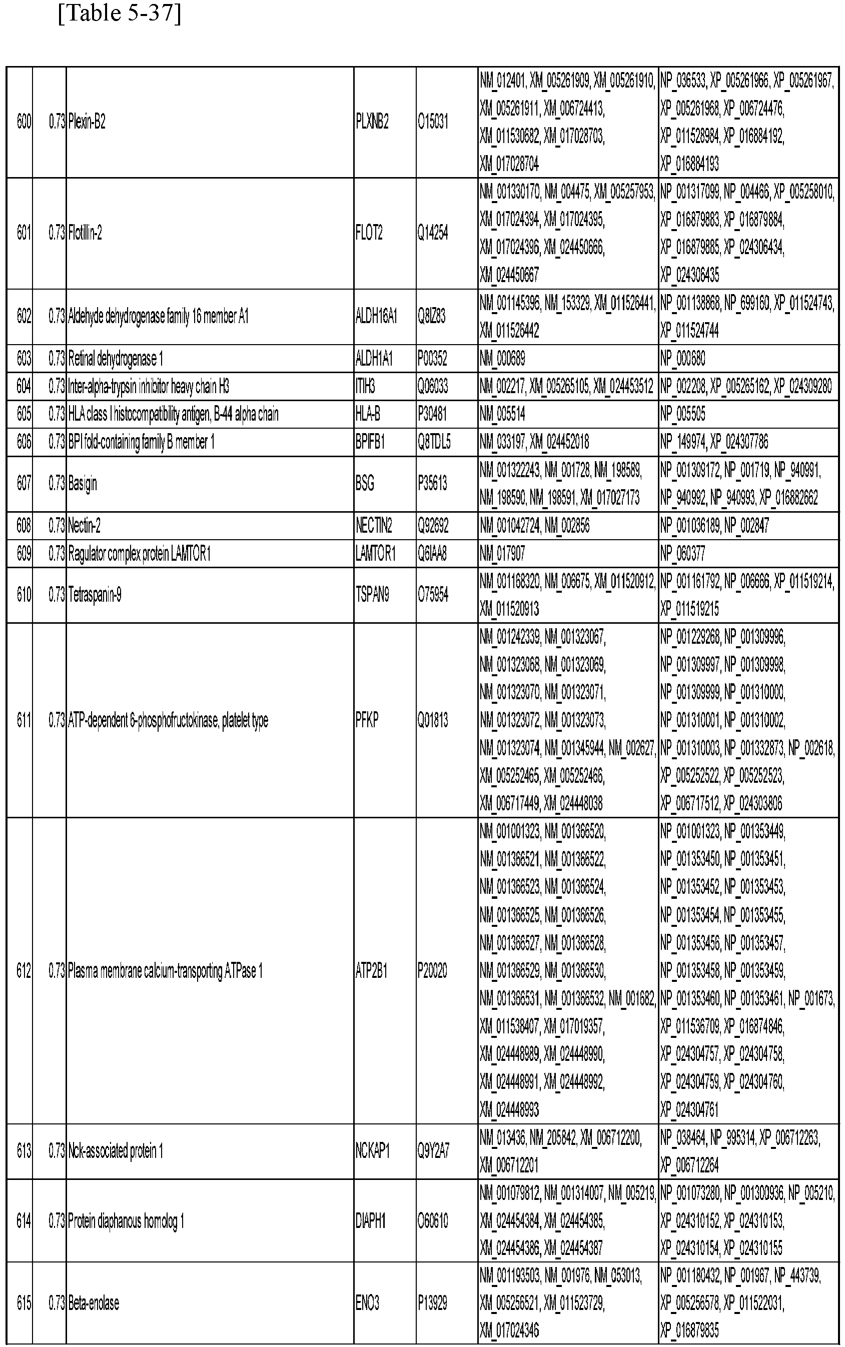

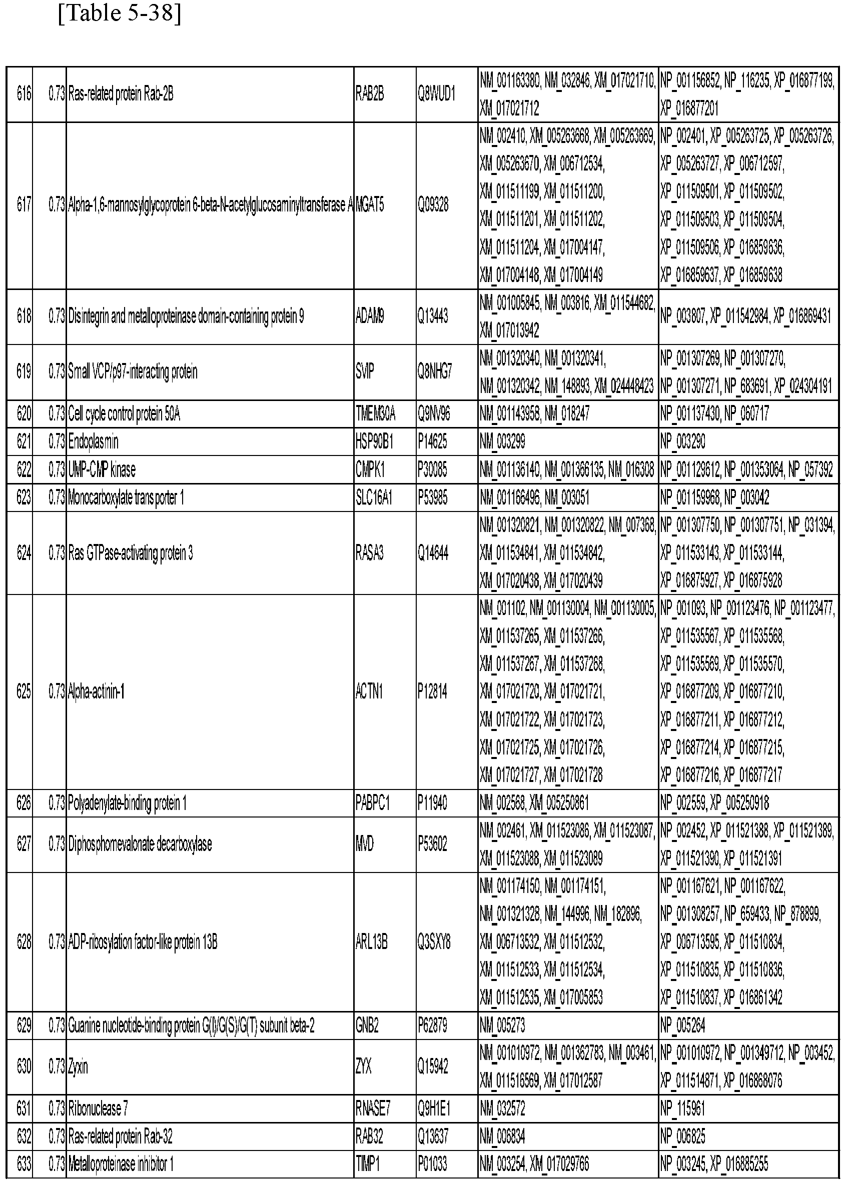

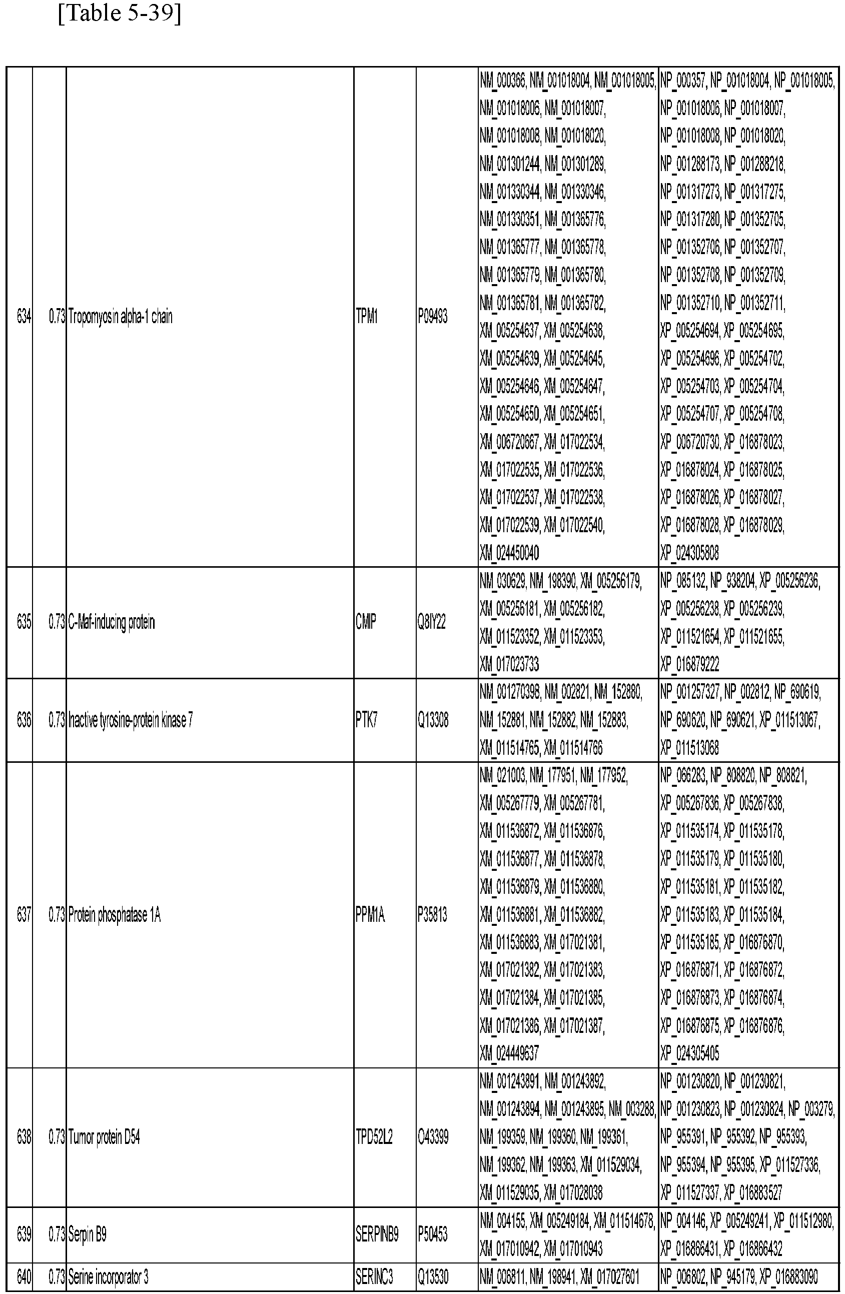

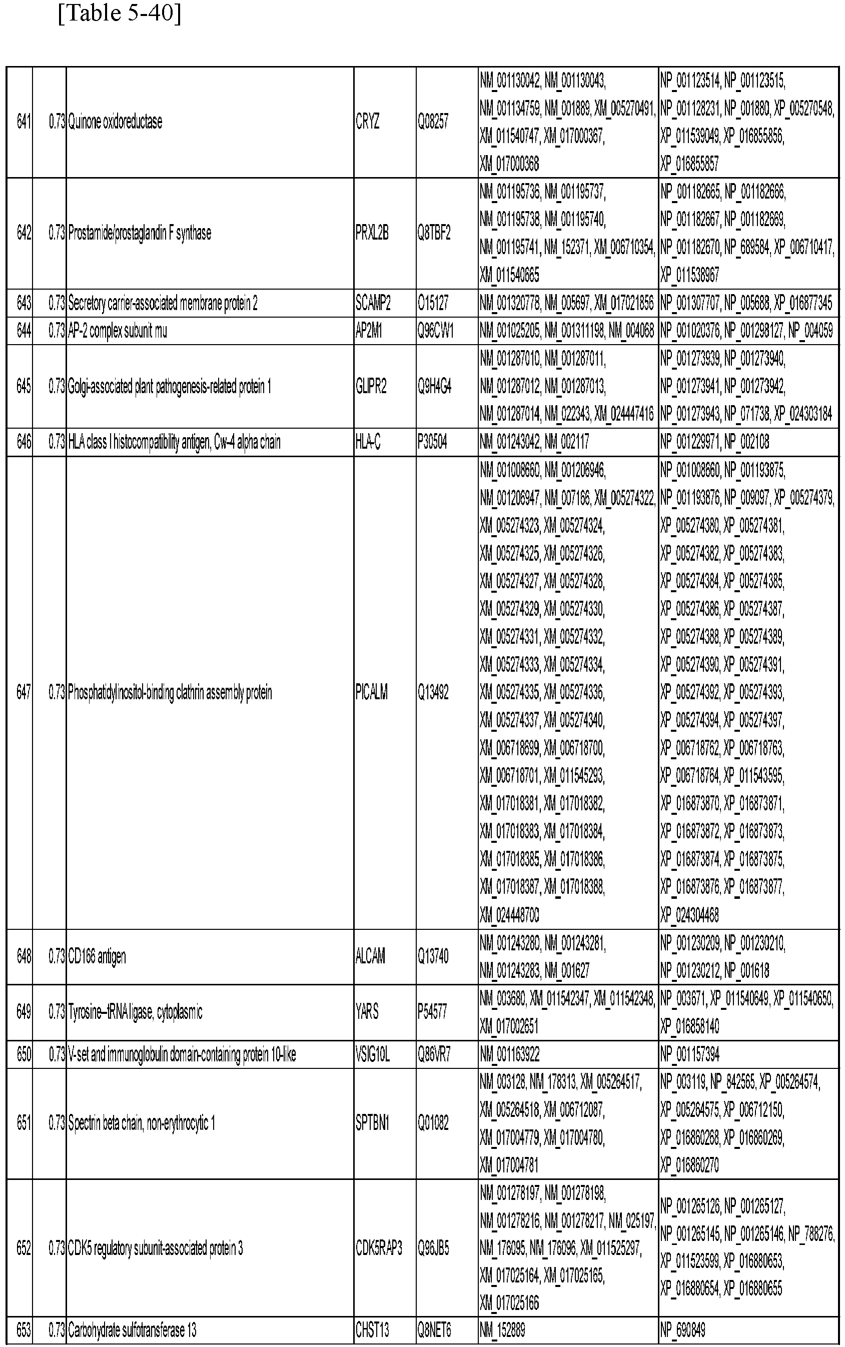

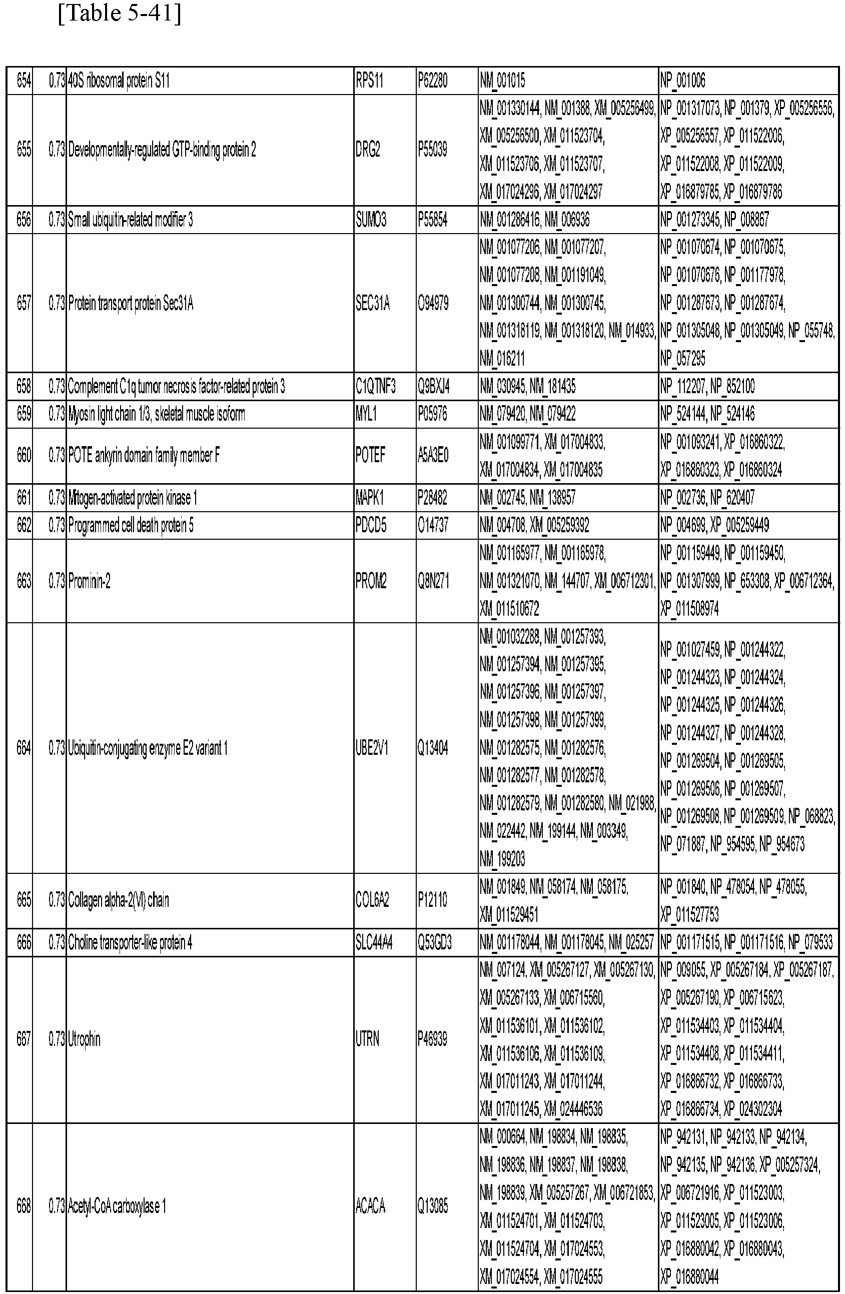

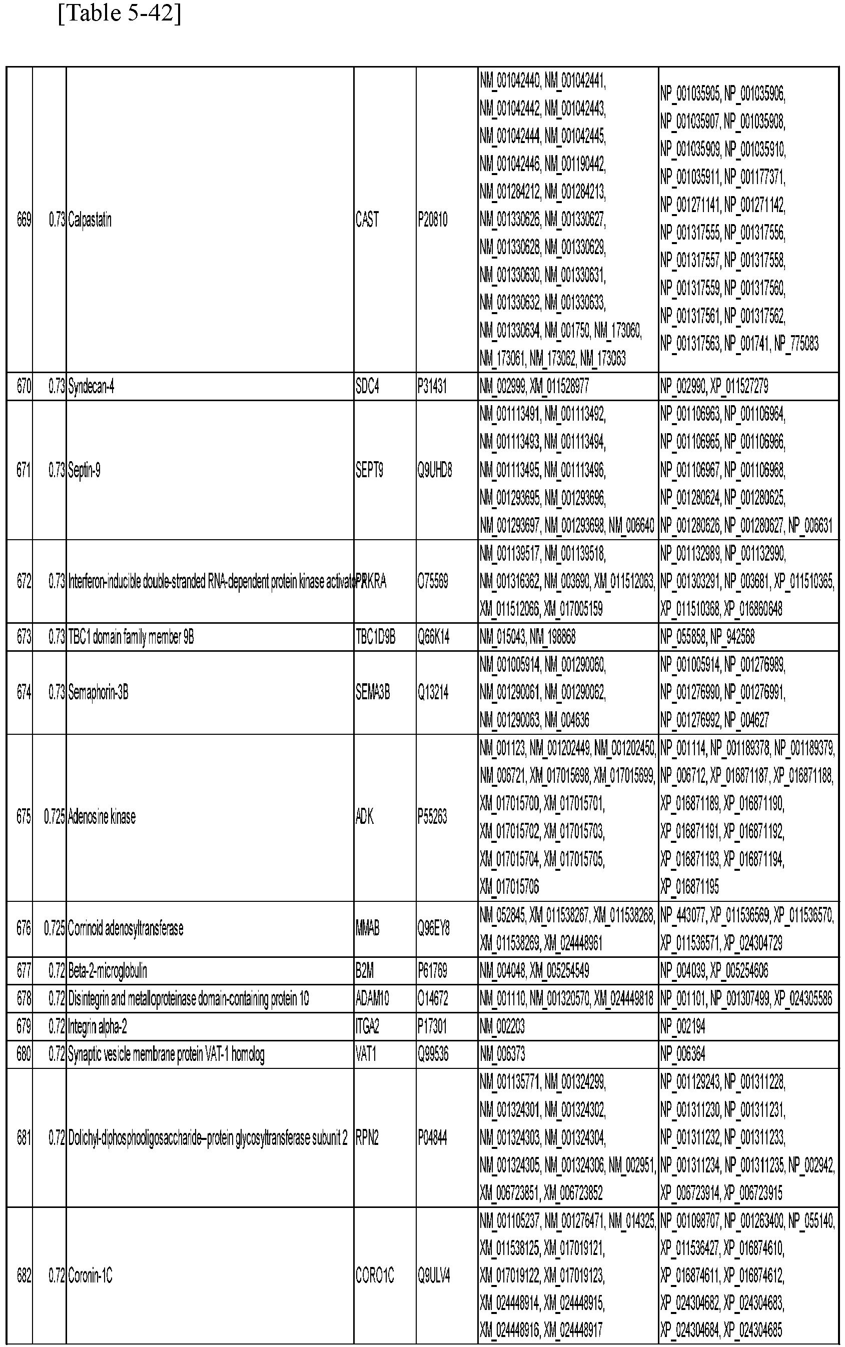

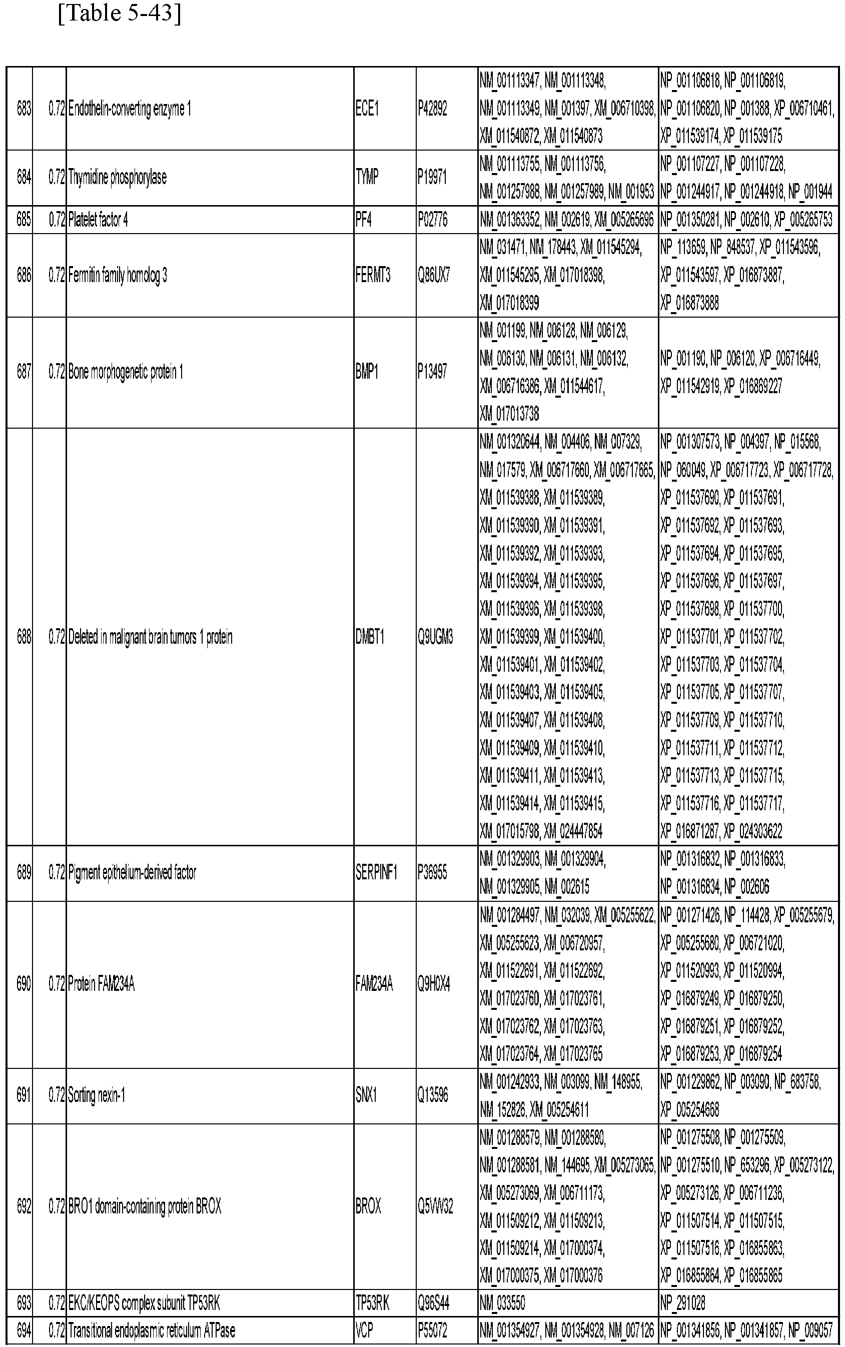

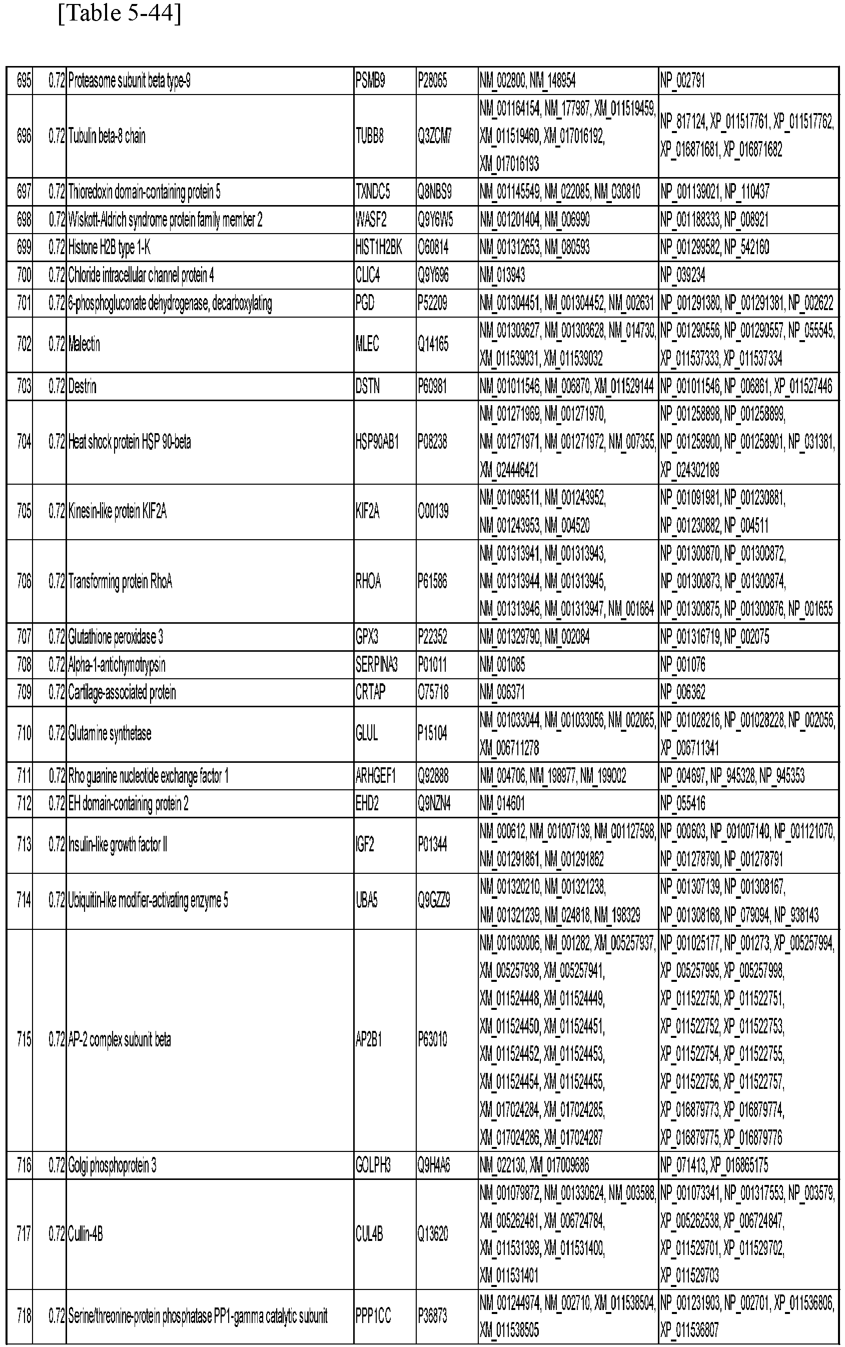

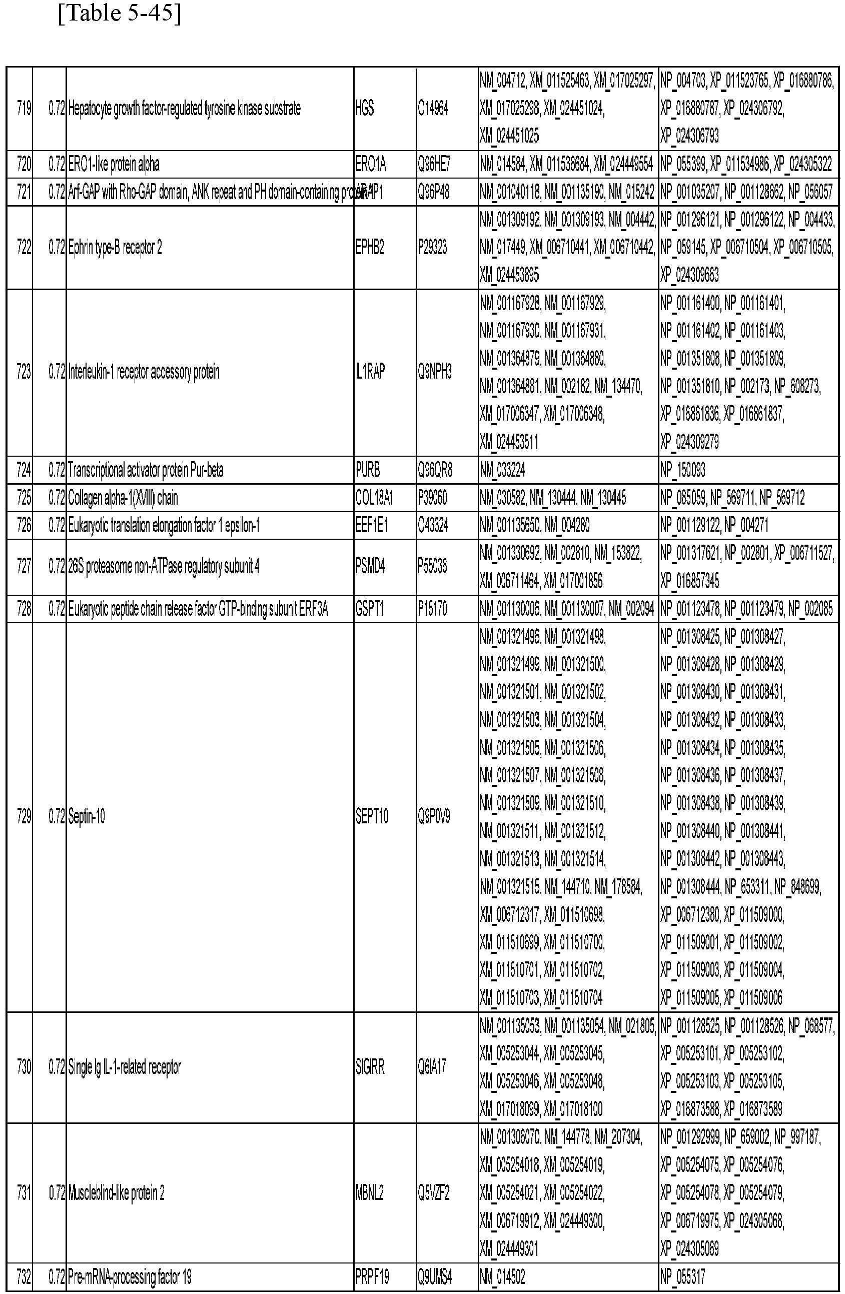

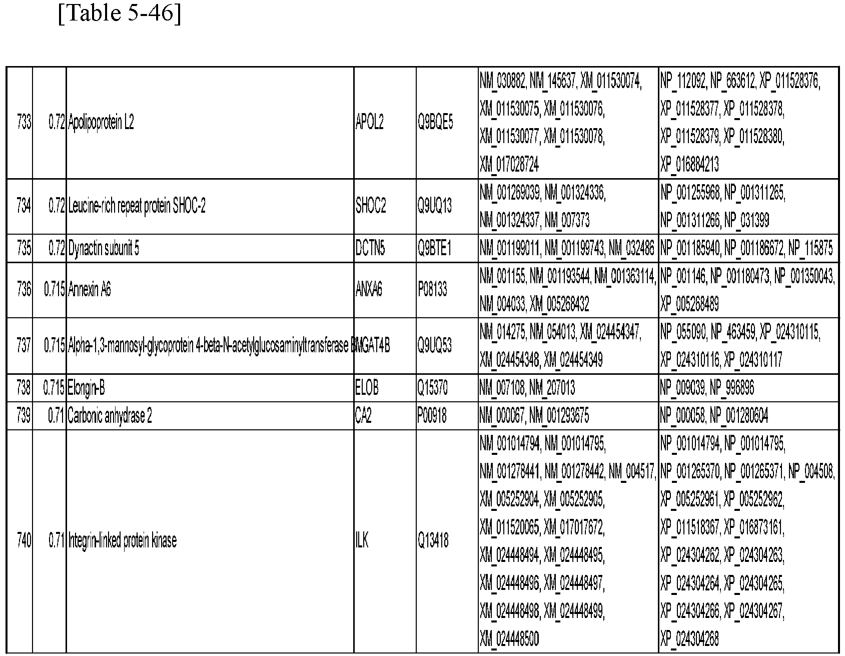

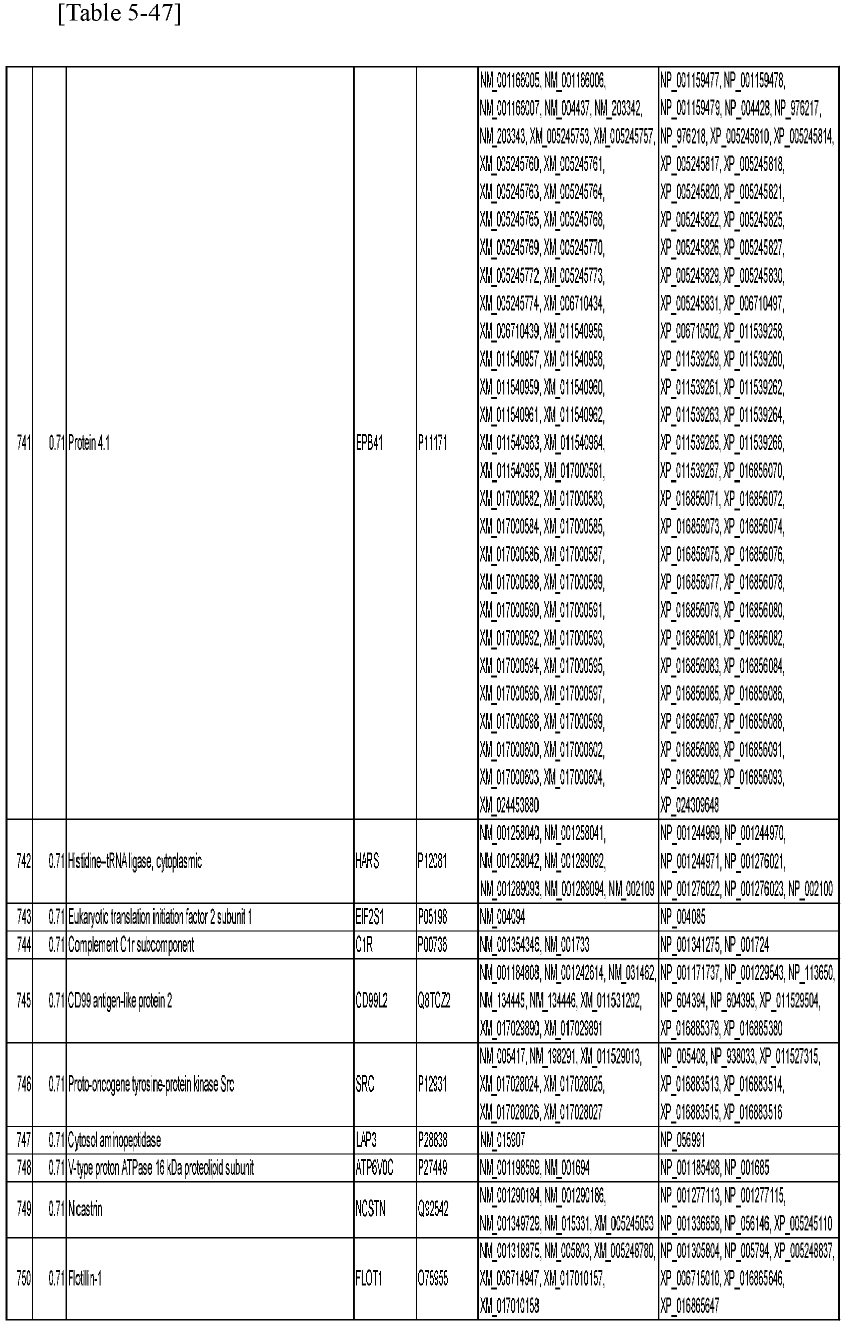

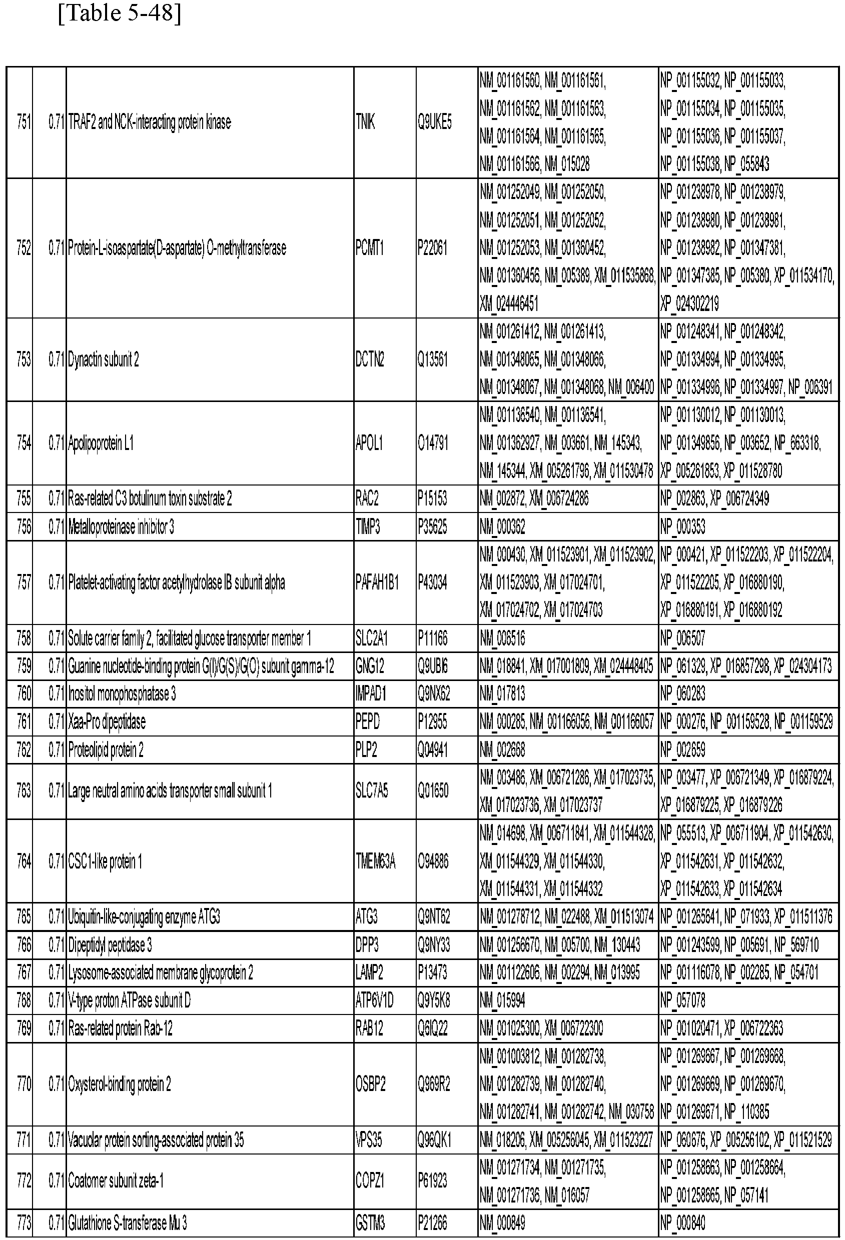

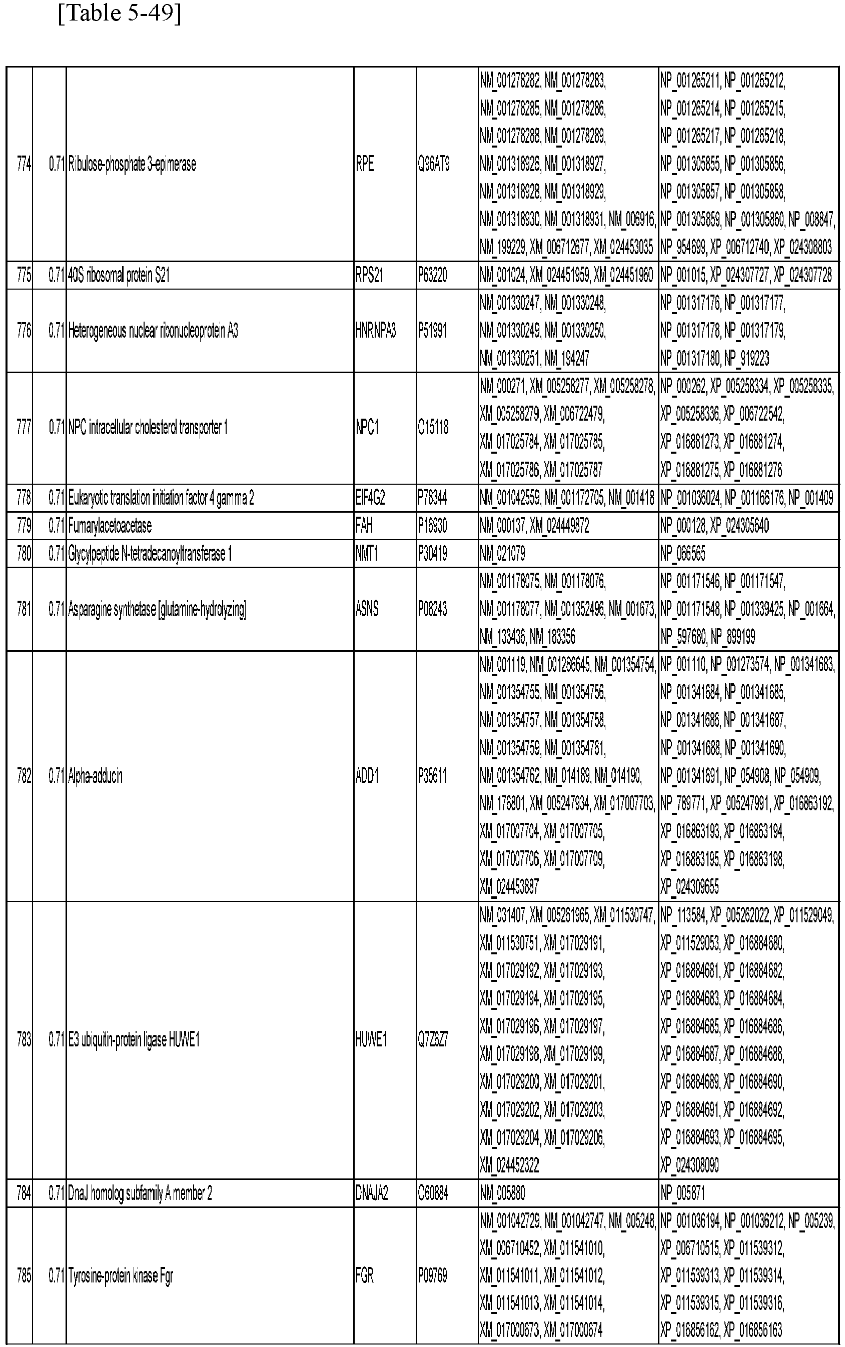

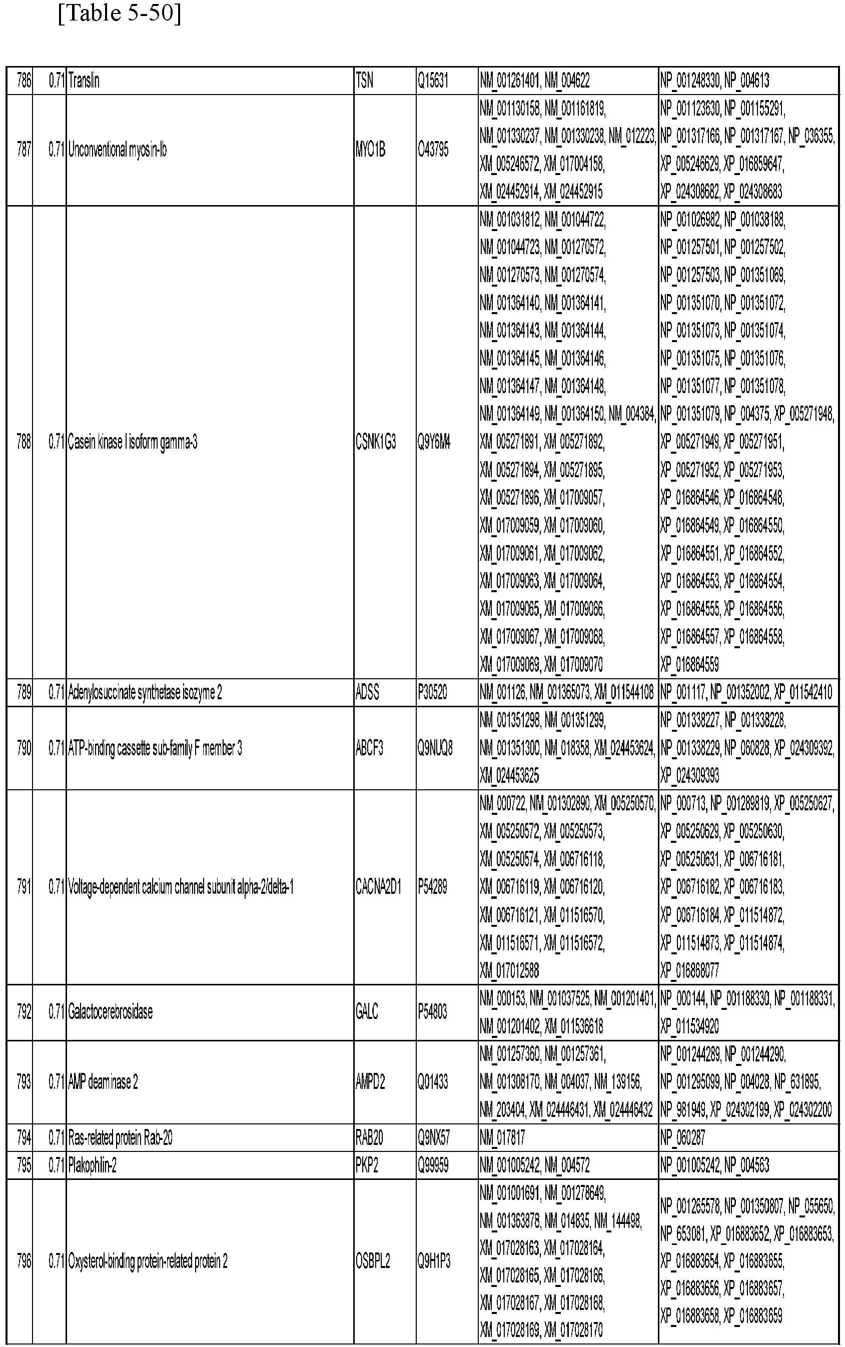

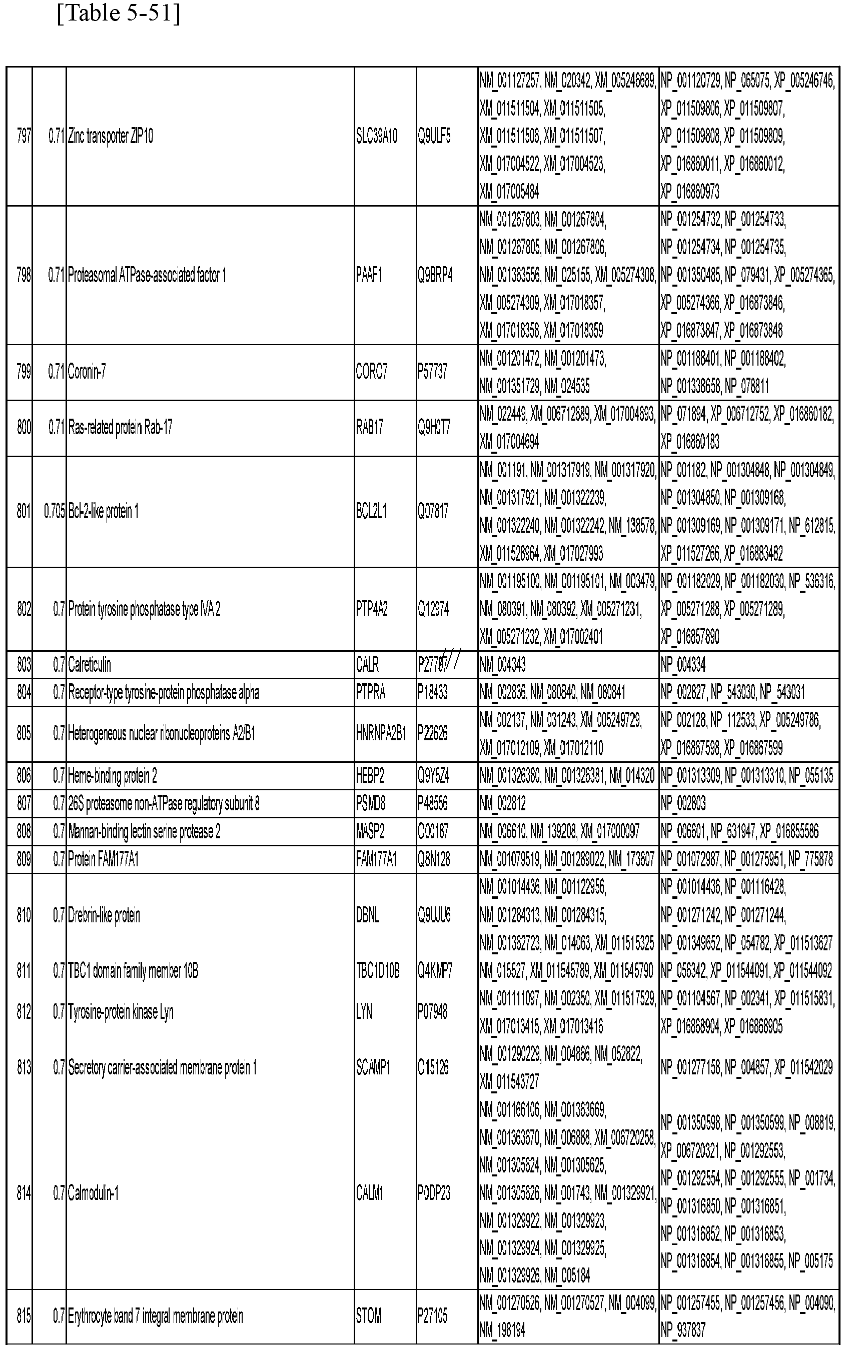

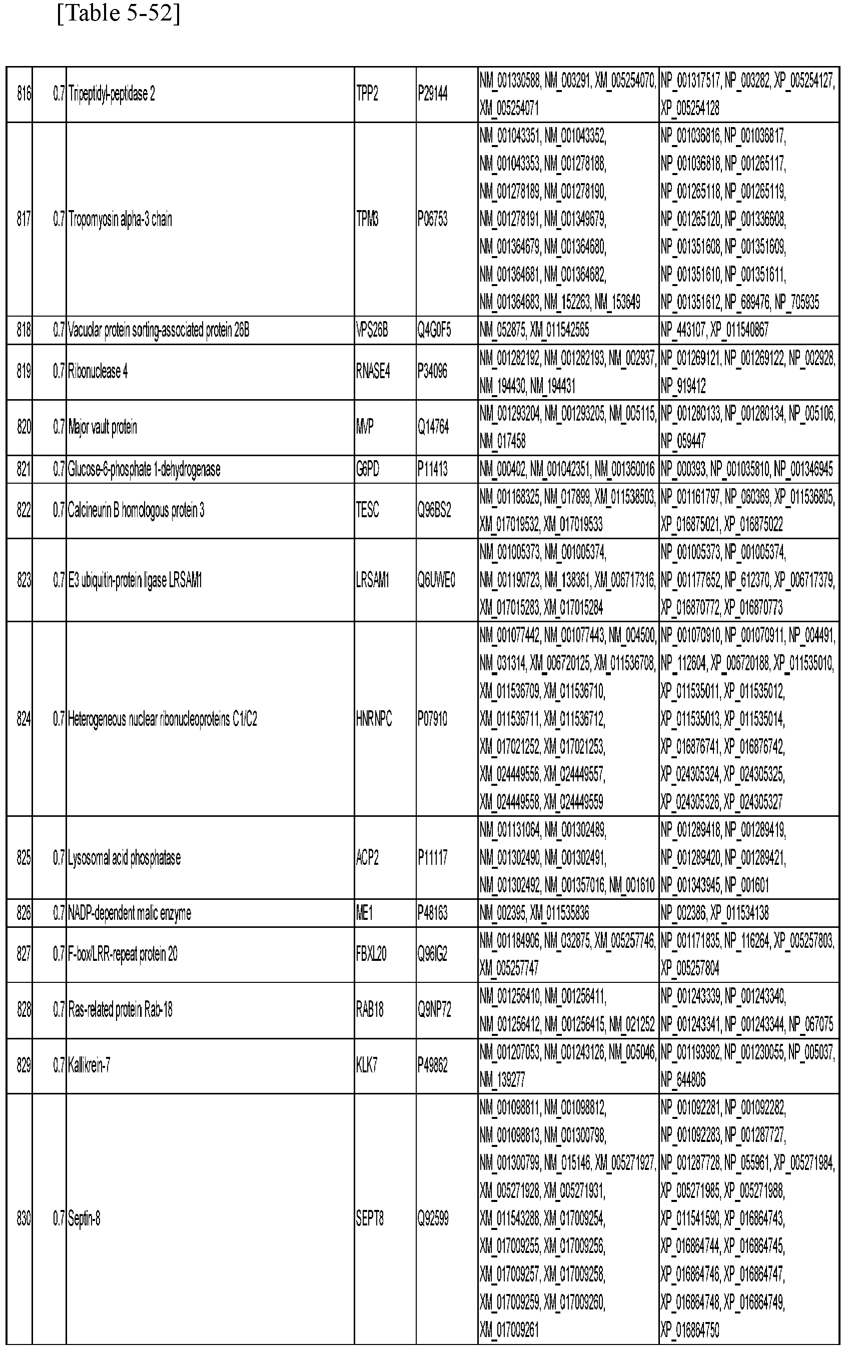

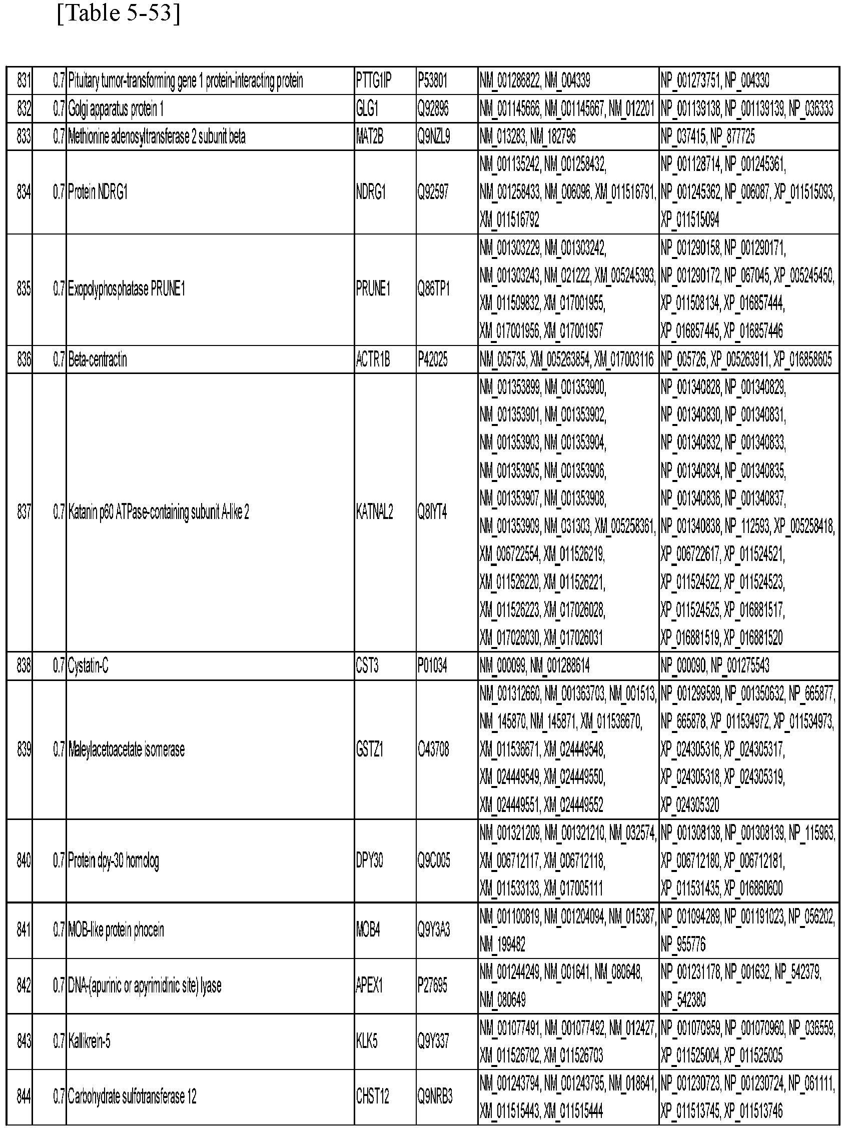

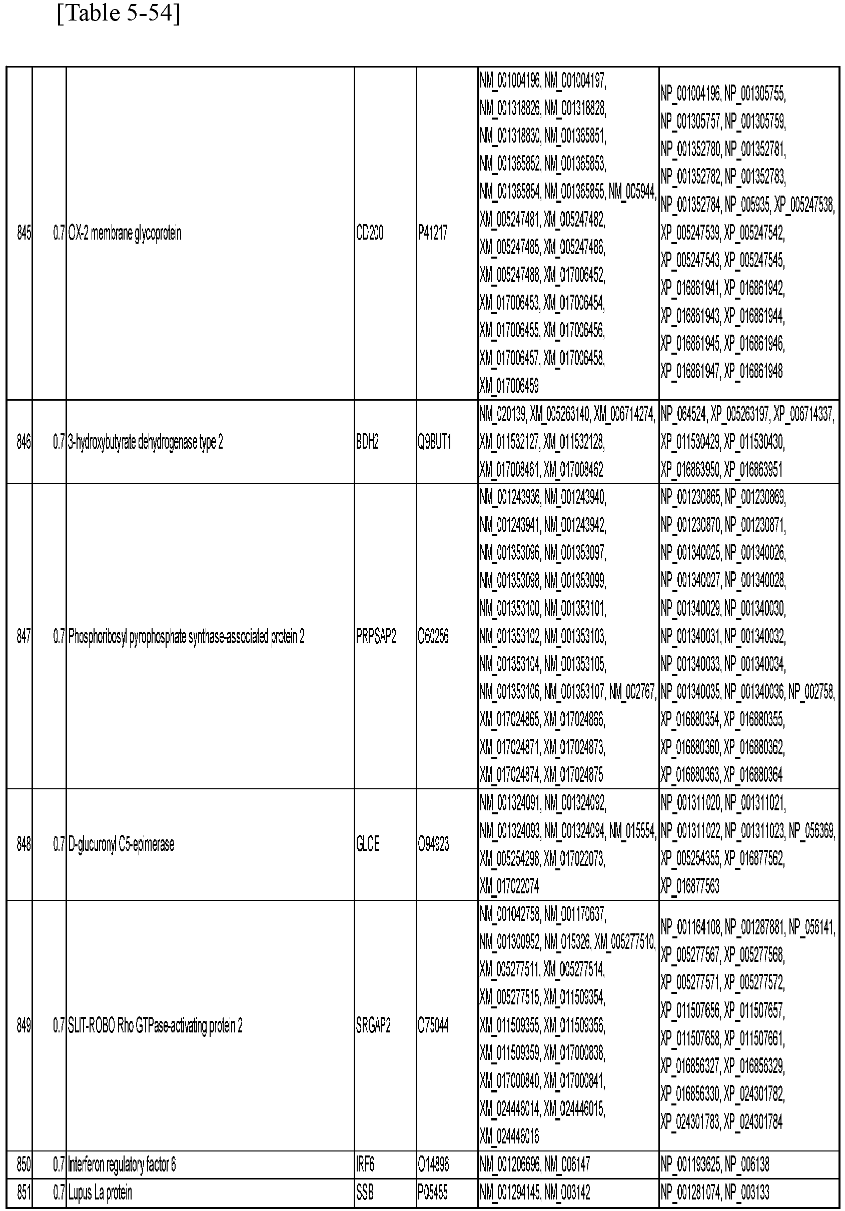

As shown in Table 5-1 to Table 5-54, a group of proteins able to be used to determine drug sensitivity was found in proteins present in exosomes in the blood of lung cancer patients.

### 3. Extraction of Membrane Protein Groups

Based on the Predicted membrane proteins information in The Human Protein Atlas (https://www.proteinatlas.org/), membrane protein groups were extracted from the protein groups in Table 5-1 to Table 5-54 described above. The list of obtained membrane proteins is shown in Tables 6-1 to 6-15.

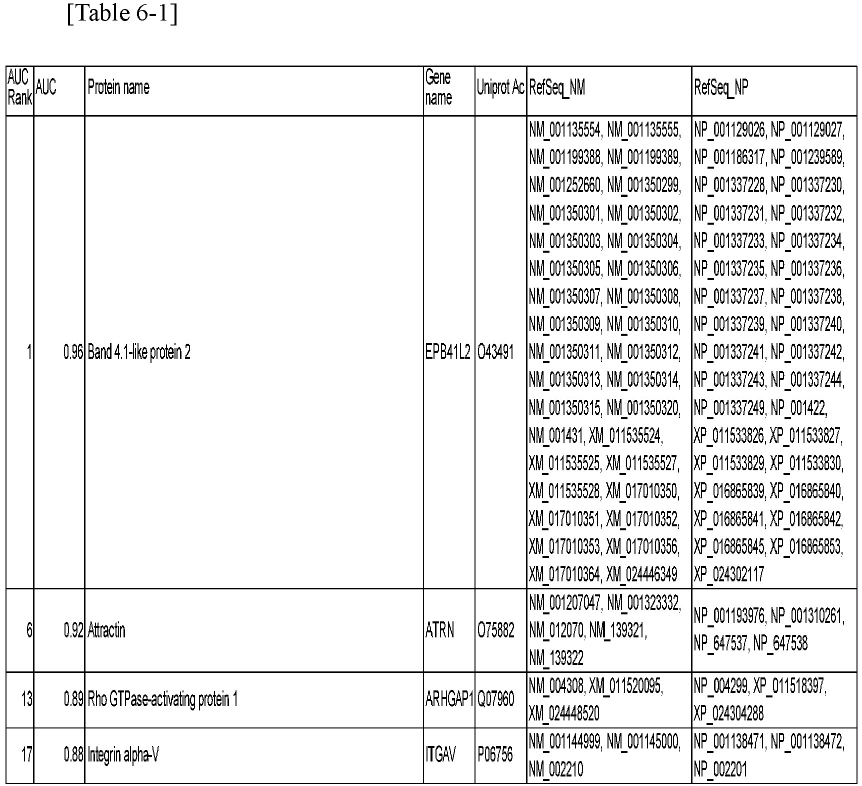

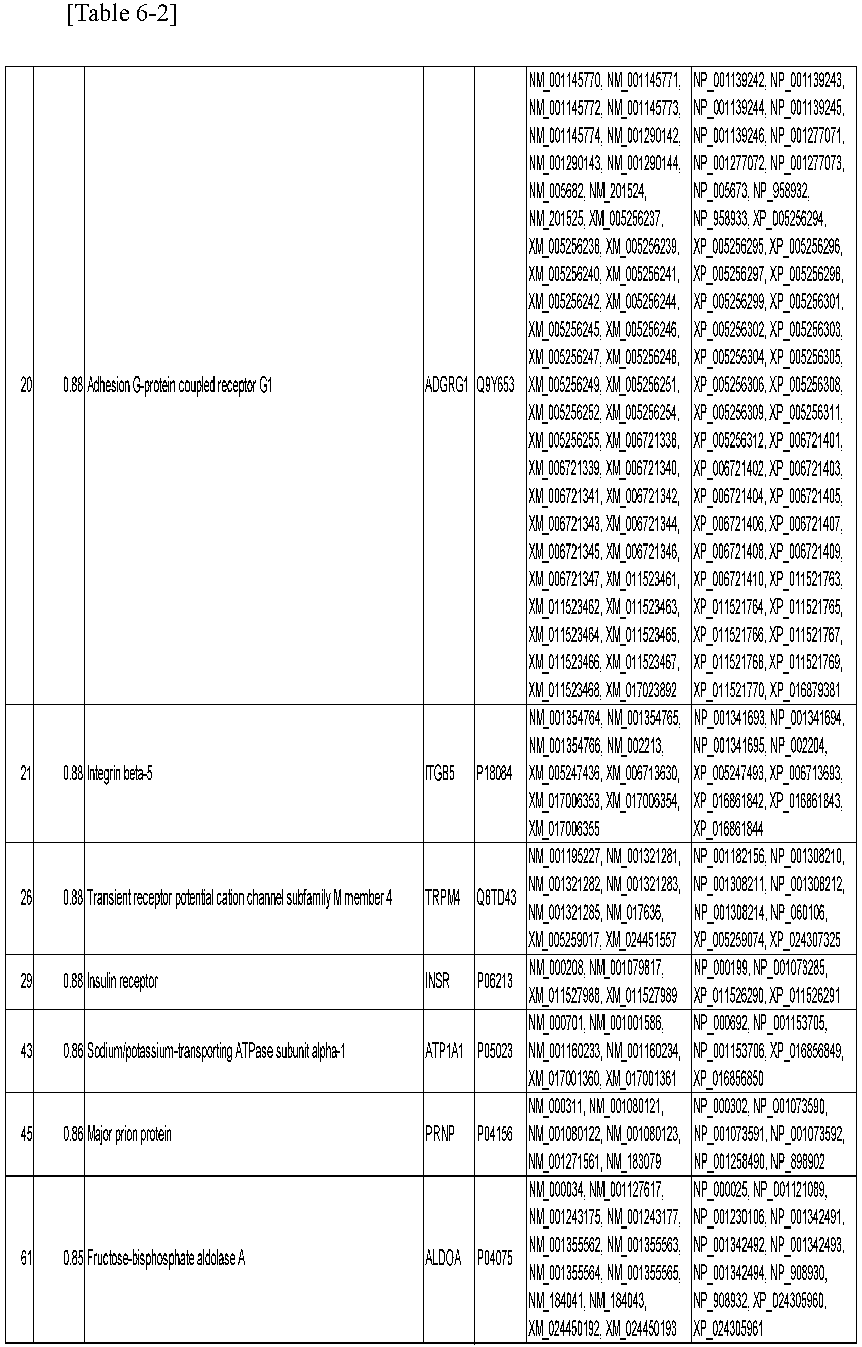

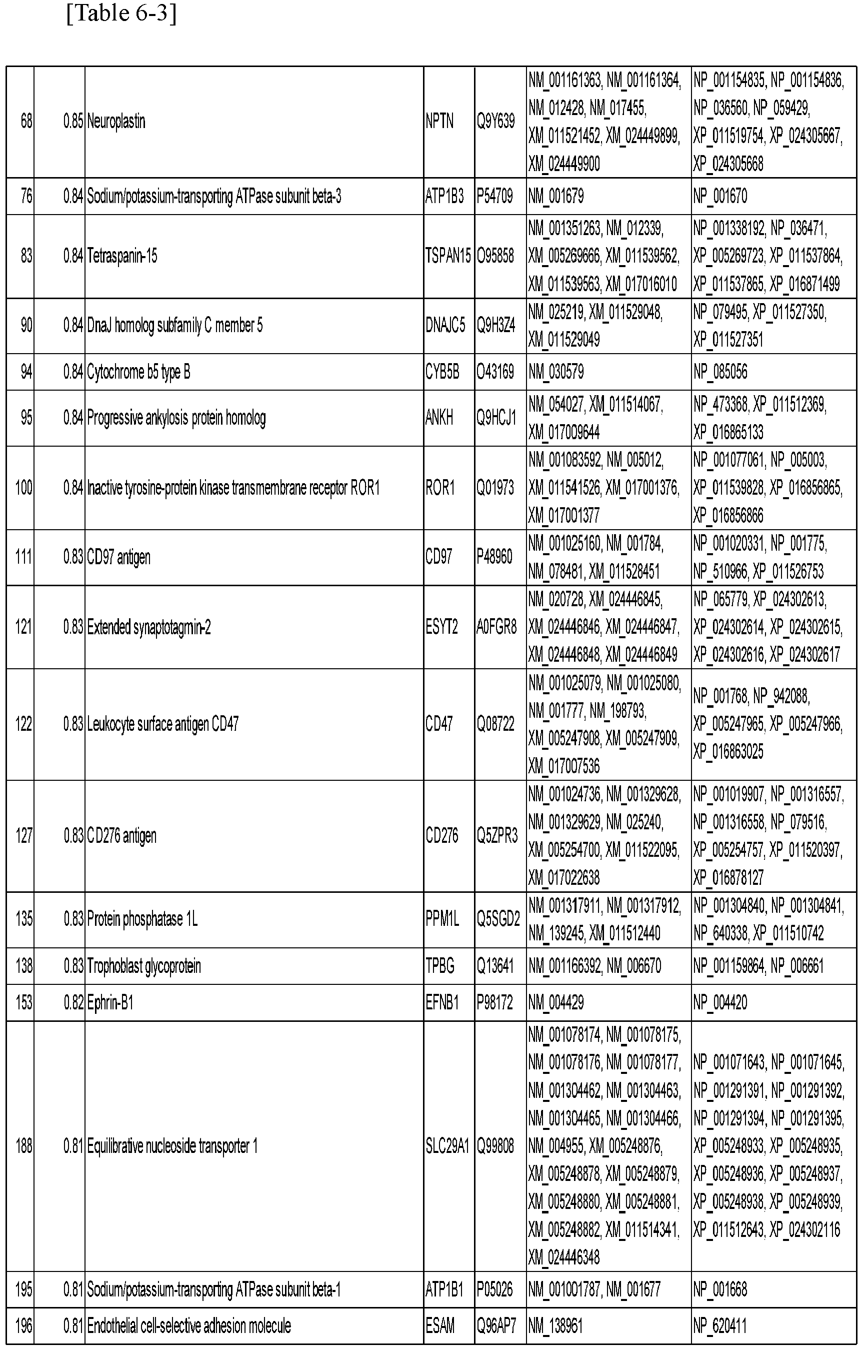

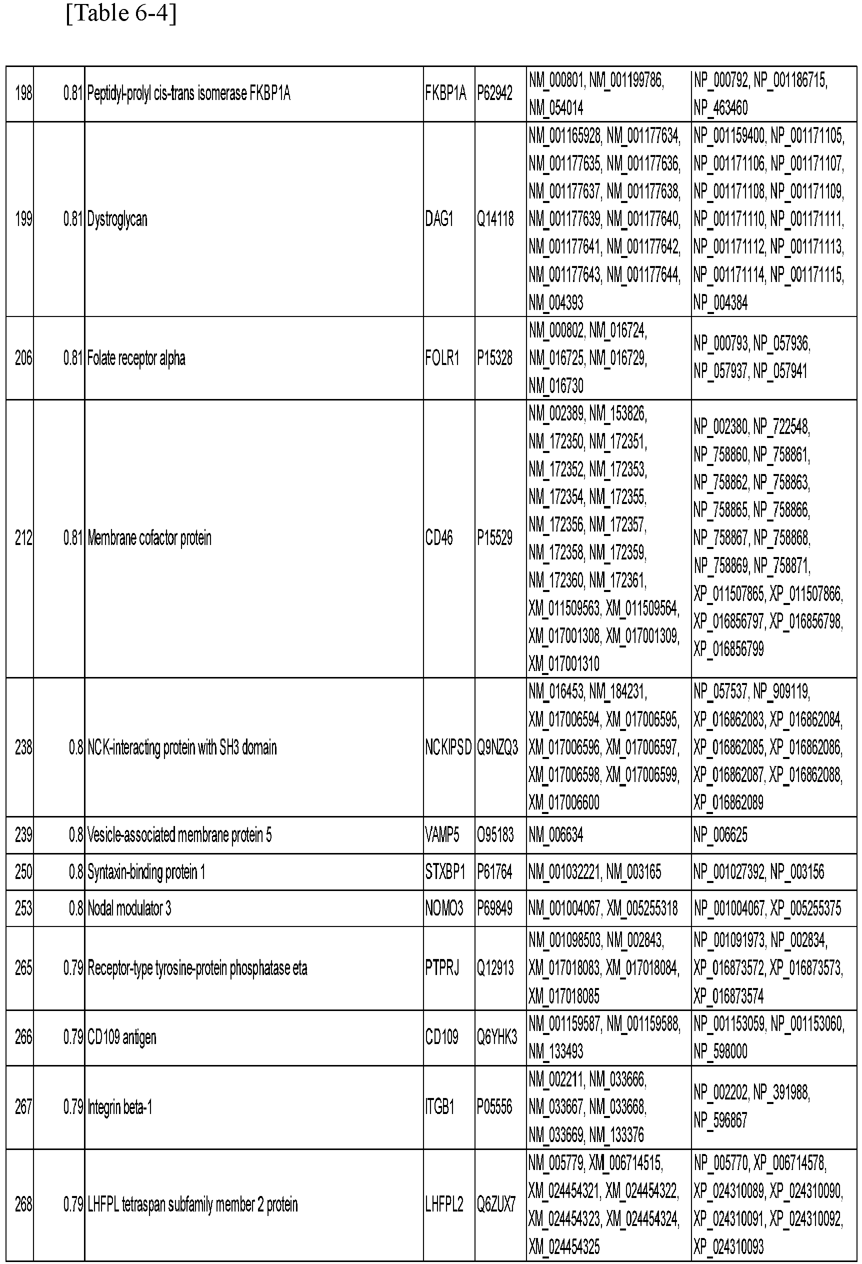

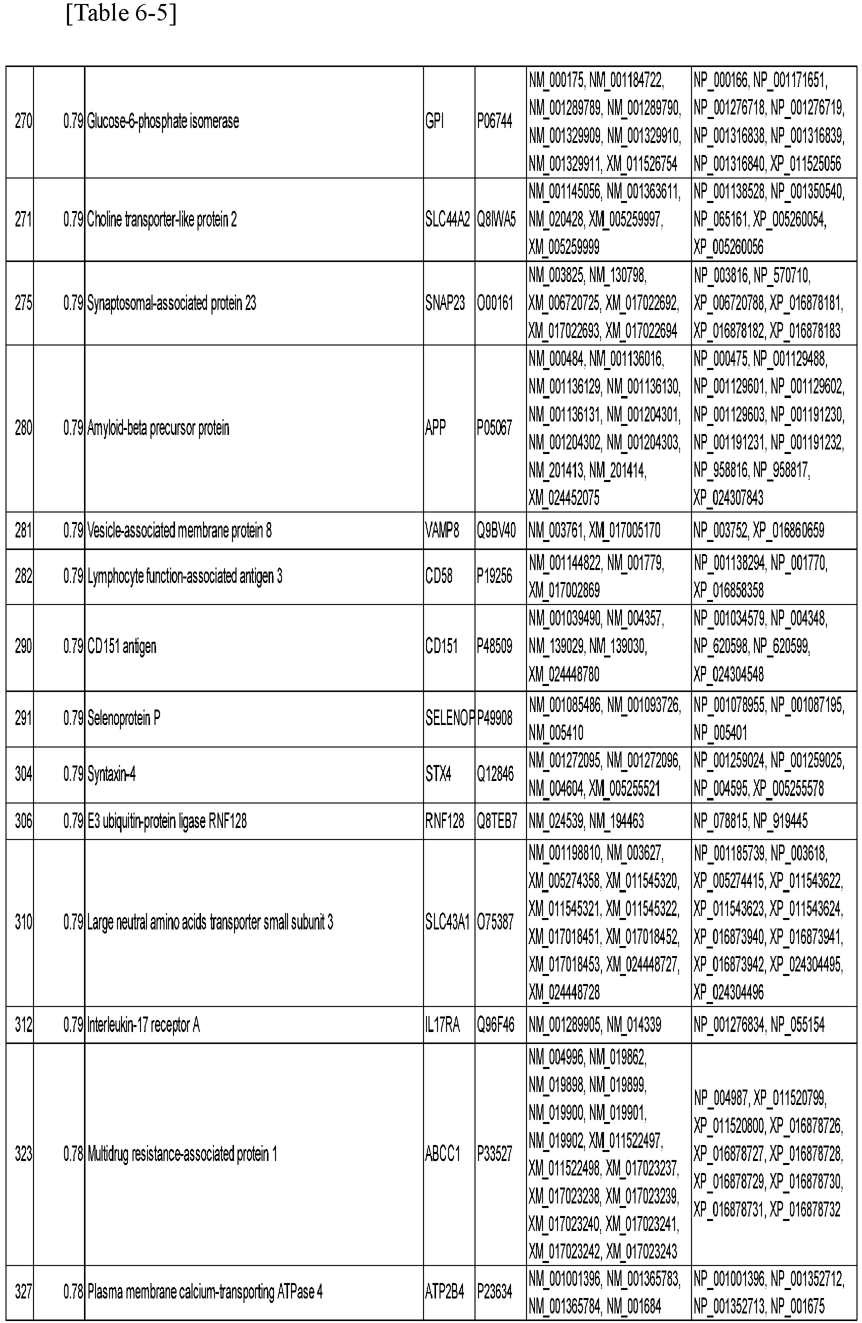

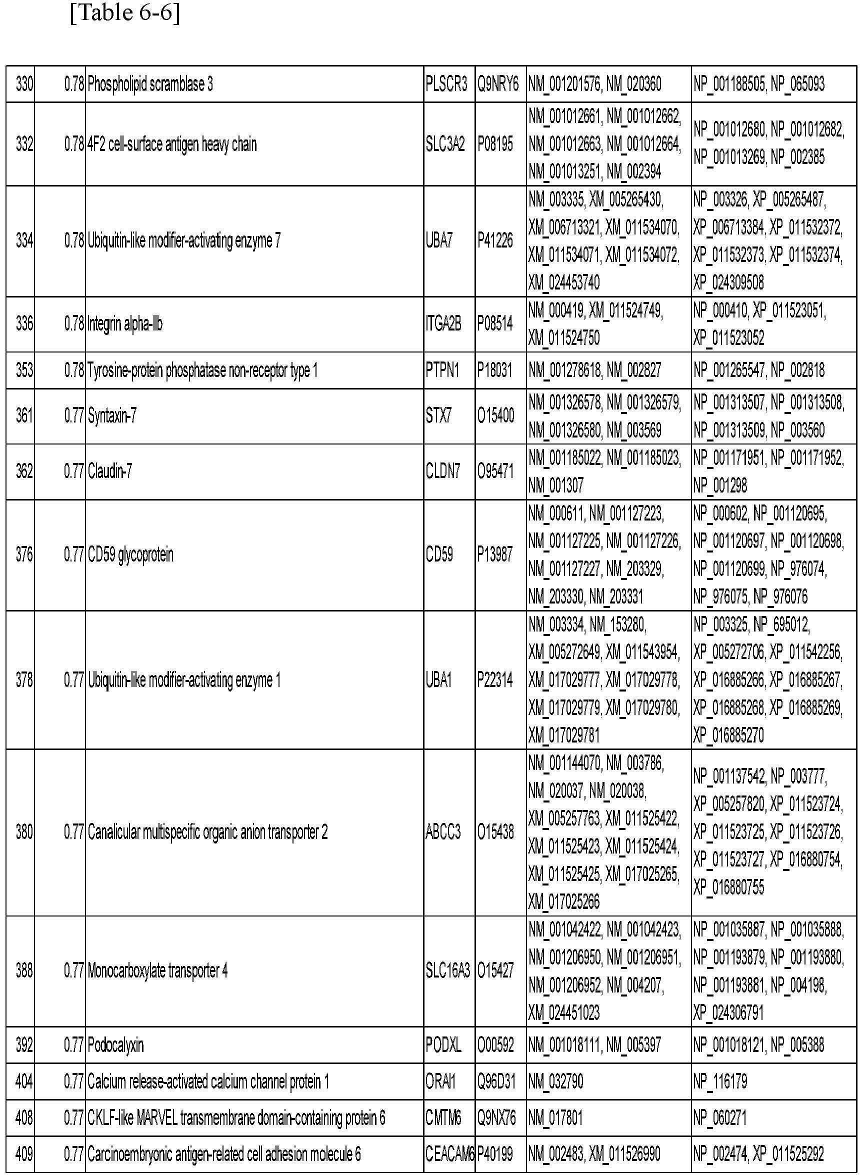

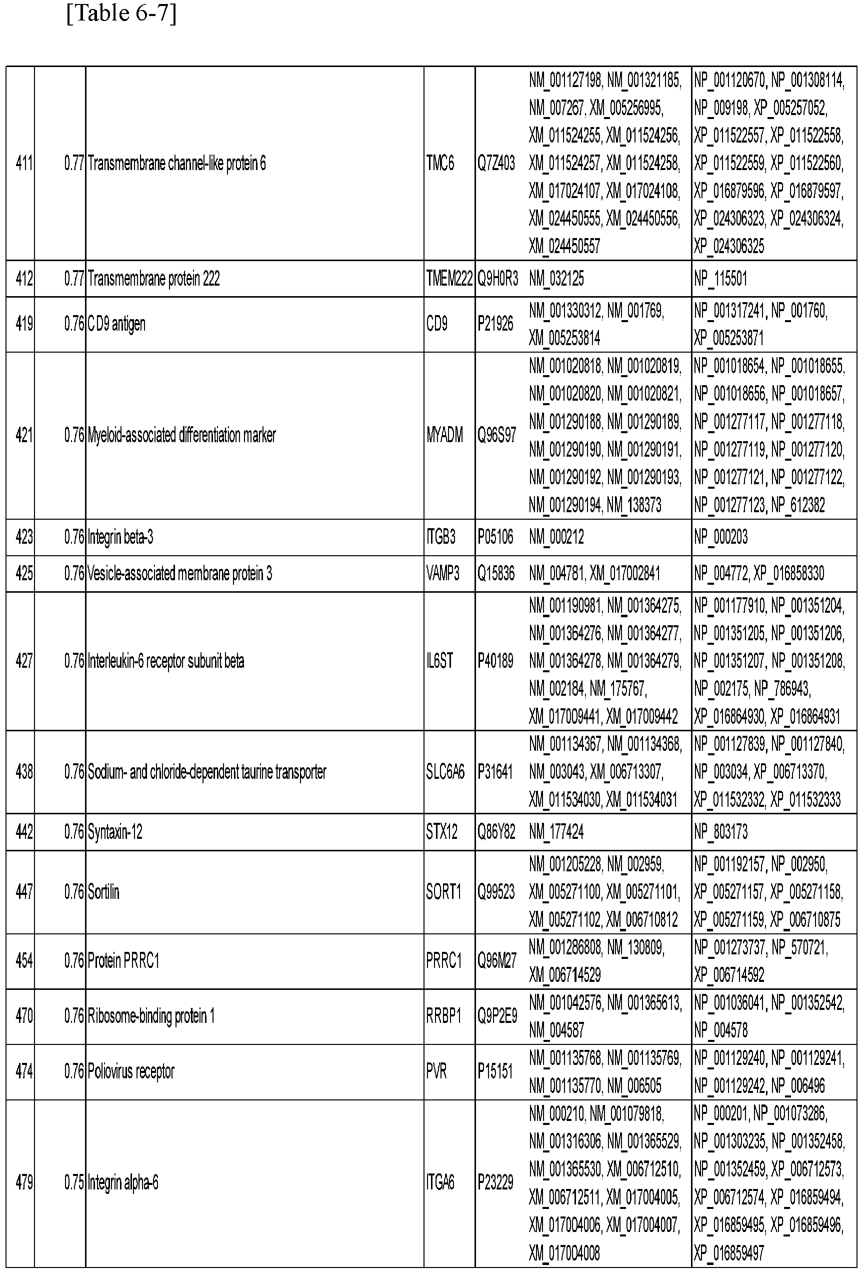

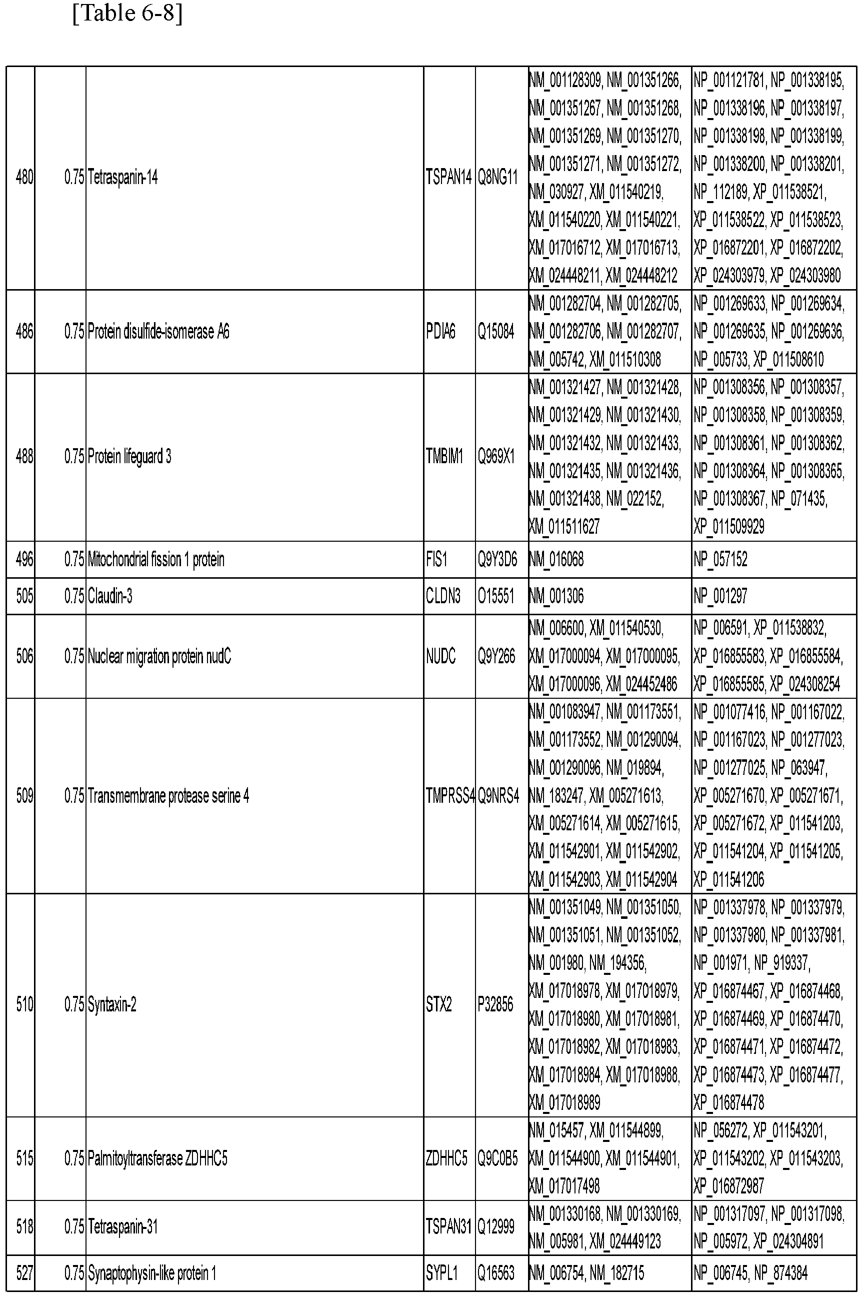

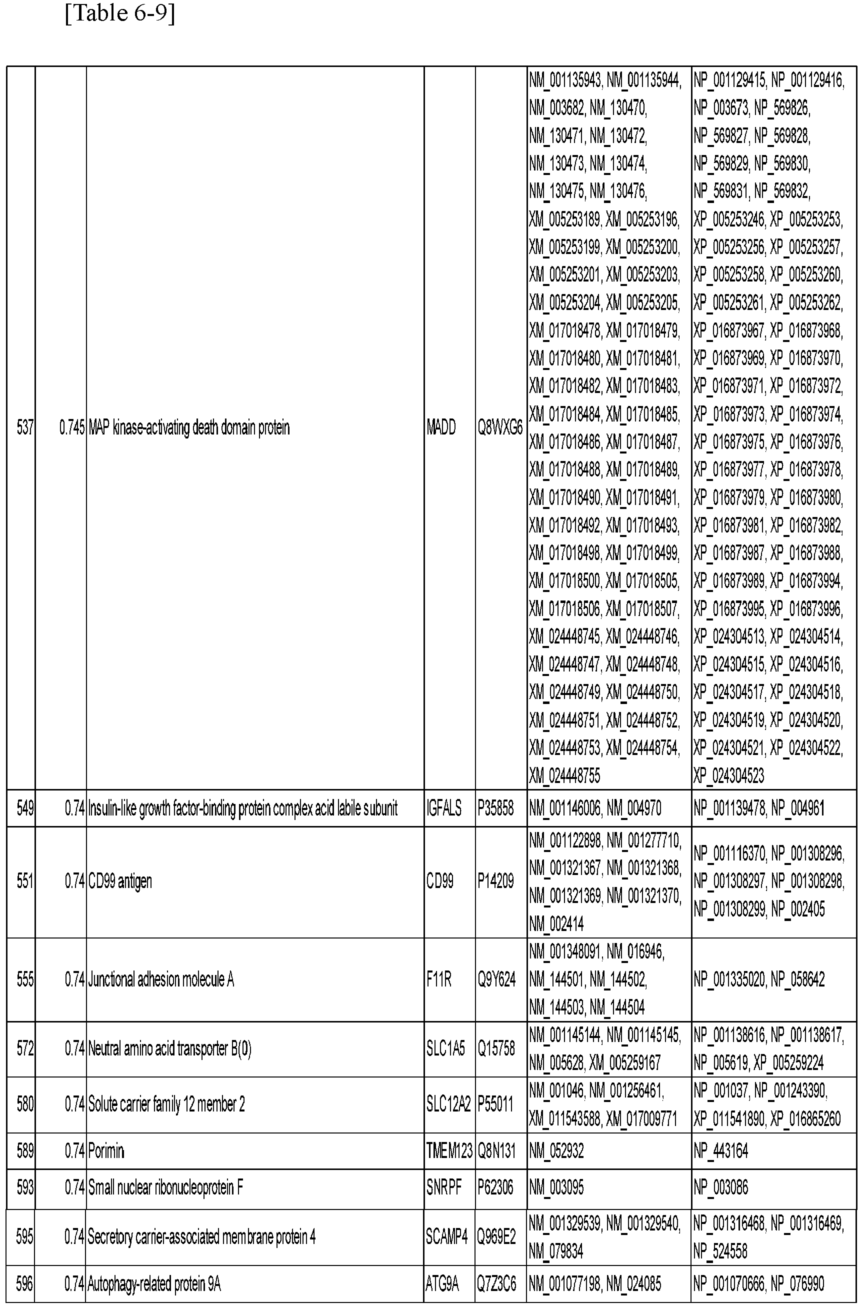

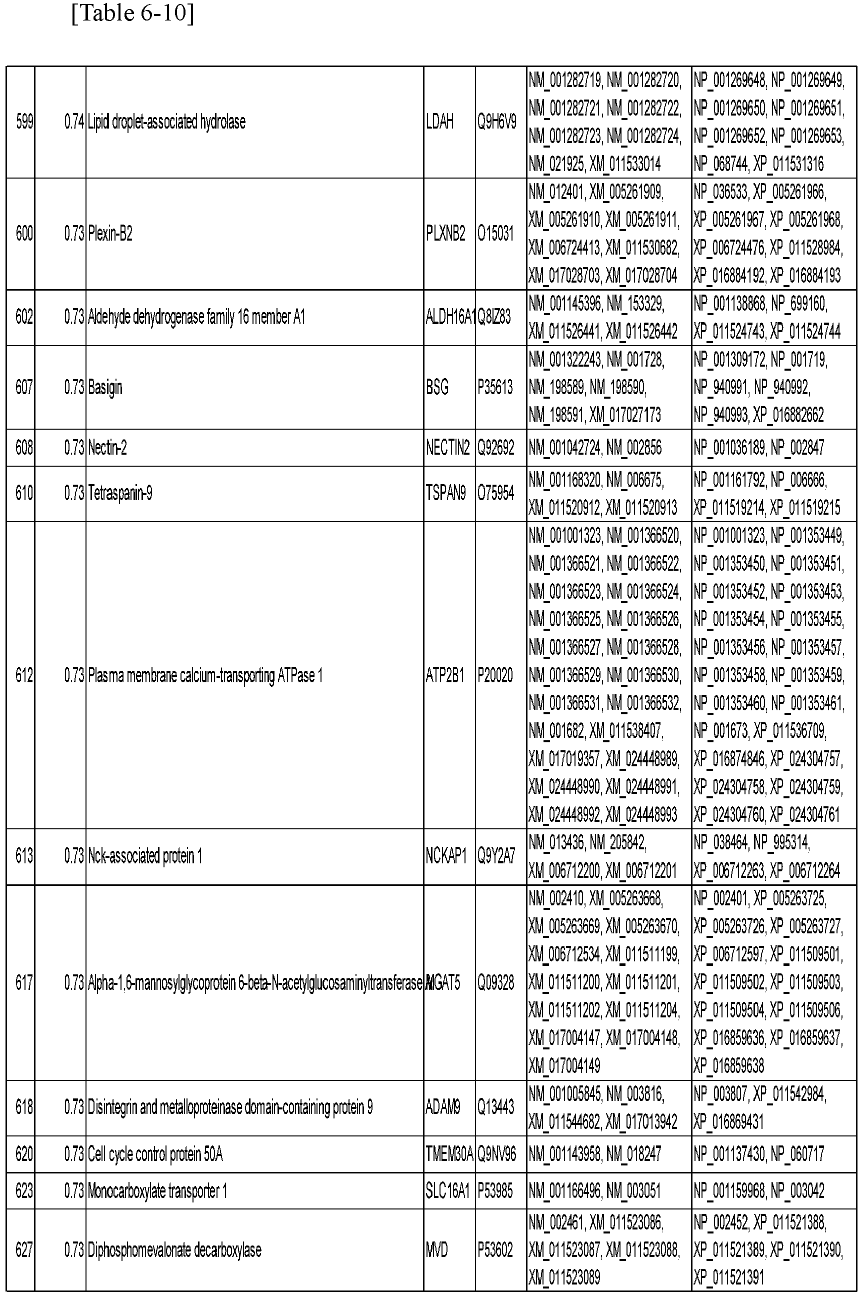

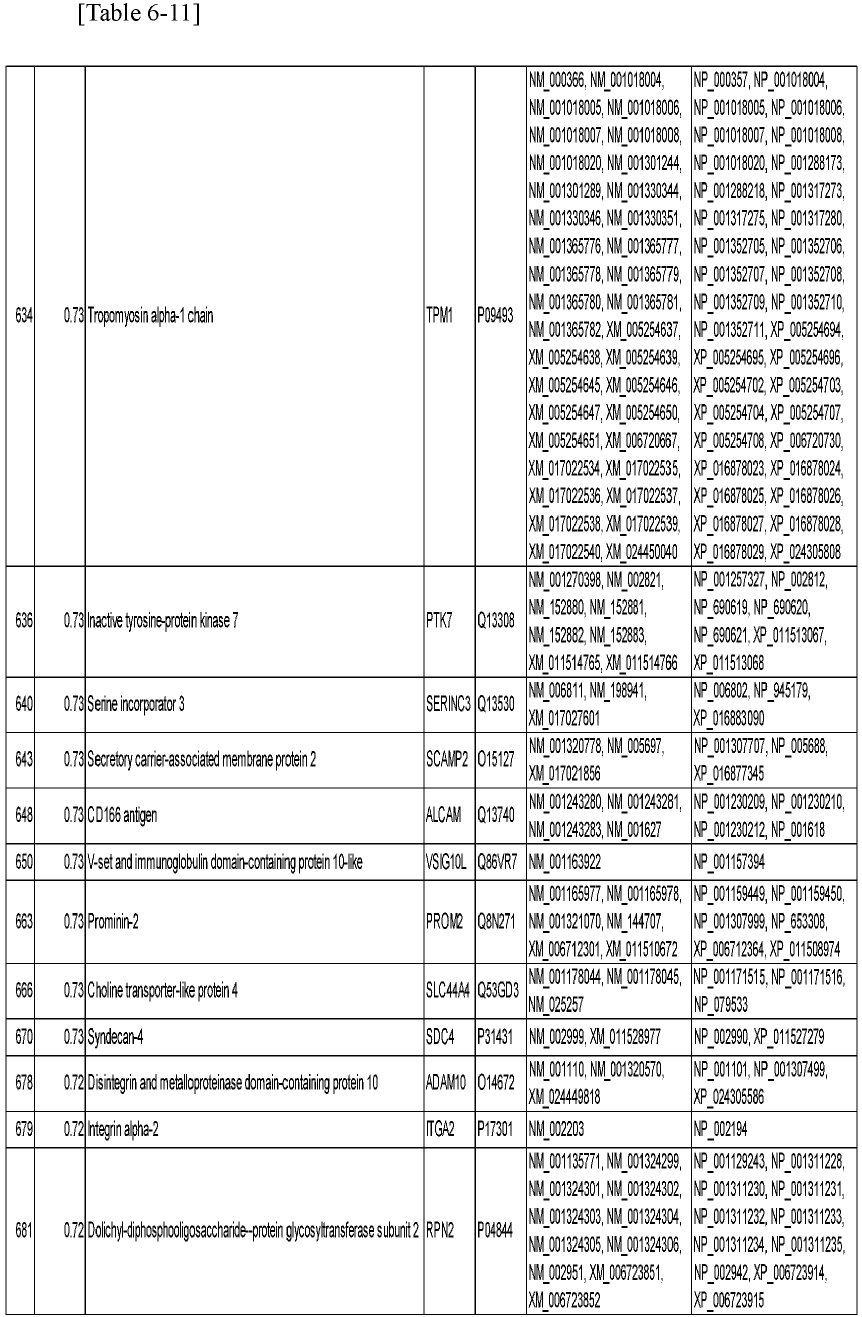

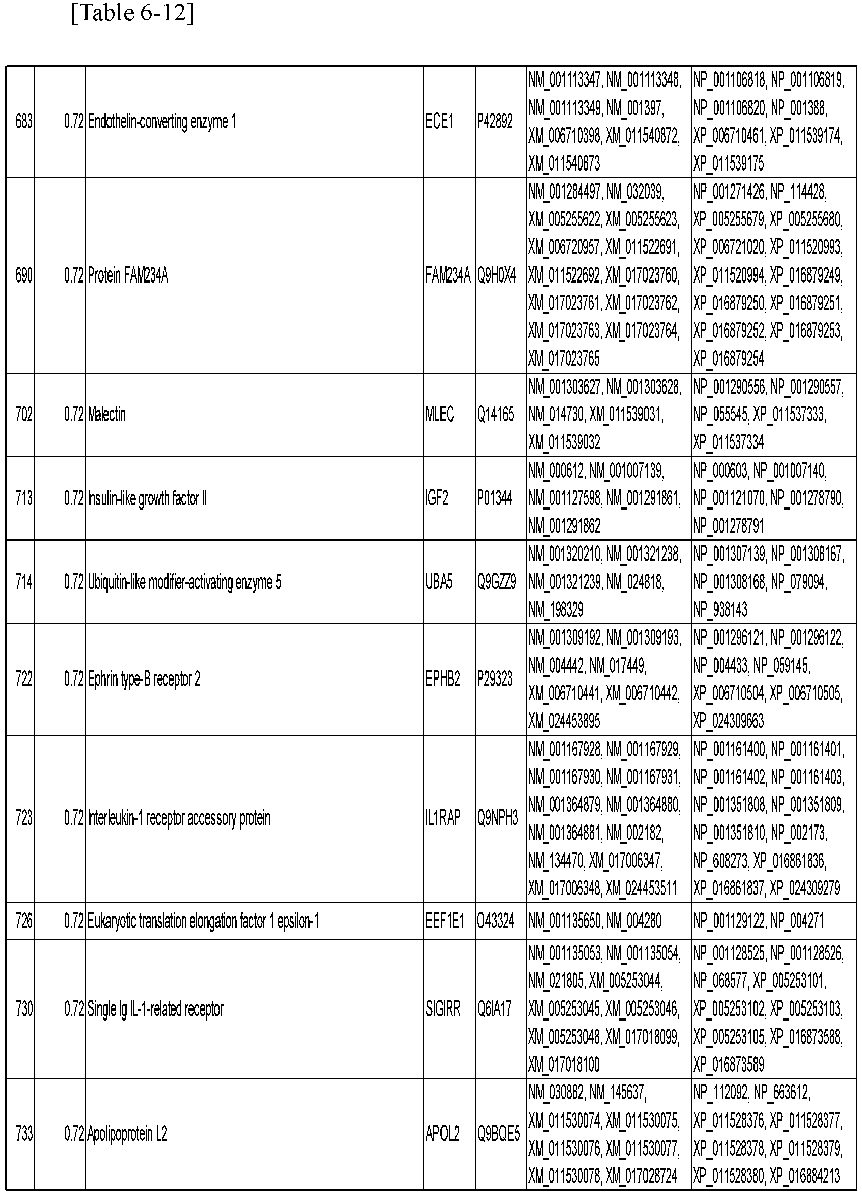

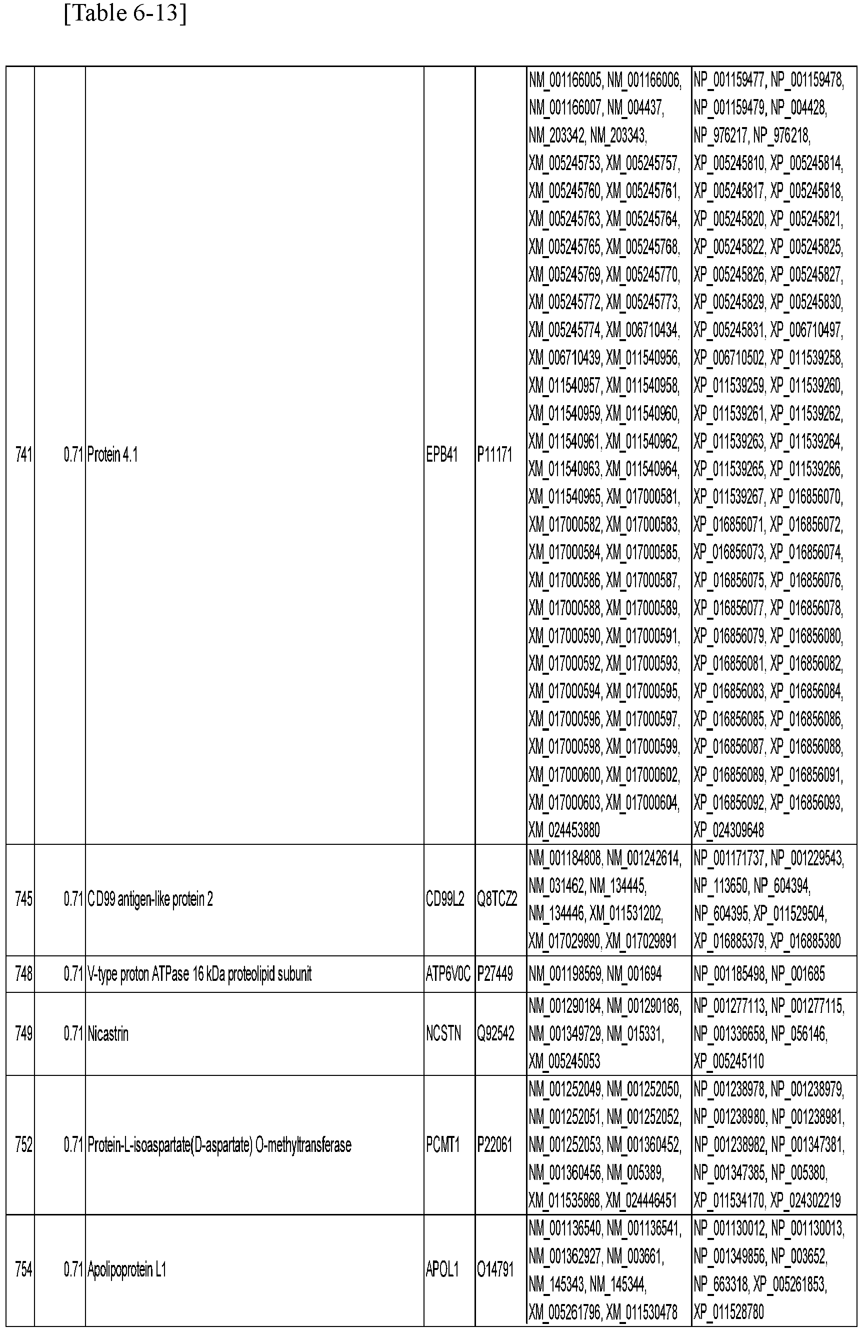

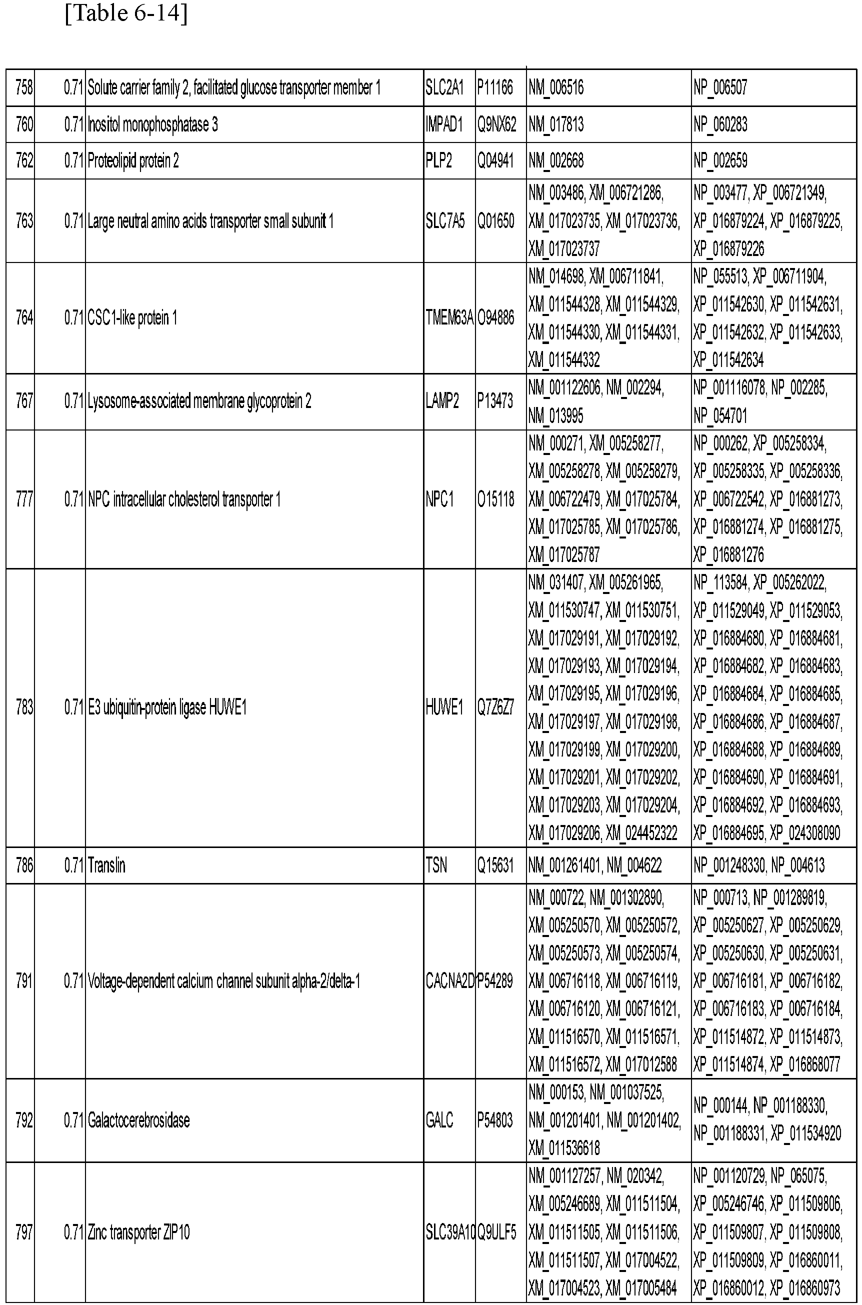

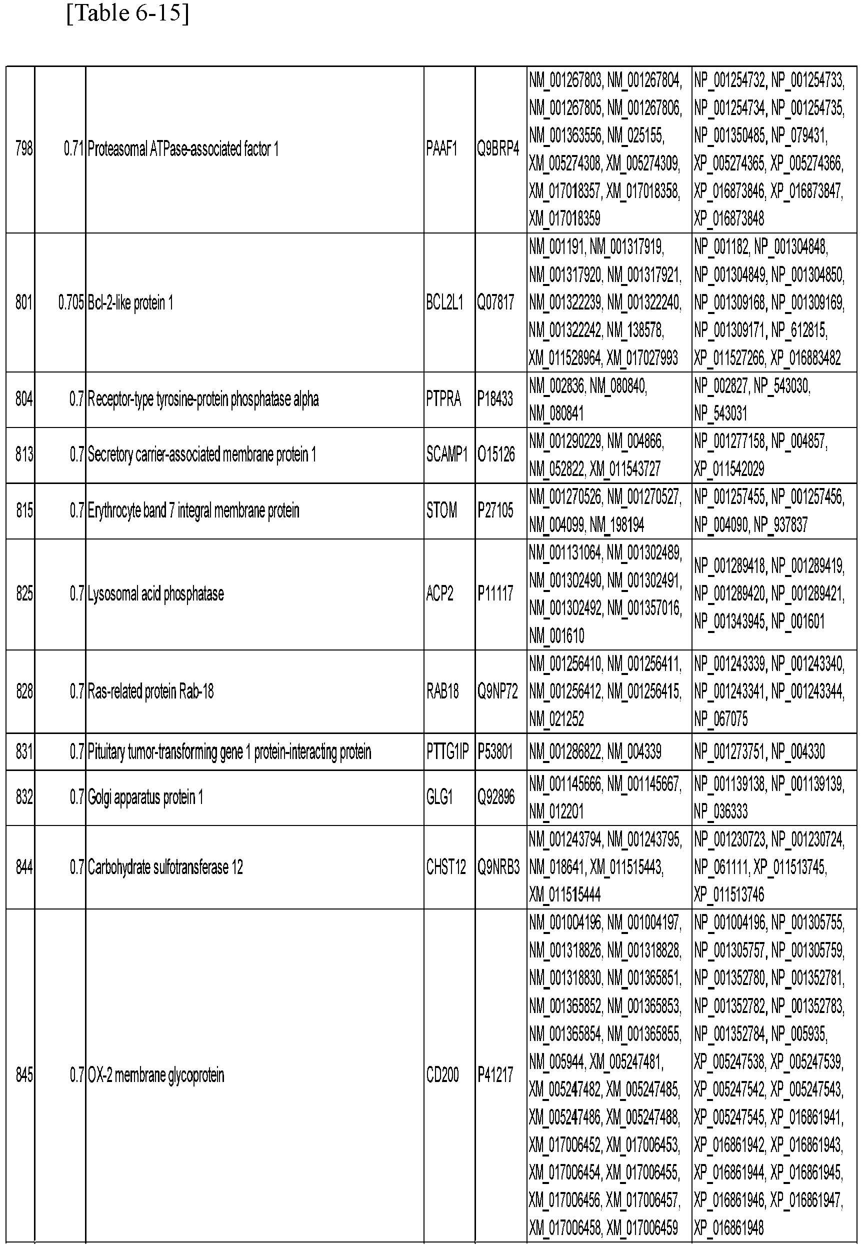

As shown in Table 6-1 to Table 6-15, 36 membrane proteins were detected for AUC 0.8 or higher and 168 membrane proteins were detected for 0.7 or higher.

### 4. Extraction of Protein Groups that did not Generate False Negatives for Patients (Responders) having Drug-Sensitivity

For 10 responders, protein groups for which false negatives were not generated based on the threshold calculated by the Youden index were extracted and the results sorted in descending order of AUC are shown in Table 7-1 to Table 7-4.

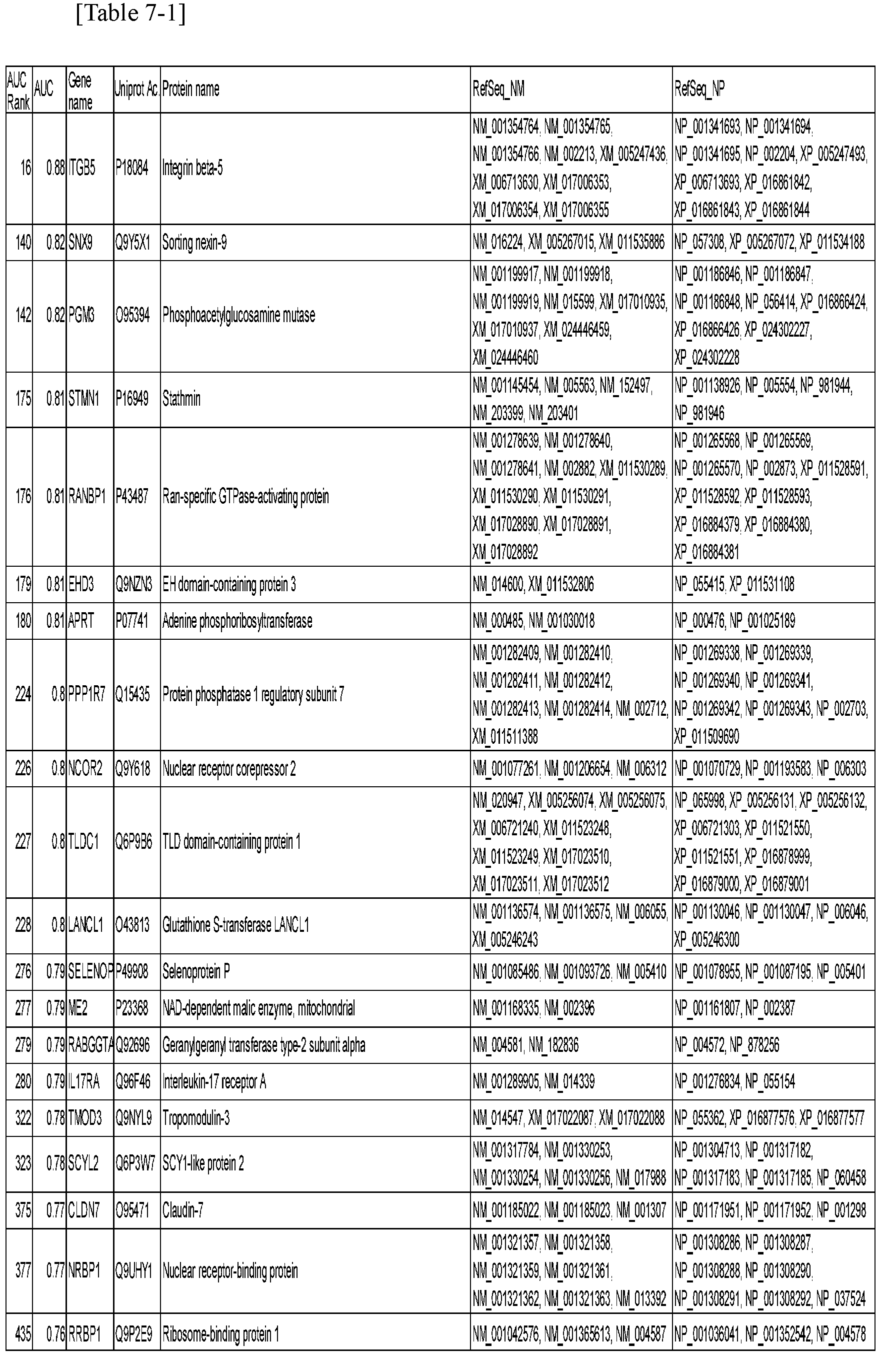

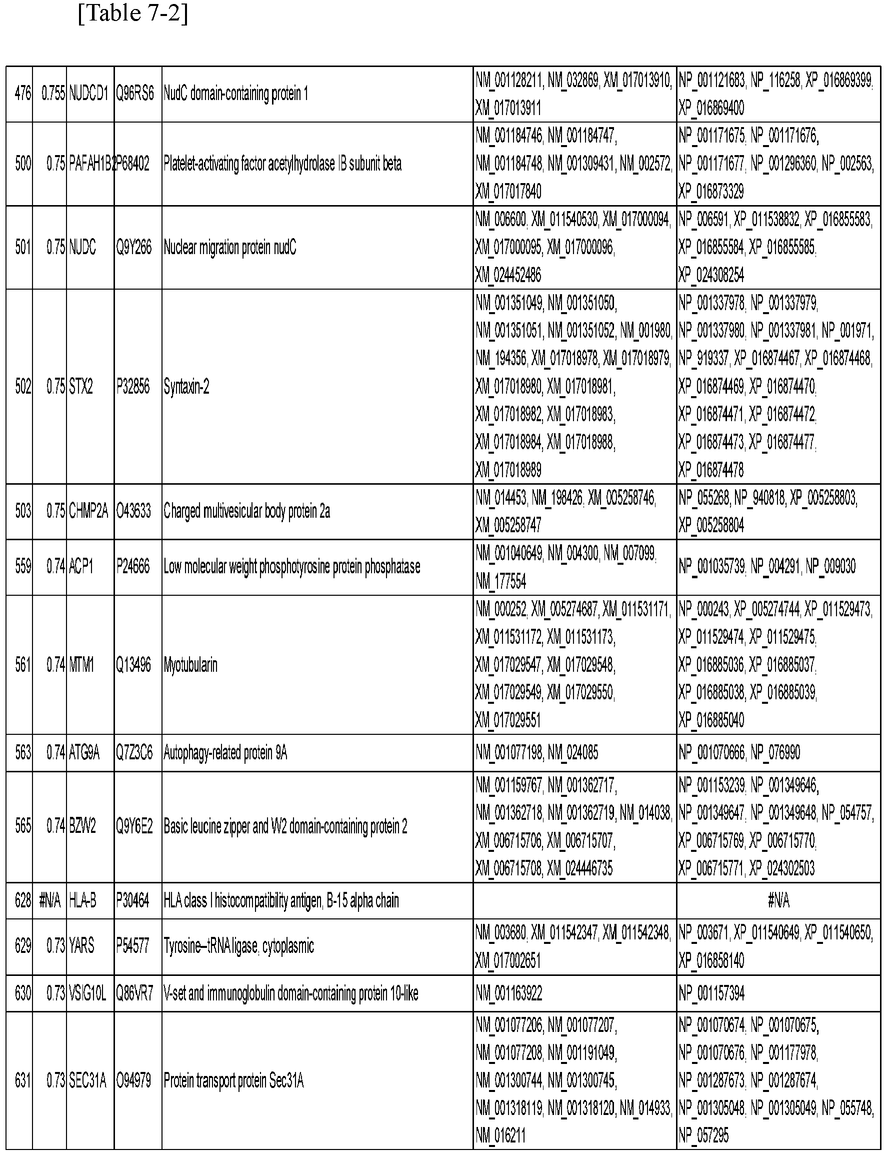

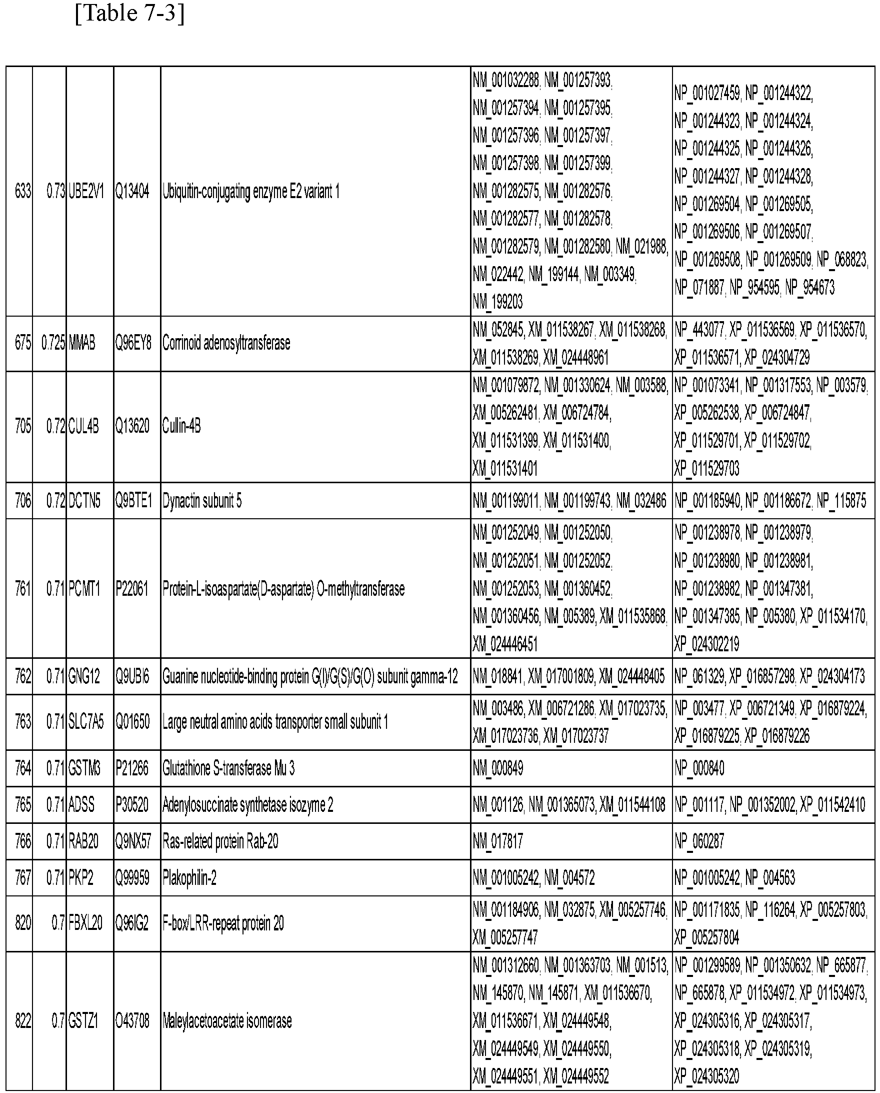

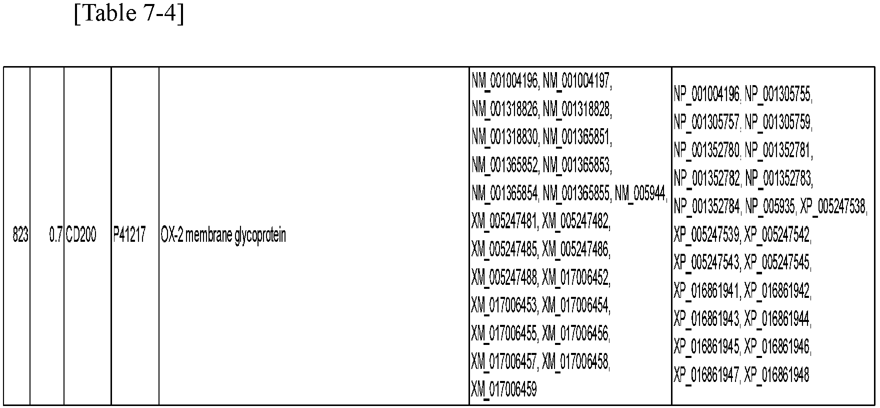

As shown in Table 7-1 to Table 7-4, 11 proteins were present for AUC 0.8 or higher and 47 for AUC 0.7 or higher. It was considered that it was possible to construct an error-resistant multi-marker by combining these markers.

### INDUSTRIAL APPLICABILITY

According to the method of the present embodiment, it is possible to predict the efficacy of treatment of a lung cancer patient using an immune checkpoint inhibitor with high accuracy.

## Claims

1. A method for predicting efficacy of treatment of a lung cancer patient using an immune checkpoint inhibitor, the method comprising:
isolating exosomes from a biological sample derived from the lung cancer patient; and
determining an expression level of a protein present in the exosomes by a mass spectrometry method,
wherein the protein is one or more proteins selected from the group of proteins shown in Table 1-1 to Table 1-6.
**[Table 1-1]**
| Protein Name |
|---|
| Band 4.1-like protein 2 |
| Cullin-2 |
| Protein Hikeshi |
| Hippocalcin-like protein 1 |
| UTP--glucose-1-phosphate uridylyltransferase |
| Attractin |
| Annexin A3 |
| Rap1 GTPase-activating protein 2 |
| Radixin |
| Annexin A5 |
| ADP-ribosylation factor 5 |
| L-lactate dehydrogenase B chain |
| Rho GTPase-activating protein 1 |
| Drebrin |
| Serine/threonine-protein kinase PAK 2 |
| Protein BRICK1 |
| Integrin alpha-V |
| Protein kinase C and casein kinase substrate in neurons protein 2 |
| Triokinase/FMN cyclase |
| Adhesion G-protein coupled receptor G1 |
| Integrin beta-5 |
| Isochorismatase domain-containing protein 1 |
| Septin-2 |
| Hydroxyacyl-coenzyme A dehydrogenase, mitochondrial |
| Serine/threonine-protein kinase 38 |
| Transient receptor potential cation channel subfamily M member 4 |
| Cytoskeleton-associated protein 5 |
| FAS-associated factor 1 |
| Insulin receptor |
| Tropomyosin alpha-4 chain |
| Programmed cell death protein 6 |
| Leukocyte elastase inhibitor |
| ADP-ribosylation factor 6 |
| Complement component C9 |
| ATP-dependent (S)-NAD(P)H-hydrate dehydratase |
| Actin, alpha cardiac muscle 1 |
| Heat shock 70 kDa protein 4 |
| 14-3-3 protein theta |
| Phosphatidylinositol transfer protein beta isoform |
| Phosphoribosyltransferase domain-containing protein 1 |
| Costars family protein ABRACL |
| Ubiquitin-conjugating enzyme E2 L5 |
| Sodium/potassium-transporting ATPase subunit alpha-1 |
| 14-3-3 protein epsilon |
**[Table 1-2]**
| |
|---|
| Major prion protein |
| F-actin-capping protein subunit alpha-2 |
| HLA class I histocompatibility antigen, Cw-4 alpha chain |
| GTP-binding protein SAR1a |
| Actin-related protein 2/3 complex subunit 5 |
| Glutathione reductase, mitochondrial |
| Guanine nucleotide-binding protein subunit alpha-11 |
| Protein disulfide-isomerase A5 |
| MARCKS-related protein |
| Septin-7 |
| Hsc70-interacting protein |
| COP9 signalosome complex subunit 8 |
| Vacuolar protein sorting-associated protein 37C |
| Acidic fibroblast growth factor intracellular-binding protein |
| ADP-ribosyl cyclase/cyclic ADP-ribose hydrolase 1 |
| Ribosome maturation protein SBDS |
| Fructose-bisphosphate aldolase A |
| BH3-interacting domain death agonist |
| Charged multivesicular body protein 4b |
| Guanine nucleotide-binding protein G(s) subunit alpha isoforms short |
| cAMP-dependent protein kinase catalytic subunit alpha |
| Cytosolic non-specific dipeptidase |
| Dynamin-1 |
| Neuroplastin |
| IST1 homolog |
| Sorting nexin-5 |
| Mitochondrial import inner membrane translocase subunit Tim13 |
| Exocyst complex component 4 |
| Moesin |
| Serum albumin |
| Myristoylated alanine-rich C-kinase substrate |
| Sodium/potassium-transporting ATPase subunit beta-3 |
| Vinculin |
| Actin-related protein 2/3 complex subunit 1B |
| Alcohol dehydrogenase class-3 |
| Cysteine and glycine-rich protein 1 |
| E3 ubiquitin-protein ligase TRIM23 |
| Actin-related protein 3 |
| Tetraspanin-15 |
| Ras-related C3 botulinum toxin substrate 1 |
| COP9 signalosome complex subunit 2 |
| Myotrophin |
| Rho-related GTP-binding protein RhoF |
| COP9 signalosome complex subunit 3 |
| Glutathione synthetase |
| DnaJ homolog subfamily C member 5 |
**[Table 1-3]**
| |
|---|
| Glutathione S-transferase omega-1 |
| Glutathione S-transferase Mu 2 |
| Ragulator complex protein LAMTOR2 |
| Cytochrome b5 type B |
| Progressive ankylosis protein homolog |
| Septin-11 |
| Rho-associated protein kinase 1 |
| Ras-related C3 botulinum toxin substrate 3 |
| Isocitrate dehydrogenase [NAD] subunit alpha, mitochondrial |
| Inactive tyrosine-protein kinase transmembrane receptor ROR1 |
| Abl interactor 2 |
| Cell division control protein 42 homolog |
| Transgelin-2 |
| Ras-related protein Rab-2A |
| Charged multivesicular body protein 6 |
| ADP-ribosylation factor 3 |
| Glyoxylate reductase/hydroxypyruvate reductase |
| Profilin-1 |
| Ras-related protein Rab-11B |
| Rab GDP dissociation inhibitor alpha |
| CD97 antigen |
| Programmed cell death protein 10 |
| Antithrombin-III |
| F-actin-capping protein subunit beta |
| Actin-related protein 2/3 complex subunit 2 |
| Twinfilin-2 |
| Ras-related protein Rab-21 |
| Vacuolar-sorting protein SNF8 |
| Caspase-6 |
| Calcineurin subunit B type 1 |
| Extended synaptotagmin-2 |
| Leukocyte surface antigen CD47 |
| COP9 signalosome complex subunit 1 |
| 26S proteasome non-ATPase regulatory subunit 7 |
| Eukaryotic translation initiation factor 3 subunit C-like protein |
| Deoxycytidylate deaminase |
| CD276 antigen |
| Caspase-3 |
| Leucine-rich repeat-containing protein 57 |
| Aldose reductase |
| Diphosphoinositol polyphosphate phosphohydrolase 1 |
| Coagulation factor XIII A chain |
| Hsp70-binding protein 1 |
| Sorting nexin-4 |
| Protein phosphatase 1L |
| Pleckstrin homology domain-containing family N member 1 |
**[Table 1-4]**
| |
|---|
| AP-2 complex subunit alpha-2 |
| Trophoblast glycoprotein |
| Ras-related protein Rab-23 |
| 14-3-3 protein eta |
| Peroxiredoxin-6 |
| 14-3-3 protein beta/alpha |
| Copine-2 |
| 14-3-3 protein gamma |
| Superoxide dismutase [Cu-Zn] |
| Microtubule-associated protein RPIEB family member 1 |
| Vasodilator-stimulated phosphoprotein |
| Phosphoglucomutase-1 |
| Adenylyl cyclase-associated protein 1 |
| Calpain small subunit 1 |
| Dual specificity protein phosphatase 23 |
| Dihydropteridine reductase |
| Ephrin-B1 |
| Golgin subfamily A member 7 |
| Xaa-Pro aminopeptidase 1 |
| Actin-related protein 2/3 complex subunit 3 |
| Sorting nexin-9 |
| Pleckstrin homology domain-containing family O member 2 |
| WD repeat-containing protein 44 |
| N(G),N(G)-dimethylarginine dimethylaminohydrolase 2 |
| COP9 signalosome complex subunit 6 |
| Guanine nucleotide-binding protein G(I)/G(S)/G(O) subunit gamma-10 |
| Rab-like protein 3 |
| Phosphoacetylglucosamine mutase |
| Sorting nexin-2 |
| DDB1- and CUL4-associated factor 11 |
| Ras GTPase-activating protein 1 |
| Ribose-5-phosphate isomerase |
| SHC-transforming protein 1 |
| Glutaminyl-peptide cyclotransferase-like protein |
| Ras-related protein Rab-5C |
| Purine nucleoside phosphorylase |
| Delta-aminolevulinic acid dehydratase |
| Ras-related protein Rab-5B |
| Abl interactor 1 |
| Beta-2-glycoprotein 1 |
| Serine/threonine-protein phosphatase 2B catalytic subunit alpha isoform |
| Actin-related protein 2 |
| Ras-related protein Rap-2a |
| Sorcin |
| Type I inositol 1 ,4,5-trisphosphate 5-phosphatase |
| Ras-related protein Rab-35 |
**[Table 1-5]**
| |
|---|
| Rho GTPase-activating protein 18 |
| Ras-related protein Rab-1A |
| Deoxyribonuclease-1-like 1 |
| Peptidyl-prolyl cis-trans isomerase A |
| Adenylate kinase isoenzyme 1 |
| Equilibrative nucleoside transporter 1 |
| GTPase NRas |
| Rho-related GTP-binding protein RhoG |
| Glycogen phosphorylase, brain form |
| UV excision repair protein RAD23 homolog B |
| Omega-amidase NIT2 |
| Serine hydroxymethyltransferase, cytosolic |
| Sodium/potassium-transporting ATPase subunit beta-1 |
| Endothelial cell-selective adhesion molecule |
| Isocitrate dehydrogenase [NADP] cytoplasmic |
| Peptidyl-prolyl cis-trans isomerase FKBP1A |
| Dystroglycan |
| Tumor susceptibility gene 101 protein |
| Peroxiredoxin-5, mitochondrial |
| Protein XRP2 |
| Actin-related protein 2/3 complex subunit 4 |
| Stathmin |
| NSFL1 cofactor p47 |
| Folate receptor alpha |
| Aldo-keto reductase family 1 member A1 |
| Ran-specific GTPase-activating protein |
| Serine/threonine-protein phosphatase 2A catalytic subunit beta isoform |
| Vacuolar protein sorting-associated protein VTA1 homolog |
| Dual specificity protein phosphatase 3 |
| Membrane cofactor protein |
| Calcium-binding protein 39 |
| Argininosuccinate lyase |
| Mitochondrial import receptor subunit TOM34 |
| Protein numb homolog |
| SUMO-conjugating enzyme UBC9 |
| EH domain-containing protein 3 |
| Ras-related protein Rab-1B |
| Adenine phosphoribosyltransferase |
| Coronin-1A |
| An nexi n A4 |
| F-actin-capping protein subunit alpha-1 |
| Protein phosphatase 1 regulatory subunit 7 |
| Peflin |
| Protein/nucleic acid deglycase DJ-1 |
| Ras-related protein Rab-10 |
| Ras-related protein Rap-2b |
**[Table 1-6]**
| |
|---|
| RNA transcription, translation and transport factor protein |
| Crk-like protein |
| Serpin B6 |
| 6-phosphogluconolactonase |
| Guanine nucleotide-binding protein G(I)/G(S)/G(T) subunit beta-1 |
| Lactadherin |
| Ras-related protein Ral-A |
| Protein transport protein Sec23A |
| Dihydropyrimidinase-related protein 2 |
| NCK-interacting protein with SH3 domain |
| Vesicle-associated membrane protein 5 |
| Protein phosphatase 1F |
| Nuclear receptor corepressor 2 |
| Ubiquitin-conjugating enzyme E2 D3 |
| WW domain-binding protein 2 |
| POTE ankyrin domain family member I |
| Copine-1 |
| Alpha-galactosidase A |
| Coronin-1B |
| ATP-dependent RNA helicase DDX1 |
| Ubiquitin-conjugating enzyme E2 K |
| Syntaxin-binding protein 1 |
| WD repeat-containing protein 37 |
| MOB kinase activator 1B |
| Nodal modulator 3 |
| Nucleoside diphosphate kinase B |
| Prostaglandin reductase 1 |
| Phosphoglycerate mutase 2 |
| TLD domain-containing protein 1 |
| Calcium-binding protein 39-like |
| Glutathione S-transferase LANCL1 |
| Prefoldin subunit 6 |
| Cytosolic phospholipase A2 |
| 14-3-3 protein zeta/delta |

2. The method according to Claim 1,
wherein the protein is one or more membrane proteins selected from the group of proteins shown in Table 2.
**[Table 2]**
| Protein Name |
|---|
| Band 4.1-like protein 2 |
| Attractin |
| Rho GTPase-activating protein 1 |
| Integrin alpha-V |
| Adhesion G-protein coupled receptor G1 |
| Integrin beta-5 |
| Transient receptor potential cation channel subfamily M member 4 |
| Insulin receptor |
| Sodium/potassium-transporting ATPase subunit alpha-1 |
| Major prion protein |
| Fructose-bisphosphate aldolase A |
| Neuroplastin |
| Sodium/potassium-transporting ATPase subunit beta-3 |
| Tetraspanin-15 |
| DnaJ homolog subfamily C member 5 |
| Cytochrome b5 type B |
| Progressive ankylosis protein homolog |
| Inactive tyrosine-protein kinase transmembrane receptor ROR1 |
| CD97 antigen |
| Extended synaptotagmin-2 |
| Leukocyte surface antigen CD47 |
| CD276 antigen |
| Protein phosphatase 1L |
| Trophoblast glycoprotein |
| Ephrin-B1 |
| Equilibrative nucleoside transporter 1 |
| Sodium/potassium-transporting ATPase subunit beta-1 |
| Endothelial cell-selective adhesion molecule |
| Peptidyl-prolyl cis-trans isomerase FKBP1A |
| Dystroglycan |
| Folate receptor alpha |
| Membrane cofactor protein |
| NCK-interacting protein with SH3 domain |
| Vesicle-associated membrane protein 5 |
| Syntaxin-binding protein 1 |
| Nodal modulator 3 |

3. The method according to Claim 1,
wherein the protein is one or more proteins selected from the group of proteins shown in Table 3.
**[Table 3]**
| Protein Name |
|---|
| Integrin beta-5 |
| Sorting nexin-9 |
| Phosphoacetylglucosamine mutase |
| Stathmin |
| Ran-specific GTPase-activating protein |
| EH domain-containing protein 3 |
| Adenine phosphoribosyltransferase |
| Protein phosphatase 1 regulatory subunit 7 |
| Nuclear receptor corepressor 2 |
| TLD domain-containing protein 1 |
| Glutathione S-transferase LANCL1 |

4. The method according to any one of Claims 1 to 3,
wherein the immune checkpoint inhibitor is an anti-PD-1 antibody or an anti-PD-L1 antibody.

5. The method according to any one of Claims 1 to 4,
wherein the immune checkpoint inhibitor is one or more selected from the group consisting of nivolumab, pembrolizumab, and atezolizumab.

6. The method according to any one of Claims 1 to 5,
wherein, as a method for isolating exosomes from the biological sample derived from the lung cancer patient, a mixture of magnetic particles bound to an anti-CD9 antibody, an anti-CD81 antibody, or an anti-CD63 antibody is used.
